# EUROPÄISCHE PATENTSCHRIFT

(11) **EP 2 114 852 B1**
(45) Veröffentlichungstag und Bekanntmachung des Hinweises auf die Patenterteilung: **23.09.2015**
(21) Anmeldenummer: 08708128.7
(22) Anmeldetag: 24.01.2008
(51) Int. Cl.: C07C 57/07, C07C 51/44, C07C 51/48, C07C 51/215, C07C 51/25

(54) **VERFAHREN ZUR HERSTELLUNG VON ACRYLSÄURE**
METHOD FOR THE PRODUCTION OF ACRYLIC ACID
PROCÉDÉ DE PRÉPARATION D'ACIDE ACRYLIQUE

(30) Priorität: 26.01.2007 US 886771 P; 26.01.2007 DE 102007004960; 16.11.2007 DE 102007055086; 16.11.2007 US 988619 P
(43) Veröffentlichungstag der Anmeldung: 11.11.2009
(73) Patentinhaber: BASF SE, 67056 Ludwigshafen (DE)
(72) Erfinder: DIEFENBACHER, Armin, 67361 Freisbach (DE); HAMMON, Ulrich, 68163 Mannheim (DE); SCHLIEPHAKE, Volker, 67105 Schifferstadt (DE); SIEDER, Georg, 67098 Bad Dürkheim (DE)
(86) Internationale Anmeldenummer: PCT/EP2008/050785
(87) Internationale Veröffentlichungsnummer: WO 2008/090190

(56) Entgegenhaltungen:
- WO-A-03/095411
- DE-A1- 10 235 847
- DE-A1- 10 332 758

## Beschreibung

Vorliegende Erfindung betrifft ein Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, die Temperatur des Acrylsäure, Wasserdampf und Nebenkomponenten enthaltenden Produktgasgemischs gegebenenfalls durch direkte und/oder indirekte Kühlung verringert, und das Acrylsäure, Wasserdampf und Nebenkomponenten enthaltende Produktgasgemisch anschließend in eine mit trennwirksamen Einbauten ausgerüstete Kondensationskolonne leitet, innerhalb der Kondensationskolonne in sich selbst aufsteigen lässt und dabei fraktionierend kondensiert sowie über einen ersten, oberhalb der Zuführstelle des Produktgasgemischs in die Kondensationskolonne befindlichen, Seitenabzug eine Wasser und Nebenkomponenten insgesamt entreichert (abgereichert) enthaltende rohe Acrylsäure als Zielprodukt und über einen zweiten, oberhalb des ersten Seitenabzugs befindlichen, Flüssigphasenabzug (vorzugsweise einen Seitenabzug) noch Acrylsäure und Nebenkomponenten enthaltendes saures Wasser und am Kopf der Kondensationskolonne ein tiefer (bei niedrigerer Temperatur (bezogen auf Atmosphärendruck)) als Wasser siedende Nebenkomponenten enthaltendes Restgasgemisch sowie aus dem Sumpfraum der Kondensationskolonne eine noch Acrylsäure und schwerer als Acrylsäure siedende (bezogen auf Atmosphärendruck) Folgeprodukte und Nebenkomponenten enthaltende Sumpfflüssigkeit aus der Kondensationskolonne herausführt, eine Teilmenge des entnommenen sauren Wassers als solches und/oder nach Abkühlung desselben als Rücklaufflüssigkeit in die Kondensationskolonne rückführt und die rohe Acrylsäure gegebenenfalls wenigstens einem weiteren thermischen Trennverfahren zum Zweck der Weiterreinigung unterwirft.

Acrylsäure ist ein bedeutendes Zwischenprodukt, das beispielsweise im Rahmen der Herstellung von Polymerisatdispersionen (gegebenenfalls auch in Form ihrer Ester mit Alkanolen) sowie von Wasser superabsorbierenden Polymerisaten Verwendung findet.

Acrylsäure ist unter anderem durch heterogen katalysierte Gasphasen-Partialoxidation von C₃-Vorläufern (von C₃-Vorläuferverbindungen) der Acrylsäure (unter diesem Begriff sollen insbesondere solche chemischen Verbindungen zusammengefasst werden, die formal durch Reduktion von Acrylsäure erhältlich sind; bekannte C₃-Vorläufer von Acrylsäure sind z. B. Propan, Propen, Acrolein, Propionaldehyd und Propionsäure; der Begriff soll aber auch Vorläuferverbindungen der vorgenannten Verbindungen umfassen, wie z. B. Glycerin (von Glycerin ausgehend kann Acrylsäure beispielsweise durch heterogen katalysierte oxidative Dehydratisierung in der Gasphase erzeugt werden; vgl. z. B. EP-A 1 710 227, WO 06/114506 und WO 06/092272) mit molekularem Sauerstoff an im festen Aggregatzustand befindlichen Katalysatoren bei erhöhter Temperatur erhältlich.

Dabei werden die genannten Ausgangsgase, in der Regel mit inerten Gasen wie z. B. Stickstoff, CO₂, gesättigten Kohlenwasserstoffen und/oder Wasserdampf verdünnt, im Gemisch mit molekularem Sauerstoff bei erhöhten Temperaturen sowie gegebenenfalls erhöhtem Druck über (z. B. übergangsmetallische) Mischoxidkatalysatoren geleitet und oxidativ in ein Acrylsäure, Wasser sowie unerwünschten Nebenprodukte wie z. B. Furfurale, Benzaldehyd, Aceton, Formaldehyd und Maleinsäureanhydrid etc. enthaltendes Produktgasgemisch umgewandelt, aus welchem die Acrylsäure abgetrennt werden muss (die Nebenprodukte und die von Wasserdampf verschiedenen inerten Verdünnungsgase sollen in dieser Schrift unter dem Begriff "Nebenkomponenten" zusammengefasst werden; außerdem soll dieser Begriff die bei den Acrylsäureabtrennverfahren üblicherweise zugesetzten Polymerisationsinhibitoren umfassen).

Von Propionaldehyd und/oder Propionsäure ausgehend handelt es sich bei der heterogen katalysierten Gasphasen-Partialoxidation mit molekularem Sauerstoff wenigstens teilweise um eine oxidative Dehydrierung.

Aus den Schriften DE-A 199 24 533, DE-A 199 24 532, WO 01/77056, DE-A 101 56 016, DE-A 102 43 625, DE-A 102 23 058, DE-A 102 35 847, WO 2004/035514, WO 00/53560 und DE-A 103 32 758 sind wie eingangs beschriebene Verfahren zur Herstellung von Acrylsäure bekannt, bei denen eine Grundabtrennung einer rohen Acrylsäure durch fraktionierende Kondensation des Produktgasgemisches der heterogen katalysierten Gasphasen-Partialoxidation vorgenommen wird. Der Begriff rohe Acrylsäure bzw. Rohacrylsäure bringt dabei zum Ausdruck, dass es sich bei der über den ersten Seitenabzug entnommenen Acrylsäure um kein reines Produkt, sondern um ein Gemisch handelt, das neben Acrylsäure (in der Regel ≥ 50 oder ≥ 60 Gew.-%, meist ≥ 70 oder ≥ 80 Gew.-%, vielfach ≥ 90 Gew.-% und häufig ≥ 95 Gew.-% oder mehr des Gesamtgewichtes) noch Wasser und Nebenkomponenten wie z. B. niedere Aldehyde (z. B. Furfurale, Acrolein, Benzaldehyd), niedere Carbonsäuren (z. B. Essigsäure, Propionsäure, Ameisensäure) etc. enthält. In jedem Fall ist der Gesamtgehalt an Wasser und Nebenkomponenten, bezogen auf den Gehalt an Acrylsäure, in der rohen Acrylsäure geringer als im Produktgasgemisch der Gasphasen-Partialoxidation, weshalb man auch sagt, dass die rohe Acrylsäure diese Bestandteile insgesamt abgereichert enthält (einzelne Bestandteile können hingegen in der rohen Acrylsäure vergleichsweise angereichert enthalten sein).

Weiterhin ist aus der WO 03/095411 A1 u.a. ein Verfahren zur Herstellung von Acrylsäure und/oder deren Ester bekannt, bei dem im Aufarbeitungsprozess aus dem Acrylsäure und Nebenkomponenten enthaltenden Produktgasgemisch einer heterogen katalysierten Gasphasenpartialoxidation sowohl eine Zielproduktacrylsäurequalität als auch ein Acrylsäure Neben-Stoffgemisch abgetrennt werden. Allerdings erfolgt die Zielproduktabtrennung aus dem Produktgasgemisch absorptiv, wobei zusätzlich der gesamte organische Extrakt in die Absorptionskolonne rezykliert wird.

Teilweise ist die Reinheit der so abgetrennten rohen Acrylsäure bereits für den ins Auge gefassten Verwendungszweck der Acrylsäure ausreichend (z. B. zum Zweck der Veresterung derselben, oder zum Zweck des Aufbaus von durch radikalische Polymerisation erhältlichen Polymerisaten). Vielfach wird man die abgetrennte rohe Acrylsäure jedoch wenigstens einem weiteren thermischen Trennverfahren unterwerfen, um aus der rohen Acrylsäure eine reinere (eine einen im Vergleich zur rohen Acrylsäure höheren Acrylsäuregehalt in Gew.-% aufweisende) Acrylsäure zu gewinnen, die den für den jeweiligen Verwendungszweck erforderlichen Reinheitsgrad aufweist.

Unter thermischen Trennverfahren werden dabei solche verstanden, bei denen unter Zufuhr oder unter Entzug von (in der Regel thermischer) Energie ein physikalisch wenigstens zweiphasiges System erzeugt wird, wobei es infolge der zwischen den Phasen bestehenden Temperatur- und Stoffmengengradienten zu einem Wärme- und Stoffaustausch kommt, der letztlich die gewünschte Auftrennung bedingt, und die Extraktion.

Häufig werden thermische Trennverfahren in trennwirksame Einbauten enthaltenden Trennkolonnen durchgeführt, in denen die vorgenannten wenigstens zwei stofflichen Phasen in der Regel zueinander im Gegenstrom geführt werden. Vielfach ist eine der beiden stofflichen Phasen gasförmig (sie wird in einer Trennkolonne in der Regel als aufsteigende Phase geführt) und die andere flüssig (sie wird in einer Trennkolonne in der Regel als absteigende Phase geführt). Grundsätzlich können die wenigstens zwei stofflichen Phasen aber auch flüssig (z. B. im Fall einer Extraktion) oder fest und flüssig (z. B. im Fall einer Kristallisation), oder fest und gasförmig (z. B. im Fall einer Adsorption) sein.

Beispiele für Fallgestaltungen thermischer Trennverfahren, bei denen eine der wenigstens zwei stofflichen Phasen flüssig und eine gasförmig ist, und damit natürliches Element des in dieser Schrift verwendeten Begriffs "thermische Trennverfahren", sind die Rektifikation (eine aufsteigende Dampfphase wird in der Trennkolonne im Gegenstrom zu einer absteigenden Flüssigphase geführt), und die Desorption (der Umkehrprozess zur Absorption; das in einer Flüssigphase gelöste Gas wird durch Erniedrigung des Drucks über der Flüssigphase, durch Erhöhung der Temperatur der Flüssigphase und/oder durch Hindurchführen einer Gasphase durch die Flüssigphase aus der Flüssigphase herausgeführt; ist die Hindurchführung einer Gasphase beteiligt, wird die Desorption auch als Strippung bezeichnet). Aber auch die Absorption (in der Regel wird ein in einer Trennkolonne aufsteigendes Gas zu wenigstens einem in der Trennkolonne flüssig absteigenden Absorptionsmittel im Gegenstrom geführt) und die fraktionierende Kondensation eines Gasgemischs (Gas-/Flüssigphase Beispiel) sind Bestandteil des Begriffs thermisches Trennverfahren. Ein besonders günstiges thermisches Trennverfahren zur Weiterreinigung von roher Acrylsäure ist die kristallisative Weiterreinigung (die Kristallisation).

Nachteilig an den bekannten Verfahren zur Grundabtrennung einer rohen Acrylsäure durch fraktionierende Kondensation des Produktgasgemischs einer heterogen katalysierten Gasphasen-Partialoxidation wenigstens eines C₃-Vorläufers der Acrylsäure ist jedoch der zusätzliche Anfall von noch Acrylsäure und Nebenkomponenten enthaltendem saurem Wasser (vereinfacht auch "Sauerwasser" genannt). Die Bezeichnung "Sauerwasser" bringt dabei zunächst zum Ausdruck, dass das Sauerwasser in der Regel ≥ 50 Gew.-%, häufig ≥ 60 Gew.-%, vielfach ≥ 70 Gew.-% und oft ≥ 80 Gew.-% an Wasser enthält (dabei handelt es sich in der Regel sowohl um Reaktionswasser, als auch um als inertes Verdünnungsgas im Rahmen der Gasphasen-Partialoxidation mitverwendetes Verdünnungswasser (Wasserdampf)).

Sie bringt aber auch zum Ausdruck, dass es neben Wasser noch Nebenkomponentensäuren wie z. B. Propionsäure, Essigsäure und Ameisensäure sowie Acrylsäure enthält und damit einen pH-Wert von < 7 aufweist (der Gesamtgehalt der von Acrylsäure verschiedenen Nebenkomponentencarbonsäuren liegt in der Regel, bezogen auf das Gewicht des Sauerwassers, bei Werten ≤ 10 Gew.-%, teilweise bei Werten ≤ 5 Gew.-%).

Normalerweise wird der Acrylsäuregehalt des Sauerwassers 4 oder 5 bis 15, häufig ca. 10 Gew.-% betragen. Nachteilig an den im zitierten Stand der Technik empfohlenen Verfahren zur Grundabtrennung von Acrylsäure aus dem Produktgasgemisch der heterogen katalysierten Gasphasen-Partialoxidation ist, dass sie das nicht in die Rektifikationskolonne rückgeführte noch Acrylsäure enthaltende Sauerwasser in seiner Gesamtheit der Verbrennung zuführen (vgl. insbesondere DE-A 102 43 625, WO 2004/035514 und DE-A 103 32 758).

Dies ist insofern von Nachteil, als die Sauerwasserverbrennung z. B. die Ausbeute am gewünschten Produkt Acrylsäure mindert.

Die Aufgabe der vorliegenden Erfindung bestand angesichts des beschriebenen Standes der Technik darin, ein verbessertes Verfahren zur Herstellung von Acrylsäure zur Verfügung zu stellen, das insbesondere dadurch ausgezeichnet ist, dass es eine erhöhte Ausbeute an Acrylsäure gewährleistet, ohne die Reinheit derselben nennenswert zu beeinträchtigen.

Demgemäß wurde ein Verfahren zur Herstellung von Acrylsäure, bei dem man durch heterogen katalysierte Gasphasen-Partialoxidation wenigstens eines C₃-Vorläufers (einer C₃-Vorläuferverbindung) der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, die Temperatur des Acrylsäure, Wasserdampf und Nebenkomponenten enthaltenden Produktgasgemischs gegebenenfalls durch direkte (durch direkten Kontakt mit einer Kühlflüssigkeit) und/oder indirekte Kühlung verringert, und das Acrylsäure, Wasserdampf und Nebenkomponenten enthaltende Produktgasgemisch anschließend in eine mit trennwirksamen Elementen ausgerüstete Kondensationskolonne leitet, innerhalb der Kondensationskolonne in sich selbst aufsteigen lässt und dabei fraktionierend kondensiert sowie über einen ersten, Oberhalb der Zuführstelle des Produktgasgemischs in die Kondensationskolonne befindlichen, Seitenabzug eine Wasser und Nebenkomponenten insgesamt entreichert enthaltende rohe Acrylsäure als Zielprodukt und über einen zweiten, oberhalb das ersten Seitenabzugs befindlichen, Flüssigphasenabzug (vorzugsweise einen Seitenabzug; alle Aussagen in dieser Schrift sind insbesondere gültig im Fall eines solchen Sauerwasserseitenabzugs) noch Acrylsäure und Nebenkomponenten enthaltendes saures Wasser (Sauerwasser) und am Kopf der Kondensationskolonne ein tiefer (bei niedrigerer Temperatur (bezogen auf Atmosphärendruck)) als Wasser siedende Nebenkomponenten enthaltendes Restgasgemisch sowie aus dem Sumpfraum der Kondensationskolonne eine noch Acrylsäure und schwerer als Acrylsäure siedende Folgeprodukte und Nebenkomponenten enthaltende Sumpfflüssigkeit aus der Kondensationskolonne herausführt, eine Teilmenge des entnommenen sauren Wassers als solches und/oder nach Abkühlung desselben als Rücklaufflüssigkeit in die Kondensationskolonne rückführt und die rohe Acrylsäure gegebenenfalls wenigstens einem weiteren thermischen Trennverfahren zum Zweck ihrer Weiterreinigung unterwirft, gefunden, das dadurch gekennzeichnet ist, dass man wenigstens bei einer Teilmenge von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes aus dem sauren Wasser heraus in das organische Lösungsmittel aufnimmt, nachfolgend die Acrylsäure aus dem organischen Extrakt durch Strippen mit einem ersten Strippgas abtrennt und das mit Acrylsäure beladene erste Strippgas in die Kondensationskolonne rückführt und/oder die im ersten beladenen Strippgas enthaltene Acrylsäure in die wässrige Lösung eines Metallhydroxids aufnimmt oder das dabei anfallende mit Acrylsäure beladene erste Strippgas als ein zweites Strippgas dazu verwendet, um in aus der Kondensationskolonne herausgeführter Sumpfflüssigkeit noch enthaltene Acrylsäure freizustrippen und das dabei anfallende mit Acrylsäure beladene zweite Strippgas in die Kondensationskolonne rückführt und/oder die im zweiten Strippgas enthaltene Acrylsäure in die wässrige Lösung eines Metallhydroxids aufnimmt.

Erfindungsgemäß vorteilhaft wird wenigstens 25 Gew.-%, besser wenigstens 50 Gew.-%, noch besser wenigstens 75 Gew.-% und bevorzugt die Gesamtmenge des nicht in die Kondensationskolonne rückgeführten Sauerwassers erfindungsgemäß extrahiert und weiterbehandelt.

Grundsätzlich kommen zur Durchführung der erfindungsgemäß erforderlichen Extraktion alle für Flüssig-Flüssig Extraktionen bekannten Extraktiorisapparate in Betracht.
Dieser sollte möglichst die Erzeugung einer großen Phasengrenzfläche und eine feine Verteilung dieser Tropfen in der kontinuierlichen Phase sowie eine schnelle und möglichst vollständige Phasentrennung nach erfolgter Stoffübertragung gewährleisten.

Im einfachsten Fall kann als eine Extraktionseinheit ein Mischer (z. B. ein Rührbehälter oder ein statischer Mischer) mit Abscheider (z. B. ein Absetzbehälter) verwendet werden. Als Rührer kommen prinzipiell alle gängigen Rührer in Betracht. Beispielhaft genannt seien Scheibenrührer, Impellerrührer, Kreuzbalkenrührer, Gitterrührer, Blattrührer, Ankerrührer, Schaufelrührer, Propellerrührer, Wendelrührer und Mehrstufen-Impuls-Gegenstrom-Rührer. Die Rührer können auch mehrstufig sein, das heißt, mehrere Rührer sind an einer gemeinsamen Achse übereinander angebracht. Vorzugsweise wird ein zweistufiger Impellerrührer verwendet. Als Absetzbehälter kommen prinzipiell alle gängigen Behälter in Betracht. Vorzugsweise wird ein liegender Behälter verwendet. Eine solche Extraktionseinheit kann kontinuierlich oder diskontinuierlich betrieben werden, wobei im diskontinuierlichen Betrieb der Absetzbehälter entfallen kann und die Phasentrennung im Rührbehälter durchgeführt wird. Der Übergang des zu extrahierenden Stoffes (des Wertstoffes) in die Aufnehmerphase erfolgt im Mischer, die Trennung der beiden Phasen (durch die Schwerkraft) im Abscheider. Im kontinuierlichen Betrieb kann im Absetzbehälter die Auftrennung des Zulaufes in eine schwere und leichte Phase durch Einbauten quer zur Strömungsrichtung verbessert werden. Als Einbauten kommen prinzipiell alle gängigen Einbauten in Betracht, beispielsweise Lochbleche, Platten, Packungen und/oder Schüttungen. Von den Schüttungen sind solche mit Ringen, Wendeln, Sattelkörpern, Raschig-, Intos- oder Pall-Ringen, Barreloder Intalox-Satteln, Top-Pak etc. oder Geflechten bevorzugt. Besonders bevorzugt sind Lochbleche in Kombination mit Schüttungen. Die Verweilzeit im Absetzbehälter beträgt typisch 0,05 bis 2 h.

Schaltet man mehrere Mischer-Abscheider-Einheiten hintereinander, so spricht man von einer Kaskade. Die Einheiten können sowohl im Gegen- wie auch im Gleich- bzw. Kreuzstrom geschaltet sein.

Anwendungstechnisch zweckmäßig wird man die erfindungsgemäß durchzuführende Extraktion in einer trennwirksame Einbauten enthaltenden Extraktionskolonne vornehmen. Die spezifisch schwerere Phase tritt oben und die spezifisch leichtere Phase unten in die Kolonne ein. In der Kolonne bewegen sich beide Phasen im Gegenstrom. Prinzipiell gleichen Extraktionskolonnen den Kolonnen für eine Gegenstromdestillation (Rektifikation). D. h., für das erfindungsgemäße Extraktionsverfahren geeignete Extraktionskolonnen können von an sich bekannter Bauart sein und die üblichen Einbauten aufweisen.

Als mögliche Kolonnentypen kommen sowohl Extraktionskolonnen mit als auch ohne Energieeintrag in Betracht. Extraktionskolonnen, die Einbauten in Form von Packungen (insbesondere strukturierte bzw. geordnete) und/oder Schüttungen enthalten, können sowohl mit als auch ohne Energieeintrag betrieben werden. Unter den Schüttungen sind solche mit Ringen, Wendeln, Sattelkörpern, Raschig-, Intos- oder Pall-Ringen, Barrel- oder Intalox-Sätteln, Top-Pak etc. bevorzugt. Für erfindungsgemäß zu verwendende Extraktionskolonnen besonders geeignete Packungen sind z. B. Packungen der Julius Montz GmbH in D-40705 Hilden, wie z. B. die Packung Montz-Pak B1-350. Vorzugsweise verwendet man gelochte strukturierte Packungen aus Edelstahlblechen. Packungskolonnen mit geordneten Packungen sind dem Fachmann an sich bekannt und z. B. in Chem.-Ing.Tech. 58 (1986) Nr. 1, S. 19 - 31 sowie in der Technischen Rundschau Sulzer 2/1979, S. 49 ff. der Gebrüder Sulzer Aktiengesellschaft in CH-Winterthur beschrieben. Desweiteren sind auch Extraktionskolonnen mit Einbauten in Form von Böden geeignet, wobei hier zwischen pulsierten Siebbodenkolonnen und Querstromsiebbodenkolonnen unterschieden werden muss. Bei den pulsierten Siebbodenkolonnen werden beide Phasen durch die Durchtrittsöffnungen (in der Regel Löcher, d. h., kreisrunde Durchtrittsöffnungen) im Siebboden geführt. Beim Aufwärtshub der Pulsation wird die leichtere Phase nach oben durch die Löcher des Siebbodens gedrückt, beim Abwärtshub entsprechend die schwere Phase nach unten. Ähnlich arbeitet auch die Karr-Kolonne, nur dass hier nicht die Flüssigkeit pulsiert wird, sondern die Siebböden auf und ab bewegt werden. Die pulsierte Siebbodenkolonne wird bevorzugt. Bei Verwendung von Querstromsiebbodenkolonnen läuft dagegen die kontinuierliche Phase über Ablaufschächte von einem Boden zum nächsten Boden und nur die disperse Phase wird auf Grund der Dichtedifferenz durch die Löcher der Siebböden gedrückt (die in dieser Schrift für Stoffaustauschböden verwendeten Bezeichnungen orientieren sich an jenen der DE-A 103 32 758).

In der Regel sind für die erfindungsgemäß durchzuführende Sauerwasserextraktion 1 bis 10 theoretische Böden ausreichend. Als theoretischer Boden (oder theoretische Trennstufe) soll in dieser Schrift ganz generell diejenige Raumeinheit einer für ein thermisches Trennverfahren eingesetzten, trennwirksame Einbauten enthaltenden, Trennkolonne verstanden werden, die eine Stoffanreicherung entsprechend dem thermodynamischen Gleichgewicht bewirkt. D. h., der Begriff des theoretischen Bodens ist sowohl auf Extraktionskolonnen mit Stoffaustauschböden, als auch auf Extraktionskolonnen mit Packungen und/oder Füllkörpern anwendbar.

Verwendet man für das erfindungsgemäße Verfahren eine Extraktionskolonne, wird man die spezifisch schwerere der beiden Phasen anwendungstechnisch zweckmäßig mittels Verteiler oben in die Kolonne, über den Querschnitt möglichst gleichmäßig verteilt, aufgeben. Die spezifisch leichtere Phase geht anwendungstechnisch zweckmäßig ebenfalls über einen Verteiler unten in die Kolonne hinein. Dementsprechend steigt in der Kolonne die leichtere Phase auf und die schwere Phase sinkt nach unten. Wird die leichtere der beiden Phasen dispergiert, liegt diese also in Tropfenform vor, so findet die Phasentrennung im Kopf der Kolonne statt, im umgekehrten Fall, bei dem die schwerere Phase dispergiert wird, findet die Phasentrennung im Sumpf der Kolonne statt. Günstige Tropfengrößen haben in beiden Fällen einen Durchmesser (eine Längstausdehnung) im Bereich von 1 bis 10 mm, vorzugsweise im Bereich von 2 bis 5 mm.

Erfindungsgemäß vorteilhaft wird das Extraktionsmittel einen höheren Siedepunkt als Acrylsäure (jeweils bezogen auf Atmosphärendruck) haben, da dies in der Regel die nachfolgende Abtrennung der Acrylsäure aus dem organischen Extrakt erleichtert. Im Rahmen der erfindungsgemäß durchzuführenden Extraktion wird es deshalb mit erhöhter Wahrscheinlichkeit so sein, dass das als Extraktionsmittel zu verwendende organische Lösungsmittel eine nennenswert höhere Viskosität als Wasser hat. In diesem Fall ist es erfindungsgemäß vorteilhaft, wenn das in die Extraktionskolonne eintretendende organische Extraktionsmittel als disperse Phase vorliegt und das Sauerwasser als kontinuierliche Phase (dies bedingt z. B. einen beschleunigten Stoffaustausch zwischen den beiden Phasen und ermöglicht bei gleichem Trennergebnis letztlich kürzere Kolonnen; auch benetzt eine kontinuierliche wässrige Phase aus Edelstahl gefertigte Extraktionskolonnen und deren Einbauten besser; darüber hinaus führt ein Transport des zu extrahierenden Stoffes aus der kontinuierlichen Phase in die disperse Phase zu einer Stabilisierung der letzteren (geringere Koaleszenzneigung)). Bei Verwendung eines organischen Extraktionsmittel mit einer höheren Massendichte als der Massendichte des Sauerwassers bedeutet dies, dass das Extraktionsmittel am Kopf der Kolonne aufgegeben und dispergiert wird und die dabei resultierenden Extraktionsmitteltropfen in der Kolonne nach unten sinken. Im umgekehrten Fall, d.h., bei der Verwendung eines Extraktionsmittels mit einer geringeren Massendichte als der Massendichte von Sauerwasser, wird das Extraktionsmittel im Sumpf der Kolonne dispergiert und die dabei resultierenden Extraktionsmitteltropfen steigen in der Kolonne auf. Bei den bisher genannten Typen von Extraktionskolonnen mit Einbauten in Form von Packungen, Füllkörpern und/oder Böden sollte die unzerteilte kontinuierliche Phase die ausgewählten Einbauten gut benetzen, da andernfalls die Tropfen der dispersen Phase in der Regel an den Einbauten entlang kriechen.

In einfachster Weise wird man das organische Extraktionsmittel dabei mittels über den Kolonnenquerschnitt angeordnete und sich über die jeweilige Querschnittslänge der normalerweise kreiszylindrischen Extraktionskolonne erstreckende, in der Regel kreisförmige Durchtrittsöffnungen (Bohrungen) aufweisende Rohre (die normalerweise einen identischen Querschnitt aufweisen; man spricht auch von Rohrverteilern) aufgeben. Wird das organische Extraktionsmittel am Kopf der Kolonne aufgegeben weisen die kreisförmigen Durchtrittsöffnungen nach unten, bei Aufgabe des Extraktionsmittels am Sumpf der Kolonne nach oben. Der Durchmesser (die Längstausdehnung) der vorgenannten Durchtrittsöffnungen wird dabei üblicherweise 1 mm bis 10 mm, vorzugsweise 3 mm bis 6 mm und vielfach 2 bis 5 mm betragen. Dabei lässt man das Extraktionsmittel in einfacher Weise in die Verteilerrohre ein- und aus den Durchtrittsöffnungen wieder ausströmen.

Grundsätzlich können für die erfindungsgemäß auszuführende Sauerwasserextraktion auch gerührte Kolonnen oder Zentrifugalextraktoren eingesetzt werden. Gerührte Kolonnen verbessern die Berührung beider Phasen. Alle Rührer der Kolonne sitzen anwendungstechnisch zweckmäßig auf einer gemeinsamen Welle. Das Kolonnenrohr ist an den Wänden zweckmäßig mit Statorringen ausgerüstet. Die in der Regel in der Mitte angeordnete Welle trägt in typischer Weise Rührorgane in der Art, dass jeweils zwischen zwei Statorringen ein Rührer rotiert. Beispielhafte zu nennen sind für die gerührten Kolonnen die RDC(rotating disc contactor)-Kolonne, die ARD(asymmetrical rotating disc)-Kolonne, die Kühni-Kolonne (gerührte Kolonne gemäß Bauart nach Kühni) oder der QVF-Rührzellenextraktor. Zentrifugalextraktoren nutzen die Zentrifugalkraft zum Vermischen und Trennen der beiden im Gegenstrom geführten Phasen. Die Zentrifugalkraft erbringt auch dann eine gute Raffinat-/Extrakttrennung, wenn beide Phasen zur Bildung einer stabilen Emulsion neigen. Beispielhaft zu nennen sind hier die Apparate Podbielnak-Extraktor oder der Westfalia-Separator.

Gefertigt wird die Extraktionseinheit für das erfindungsgemäße Verfahren vorzugsweise aus dem Werkstoff 1.4571. Dies trifft so auch auf die übrigen Vorrichtungen zu, die für die Abtrennung der Acrylsäure vom Produktgasgemisch der heterogen katalysierten Gasphasen-Partialoxidation eingesetzt werden können.

Triebkraft für die Trennung von Extrakt und Raffinat ist der Unterschied in der Massendichte (g/cm³) zwischen beiden Phasen. Eine hohe Massendichtedifferenz der beiden flüssigen Phasen erleichtert die Phasentrennung und mindert Emulsionsbildung.

Mit Vorteil werden für die erfindungsgemäß durchzuführende Extraktion daher organische Lösungsmittel verwendet, deren Massedichte in kg/m³ sich von der Massendichte von Wasser (ebenfalls in kg/m³) um ≥ 25 kg/m³, vorzugsweise um ≥ 50 kg/m³ unterscheidet (bezogen auf den bei der Extraktion angewandten Druck und die bei der Extraktion angewandte Temperatur). In der Regel wird der vorgenannte Massendichteunterschied jedoch ≤ 250 kg/m³, in der Regel ≤ 150kg/m³ betragen.

Desweiteren ist es für das erfindungsgemäße Verfahren günstig, wenn die dynamische Viskosität des organischen Extraktionsmittels unter den Extraktionsbedingungen ≤ 100 mPa·s, vorzugsweise ≤ 50 mPa·s beträgt. In der Regel wird die vorgenannte dynamische Viskosität jedoch ≥ 1 mPa·s betragen. Erfindungsgemäß besonders günstig sind dynamische Viskositäten im Bereich von 2 bis 10 mPa·s.

Weiterhin ist es beim erfindungsgemäßen Verfahren vorteilhaft, wenn die Grenzflächenspannung zwischen den beiden fluiden Phasen vergleichsweise hoch ist.
Vor dem Hintergrund des bisher gesagten kommen als erfindungsgemäß geeignete Extraktionsmittel für die Sauerwasserextraktion unter anderem organische Flüssigkeiten in Betracht, deren Siedepunkt bei Normaldruck (1 atm) oberhalb von 150 bzw. oberhalb von 160 °C liegt. Beispielhaft genannt seien Mittelölfraktionen aus der Paraffindestillation, Diphenylether, Diphenyl, oder Mischungen der vorgenannten Flüssigkeiten, wie z. B. ein Gemisch aus 70 bis 75 Gew.-% Diphenylether und 25 bis 30 Gew.-% Diphenyl. Günstig ist auch die Verwendung eines Gemisches bestehend aus einer Mischung aus 70 bis 75 Gew.-% Diphenylether und 25 bis 30 Gew.-% Diphenyl, sowie bezogen auf des Gemisch, 0,1 bis 25 Gew.-% o-Dimethylphthalat.

Erfindungsgemäß besonders bevorzugte organische Lösungsmittel für die Sauerwasserextraktion sind die Ester von aliphatischen oder aromatischen Mono- oder Dicarbonsäuren (insbesondere wenn beide Carboxylgruppen verestert sind), deren alkoholische Komponente 1 bis 8 C-Atome und deren Carbonsäurekomponente 5 bis 20 C-Atome enthält. Vorzugsweise weist die alkoholische Komponente vor der Veresterung lediglich zwei oder nur eine Hydroxylgruppe auf. Besonders bevorzugt handelt es sich bei der alkoholischen Komponente um einwertige (eine OH-Gruppe) oder um zweiwertige (zwei OH-Gruppen) Alkanole. Mit Vorteil beträgt die Anzahl der C-Atome der alkoholischen Komponente (insbesondere im Fall von einwertigen oder zweiwertigen Alkanolen) 1 bis 6, besonders bevorzugt 1 bis 4 und ganz besonders bevorzugt 1 oder 2. Die aliphatischen oder aromatischen Mono- oder Dicarbonsäuren enthalten mit Vorteil 5 bis 15 C-Atome, bevorzugt 5 bis 10 C-Atome und besonders bevorzugt 6 bis 8 C-Atome (insbesondere im Fall einer jeweiligen Veresterung (auch im Diesterfall) mit 1 bis 4 bzw. mit 1 oder 2 C-Atome aufweisenden Alkanolen). Dicarbonsäuren sind gegenüber Monocarbonsäuren als Säurekomponente der relevanten Ester bevorzugt (insbesondere wenn beide Carboxylgruppen verestert sind). Phthalsäure, isoPhthalsäure und Terephthalsäure sowie Adipinsäure sind erfindungsgemäß ganz besonders bevorzugte Säurekomponenten der relevanten Ester. Das letztere trifft insbesondere im Fall der Dialkylester (C₁- bis C₈-Alkyl, vorteilhaft C₁-bis C₆-Alkyl, ganz besonders vorteilhaft C₁- bis C₄-Alkyl und noch besser C₁- oder C₂-Alkyl) zu. D. h., für das erfindungsgemäße Verfahren ganz besonders günstige Extraktionsmittel sind das Dimethylphthalat, das Diethylphthalat (z. B. Palatinol^{®} A der BASF Aktiengesellschaft), das Dimethylisophthalat, das Diethylisophthalat, das Dimethylterephthalat, das Diethylterephthalat, der Adipinsäuredimethylester und der Adipinsäurediethylester. Weitere für die erfindungsgemäße Sauerwasserextraktion geeignete Ester sind die Triester der Phosphorsäure, wie z. B. Tributylphosphat oder Trikresylphosphat. Als Kresylrest kommen dabei sowohl das ortho-Kresyl, das meta-Kresyl- und das para-Kresyl in Betracht. Außerdem kommen als Extraktionsmittel für die erfindungsgemäße Sauerwasserextraktion Ester aus Acrylsäure und verzweigten oder linearen einwertigen C₆- bis C₁₂-Alkanolen (z. B. 2-Propylheptylacrylat oder 2-Ethylhexylacrylat) sowie Mono- und Diester aus Maleinsäure und einwertigen C₄- bis C₁₀-Alkanolen in Betracht. Dabei gilt auch für alle vorgenannten Extraktionsmittel, dass diejenigen unter ihnen, die bei Normaldruck einen Siedepunkt oberhalb von 150 °C bzw. oberhalb von 160 °C, oder oberhalb von 170 °C, oder oberhalb von 180 °C, oder oberhalb von 190 °C aufweisen, erfindungsgemäß bevorzugt werden.

In der Regel wird das zu extrahierende Sauerwasser neben Acrylsäure und Wasser als weiteren Bestandteil (in der Regel in Gew.-% bezogen auf die Gesamtmenge des Sauerwassers der drittgrößte Bestandteil) Essigsäure enthalten. Je nach Art und Weise der durchgeführten Partialoxidation (ausgewähltem Katalysator, Wasserdampfgehalt des Reaktionsgasgemischs, Temperatur der Partialoxidation) kann das Sauerwasser bis zu 10 Gew.-%, bzw. bis zu 5 Gew.-% (häufig 2 bis 8 Gew.-%) oder mehr an Essigsäure enthalten. Häufig enthält das Sauerwasser etwa den 2-fachen Gewichtsanteil an Acrylsäure bezogen auf den Gewichtsanteil an Essigsäure. Die Gehalte der anderen möglichen sauren Nebenkomponenten liegen normalerweise signifikant tiefer. Erfindungsgemäß bevorzugt sind daher jene Extraktionsmittel, die die Acrylsäure im Vergleich zur Essigsäure bevorzugt aufnehmen. Zu diesen Extraktionsmitteln zählt im besonderen der Phthalsäurediethylester.

Erfindungsgemäß vorteilhaft ist es auch, wenn das Extraktionsmittel sich unter den Extraktionsbedingungen nicht mit Wasser umsetzt und in Wasser nur eine geringe Löslichkeit aufweist. So ist z. B. der Phthalsäurediethylester besonders hydrolysestabil. Ein weiterer Vorteil des Phthalsäurediethylesters ist sein bei Normaldruck (1 atm) vergleichsweise hoher Siedepunkt, der erfindungsgemäß vorteilhaft für zu verwendende Extraktionsmittel (organische Lösungsmittel) ≥ 200°C, besser ≥ 225°C und noch besser ≥ 250°C beträgt.

Zusätzlich weist er eine vergleichsweise geringe Löslichkeit in Wasser auf (dies mindert auch die Extraktionsmittelverluste). In der Regel fällt das Sauerwasser bei der erfindungsgemäß durchzuführenden fraktionierenden Kondensation des Produktgasgemischs mit einer Temperatur von 50 bis 80°C, vorzugsweise 60 bis 70°C an. D. h., mit dieser Temperatur wird es normalerweise über den zweiten Flüssigphasenabzug (vorzugsweise einen Seitenabzug) entnommen (je tiefer die Temperatur, desto geringer ist der Bedarf an Polymerisationsinhibitor; in günstigen Fällen bedarf es nicht des separaten Zusatzes eines solchen ins Sauerwasser, Extraktionsmittel, Raffinat und/oder Extrakt). Anwendungstechnisch zweckmäßig wird man daher auch die Extraktion in diesem Temperaturbereich durchführen. D. h., erfindungsgemäß vorteilhaft wird man das Sauerwasser im wesentlichen mit seiner vorgenannten Temperatur in die Extraktionseinheit, vorzugsweise eine Extraktionskolonne (besonders bevorzugt eine Packungskolonne, vorteilhaft Montz-Pak B1-350) führen. Mit Vorteil erfolgt die Zufuhr von unten in die Extraktionskolonne und von oben wird das spezifisch schwerere Extraktionsmittel (mit Vorteil Diethylphthalat) aufgegeben. Üblicherweise wird die Temperatur des aufgegebenen Extraktionsmittels von jener des zugeführten Sauerwassers nicht sehr verschieden sein. In typischer Weise beträgt der Betrag dieses Temperaturunterschieds ≥ 0°C und ≤ 20°C, vorzugsweise ≥ 0°C und ≤ 15°C sowie vielfach ≥ 0°C und ≤ 10°C. Der Druck des der Kondensationskolonne entnommenen Sauerwassers beträgt an der Entnahmestelle erfindungsgemäß typisch > 1 bis 1,5 bar, häufig 2 bar. Das entnommene Sauerwasser wird mittels einer Pumpe in die Extraktionskolonne geführt. Der Förderdruck kann z. B. 2 bis 6 bar betragen. Der Arbeitsdruck in der Extraktionskolonne wird erfindungsgemäß so gewählt, dass es keiner zusätzlichen Pumpe bedarf, um das organische Extrakt in die erste Strippkolonne zu fördern. Grundsätzlich kann die Sauerwasserextraktion aber auch bei höheren oder tieferen Temperaturen sowie bei höheren oder niedrigeren Drucken durchgeführt werden. Bei der Inbetriebnahme einer Extraktionskolonne wird man anwendungstechnisch zweckmäßig so vorgehen, dass man die Extraktionskolonne zunächst mit Sauerwasser füllt und anschließend, wie bereits beschrieben, das organische Extraktionsmittel vorteilhaft am Kopf der Extraktionskolonne tropfenförmig aufgibt. Die Zufuhr des Sauerwassers (der bevorzugt kontinuierlichen Phase) kann prinzipiell unmittelbar über einen entsprechenden Zufuhrstutzen erfolgen. Grundsätzlich kann das Sauerwasser aber auch über ein ein (oder mehrere) Durchtrittsöffnungen in seiner Wandung aufweisendes Zufuhrrohr (Durchmesser der Durchtrittsöffnungen liegen bei typisch 5 bis 10 mm) zugeführt werden.

Wie bereits erwähnt, wird die Extraktionskolonne anwendungstechnisch zweckmäßig aus Edelstahl gefertigt. Typische Wanddicken betragen 5 bis 20 mm. Nach außen ist die Extraktionskolonne normalerweise in herkömmlicher Weise thermisch isoliert.

Das Verhältnis V der der Extraktionskolonne zugeführten Mengenströme an organischem Extraktionsmittel (E; in kg/h) und saurem Wasser (S; in kg/h), d. h., E:S, kann beim erfindungsgemäßen Verfahren 0,05 bis 20, vorzugsweise 0,1 bis 10, besser 0,8 bis 1,2 und besonders bevorzugt 1:1 betragen.

Das an Acrylsäure ausgelaugte (extrahierte, abgereicherte) Sauerwasser wird normalerweise seiner Entsorgung zugeführt (z. B. verbrannt oder in die Kläranlage gefahren). Es verlässt die Extraktionskolonne erfindungsgemäß typisch an deren Kopf (als Raffinat), während das organische, die Acrylsäure enthaltende, Extrakt die Extraktionskolonne in typischer Weise unten verlässt.

Die Abtrennung der Acrylsäure aus dem organischen Extrakt, dessen Entnahmetemperatur aus der Extraktionskolonne im wesentlichen der Zufuhrtemperatur des Sauerwassers in die Extraktionskolonne entspricht, kann grundsätzlich unter Anwendung unterschiedlicher thermischer Trennverfahren oder auch unter Anwendung von Kombinationen solcher thermischer Trennverfahren vorgenommen werden.

Eine geeignete Abtrennvariante ist die kristallisative Abtrennung. Dabei kommen alle-Kristallisationsverfahren in Betracht, die in der DE-A 19838845 und in der DE-A 10 2005 015 637 empfohlen werden. Der Vorzug der kristallisativen Abtrennung liegt darin begründet, das die Kristallisation im Unterschied zu anderen thermischen Trennverfahren ein scharfes Trennverfahren ist. D. h., die Zusammensetzung der sich bildenden Acrylsäurekristalle ist weitgehend unabhängig (im Idealfall besteht völlige Unabhängigkeit) von der Zusammensetzung der flüssigen Phase, des flüssigen Extrakts. Im Unterschied dazu ist ein unscharfes thermisches Trennverfahren ein solches, bei dem die Zusammensetzung der sich bei Anwendung des Trennverfahrens bildenden, Zielprodukt angereichert enthaltenden, Phase von der Zusammensetzung des zu trennenden Gemischs in ausgeprägter Weise abhängig ist. D. h., beim scharfen Trennverfahren ist in der thermodynamischen Theorie eine einmalige Gleichgewichtseinstellung ausreichend, um das reine Zielprodukt zu erhalten, während es dazu bei unscharfen Trennverfahren einer mehrmaligen aufeinanderfolgend durchgeführten Einstellung des thermodynamischen Gleichgewichtes bedarf. Erfindungsgemäß besonders geeignete Kristallisationsverfahren zum Zweck der Acrylsäureabtrennung aus dem Extrakt sind die Suspensionskristallisation und die Schichtkristallisation.

Erfindungsgemäß ganz besonders vorteilhaft (insbesondere dann, wenn als Extraktionsmittel ein solches verwendet wird, dessen Siedepunkt (bei Normaldruck) wenigstens 60 °C, vorzugsweise wenigstens 80 °C und ganz besonders bevorzugt wenigstens 100 °C oberhalb des entsprechenden Siedepunktes der Acrylsäure (= 141 °C) liegt) wird man die Abtrennung der Acrylsäure aus dem Extrakt durch Desorption vornehmen. Als Desorptionseinheit wird überlicherweise eine Kolonne mit trennwirksamen Einbauten verwendet. Als solche Kolonneneinbauten kommen prinzipiell alle bekannten trennwirksamen Einbauten in Betracht. Dazu zählen insbesondere Böden, Packungen und/oder Schüttungen. Unter den Stoffaustauschböden sind Glockenböden, Siebböden (z. B. Zwangssiebböden bzw. Regensiebböden (Dual-Flovv-Böden), Ventilböden und/oder Thormannböden (bzw. Thormann^{®}-Böden) bevorzugt. Unter den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Ganz besonders bevorzugt sind Dual-Flow-Böden und/oder Thormannböden. Unter den Thormannböden sind die einflutigen Thormannböden bevorzugt. Bei Verwendung von Dual-Flow-Böden und Thormannböden werden die Dual-Flow-Böden ganz besonders bevorzugt im unteren Abschnitt der Desorptionseinheit und die Thormannböden im oberen Abschnitt der Desorptionseinheit eingesetzt. In der Regel sind 5 bis 10 theoretische Böden in der Desorptionseinheit-ausreichend. Grundsätzlich kann die Desorption ausschließlich durch Druckerniedrigung bewirkt werden. Anwendungstechnisch ganz besonders vorteilhaft wird der Partialdruck der zu desorbierenden Acrylsäure durch Verdünnen mit einem unter den Desorptionsbedingungen nicht kondensierbaren (vorzugsweise liegt der Siedepunkt des Strippgases bei 1 atm wenigstens 50 °C unterhalb des Siedepunktes von Wasser) und (gegenüber chemischer Veränderung) im wesentlichen inerten Gas gesenkt. D. h., ganz besonders bevorzugt wird die Abtrennung der Acrylsäure aus der Extraktphase der Sauerwasserextraktion durch Strippen mittels eines Gases (durch Herausstrippen mit einem Gas) vorgenommen. D. h., durch das Durchleiten eines Gases durch das Extrakt wird die Acrylsäure in das Gas aufgenommen und aus dem Extrakt herausgeführt. Die treibende Kraft hinter diesem Prozess ist, dass der Dampfdruck der aus dem Extrakt zu entfernenden Acrylsäure größer als ihr Partialdruck im Gas selbst ist, so dass ein Übertritt der Acrylsäure aus dem Extrakt in das Gas erfolgt. Vorteilhaft wird das Extrakt zum Zweck der Strippung gegen die Fließrichtung des Strippgases (d. h., im Gegenstrom) geführt. D. h., das Extrakt wird vorteilhaft am Kopf der Strippkolonne aufgegeben und das Strippgas wird unten in die Strippkolonne geführt. Ganz besonders vorteilhaft wird als Strippgas das am Kopf der Kondensationskolonne herausgeführte Restgas (bzw. Restgasgemisch), oder eine Teilmenge dieses Restgases mit gleicher oder unterschiedlicher Zusammensetzung wie das gesamte Restgas, oder Gas verwendet, dessen Zusammensetzung einem oder mehreren inerten Bestandteilen des Restgases entspricht (z. B. Wasserdampf, N₂, CO₂, und/oder Luft). Die erfindungsgemäß bevorzugte Verwendung von Restgasgemisch als Strippgas hat den Vorteil, dass einerseits kein zusätzliches Strippgas bereitgestellt werden muss (welches später auch zusätzlich entsorgt werden müsste) und andererseits damit ein Strippgas eingesetzt wird, das keine für das gesamte Abtrennverfahren der Acrylsäure aus dem Produktgasgemisch der Partialoxidation fremden Bestandteile enthält. Im Regelfall besteht Restgas überwiegend aus den für die Partialoxidation des C₃-Vorläufers der Acrylsäure verwendeten inerten Verdünnungsgasen (bleiben bei der Partialoxidation im wesentlichen chemisch unverändert erhalten) sowie aus bei der Partialoxidation üblicherweise als Nebenprodukt gebildetem sowie gegebenenfalls als Verdünnungsgas zugesetztem Wasserdampf und durch unerwünschte vollständige Oxidation als Nebenreaktion gebildeten Kohlenoxiden (häufig wird auch eine Restgasgemischzusammensetzung aufweisende Teilmenge des Restgases als Verdünnungsgas in die Partialoxidation rückgeführt; diese soll in dieser Schrift als Partialoxidationskreisgas bzw. Kreisgas bezeichnet werden). Teilweise enthält es noch geringe Mengen an bei der Partialoxidation nicht verbrauchtem molekularem Sauerstoff (Restsauerstoff, was vorteilhaft ist (z. B. zur Vermeidung unerwünschter Polymerisatbildung)) und/oder an nicht umgesetztem C₃-Vorläufer und/oder nicht umgesetztem Zwischenprodukt. Die bei der Partialoxidation mitverwendeten inerten Verdünnungsgase (z. B. N₂, CO₂, H₂O und/oder gesättigte Kohlenwasserstoffe etc.) sind einerseits dabei behilflich, die bei der Partialoxidation frei werdende Reaktionswärme aufzunehmen und gewährleisten andererseits in der Regel gleichzeitig einen sicheren Betrieb der heterogen katalysierten Gasphasen-Partialoxidation des C₃-Vorläufers, indem sie das Reaktionsgasgemisch entweder außerhalb des Explosionsbereichs oder in einer noch sicher beherrschbaren Region des explosiven Bereichs halten (vgl. z. B. DE-A 197 40 253, DE-A 197 40 252, DE-A 102 43 625, DE-A 103 32 758 und WO 2004/035514).

Bei Verwendung von Dual-Flow-Böden als trennwirksame Einbauten in der Desorptionskolonne (Strippkolonne) beträgt der Lochdurchmesser (der Durchmesser der Durchtrittsöffnungen in den Böden) in der Regel 8 bis 50 mm, vorzugsweise 10 bis 35 mm. Bevorzugt nimmt der Lochdurchmesser von oben nach unten zu. Der Bodenabstand (üblicherweise sind die Böden äquidistant angeordnet) beträgt häufig 300 bis 800 mm, vielfach 400 bis 600 mm und häufig 500 mm. Gefertigt wird die Desorptionseinheit, wie bereits gesagt, üblicherweise aus austenitischem Stahl, vorzugsweise aus dem Werkstoff 1.4571 (nach DIN EN 10020).

Der Kolonnenzulauf an zu strippendem Extrakt erfolgt im oberen Bereich der Desorptionseinheit, vorzugsweise auf deren obersten (theoretischen) Boden. Anwendungstechnisch zweckmäßig wird das Extrakt zuvor erwärmt (typisch auf Temperaturen von 80 bis 120°C, häufig ca. 100°C). Über innen- und/oder außenliegende indirekte Wärmeaustauscher herkömmlicher Bauart (z. B. Robertverdampfer, Zwangsumlaufrohrbündelwärmeübertrager, Zwangsumlaufrohrbündelentspannungswärmeübertrager, Plattenwärmeübertrager etc.; vgl. z. B. EP-A 854 129) und/oder über Doppelwandheizung (als Wärmeträger wird vorteilhaft mit der Abwärme der Partialoxidation gewonnener Wasserdampf verwendet) wird in vorteilhafter Weise dem Sumpf der Strippkolonne (der Desorptionskolonne) zusätzlich Wärme zugeführt. Vorzugsweise erfolgt sie über außenliegende Umlaufverdampfer mit Natur- oder Zwangsumlauf. Besonders bevorzugt werden außenliegende Umlaufverdampfer mit Zwangsumlauf (insbesondere Entspannungsumlauf) eingesetzt. Der Einsatz mehrerer Verdampfer, in Reihe oder parallel geschaltet, ist möglich (typische Sumpftemperaturen liegen bei 145 bis 165 °C, häufig bei ca. 155 °C).

Das unter den angewandten Desorptionsbedingungen nicht kondensierbare und inerte Strippgas wird vorteilhaft unmittelbar in den Sumpf der Desorptionseinheit eingeleitet. Der Zulauf aus dem außenliegenden Wärmeübertrager (in welchem zuvor entnommener Sumpfflüssigkeit die benötigte Wärme zugeführt wird) wird mit Vorteil auf einen der unteren (theoretischen) Kolonnenböden (vorzugsweise im Bereich der theoretischen Böden 2 bis 4 von unten) oder darunter vorgenommen. Das im Kolonnensumpf der Desorptionskolonne anfallende, an Acrylsäure im wesentlichen freie Extraktionsmittel wird vorteilhaft auf den Kopf der Sauerwasserextraktion rückgeführt.

Da es im Vergleich zum in der Extraktionseinheit anfallenden Extrakt normalerweise eine erhöhte Temperatur aufweist, ist es zweckmäßig, das von der Extraktion zur Strippkolonne zu führende Extrakt und das aus dem Sumpf der Strippkolonne zur Extraktion rückzuführende Extraktionsmittel (eine gegebenenfalls erforderliche aktive Polymerisationsinhibierung von Extraktions- und Strippkolonne würde man aufgrund der Rückführung vorteilhaft in den Sumpf der Strippkolonne hinein vornehmen; z. B. mittels MEHQ und/oder PTZ, wenn das Strippgas molekularen Sauerstoff enthält) in indirektem Wärmeaustausch durch einen indirekten Wärmeüberträger herkömmlicher Bauart (Rohrbündelwärmeübertrager oder Plattenwärmeübertrager) zu führen, um das Extrakt zu erwärmen und das Extraktionsmittel abzukühlen. Nachfolgend wird das Extraktionsmittel normalerweise zusätzlich in einem indirekten Wärmeübertrager mittels z. B. Wasserkühlung auf seine Einsatztemperatur in der Sauerwasserextraktion gebracht. Bezogen auf den aus dem Sumpf der Strippkolonne in die Extraktion rückgeführten Extraktionsmittelstrom werden normalerweise zusätzlich 0,01 bis 1 Gew.-% der Sumpfflüssigkeit der Strippkolonne ausgelassen (purge-Strom) (hinter der vorgenannten Wasserkühlung wird normalerweise ein entsprechender Mengenstrom an frischem Extraktionsmittel in dem Extraktionsmittelrückführstrom ergänzt). Dieser purge-Strom kann entsorgt (z. B. gemeinsam mit Restgas verbrannt) oder rektifikativ aufgearbeitet und in frisches Extraktionsmittel überführt werden.

Das Strippgas wird vorzugsweise mit einer Temperatur in die Strippkolonne geführt, die wenigstens derjenigen der Sumpfflüssigkeit in der Strippkolonne weitgehend entspricht (im Regelfall werden die beiden Temperaturen nicht mehr als 30 °C, besser nicht mehr als 20 °C auseinanderliegen). Wird Restgas als Strippgas verwendet, kann es z. B. dadurch auf vorgenannte Temperatur gebracht werden, dass man es auf einen erhöhten Druck verdichtet. Vorteilhaft kann diese Verdichtung gemeinsam mit Kreisgas auf einen auch für die Rückführung von Restgas in die C₃-Vorläuferpartialoxidation geeigneten Druck (üblicherweise mit Hilfe eines Radialverdichters) erfolgen. In typischer Weise liegen vorgenannte Druck bei 2 bis 4 bar, häufig bei 2,5 bis 3,5 bar (bei einem Restgasdruck von typischerweise > 1 und ≤ 1,5 bar).

Bezogen auf 1 kg Extrakt liegt die zum Freistrippen der Acrylsäure benötigte Menge an Strippgas in typischer Weise bei 1,5 bis 2,5 Nm³ Strippgas. Typische Beladungen des Strippgases mit Acrylsäure betragen (am Kopf der Strippkolonne) 2 bis 6 Gew.-%.

Grundsätzlich gilt, dass mit zunehmender verwendeter Strippgasmenge der Wärmeeintrag in den Sumpf der Strippkolonne gemindert werden kann. Der Vorzug einer Abtrennung der Acrylsäure aus dem Extrakt durch Strippen mit einem unter den Desorptionsbedingungen üblicherweise nicht kondensierbaren und inerten Strippgas liegt unter anderem darin begründet, dass es sich um eine besonders effiziente und gleichzeitig energetisch sowie apparativ wenig aufwendige Abtrennweise handelt. Darüber hinaus bringt sie im Unterschied zur z. B. kristallisativen oder rektifikativen Abtrennung aus dem Extrakt den Vorteil mit sich, dass sie keine zusätzliche, Acrylsäure in erhöhter Konzentration aufweisende, flüssige Phase erzeugt, die des Aufwandes einer zusätzlichen ausgeprägten Polymerisationsinhibierung bedarf.

Vorstehendes ist nicht zuletzt darauf zurückzuführen, dass das mit Acrylsäure beladene Strippgas, insbesondere dann, wenn zum Strippen Restgas verwendet wurde, als solches in die Kondensationskolonne (die für die fraktionierende Kondensation des Produktgasgemischs der Partialoxidation eingesetzt wird) rückgeführt werden kann. Zweckmäßigerweise erfolgt eine solche Rückführung unterhalb des ersten Seitenabzugs in die Kondensationskolonne. Vorteilhaft Wird man die Rückführung unmittelbar in den Sumpfraum der Kondensationskolonne vornehmen. Grundsätzlich kann sie unmittelbar in die Sumpfflüssigkeit hinein erfolgen.

Vorliegende Anmeldung umfasst deshalb im Besonderen ein solches erfindungsgemäßes Verfahren, das dadurch gekennzeichnet ist, dass man wenigstens bei einer Teilmenge (mit Vorteil eine Teilmenge von wenigstens 25 Gew.-%, besser wenigstens 50 Gew.-%, bevorzugt wenigstens 75 Gew.-%, oder die Gesamtmenge) von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes aus dem sauren Wasser in das organische Lösungsmittel aufnimmt.

Ganz besonders vorteilhaft wird man beim erfindungsgemäßen Verfahren das mit Acrylsäure beladene Strippgas (bzw. wenigstens eine Teilmenge desselben) jedoch dazu verwenden, um in einer zweiten Strippkolonne in der der Kondensationskolonne entnommenen Sumpfflüssigkeit noch enthaltene Acrylsäure ebenfalls freizustrippen (und es erst dann in die Kondensationskolonne rückführen), bevor die Sumpfflüssigkeit der Entsorgung zugeführt wird. Erfindungsgemäß vorteilhaft wird das beladene Strippgas dieser zweiten Strippung noch einer (Gegenstrom-)Rektifikation unterworfen, wie es bereits in der WO 2004/035514 und in der DE-A 103 32 758 (jedoch unter unmittelbarer Verwendung von Restgas als Strippgas) beschrieben ist (und erst dann in die Kondensationskolonne rückgeführt).

Der Sumpfraum in der beim erfindungsgemäßen Verfahren verwendeten Kondensationskolonne (bzw. ganz generell bei einer trennwirksame Einbauten aufweisenden Kolonne) ist der Raum unterhalb der untersten trennwirksamen Einbaute (z. B. Böden, Packungen und Füllkörper, die die Grenzfläche zwischen den in der Trennkolonne im Gegenstrom geführten Phasen vergrößern) innerhalb der Kondensationskolonne.

In ihm sammelt sich die sogenannte Sumpfflüssigkeit, die insbesondere solche Bestandteile umfasst, deren Siedepunkt bei Normaldruck (1 atm) oberhalb des Siedepunktes von Acrylsäure liegt. Dies sind zum einen schwerer als Acrylsäure siedende Nebenprodukte wie z. B. Maleinsäureanhydrid, Nebenkomponenten wie Phenothiazin, die als Polymerisationsinhibitoren zugesetzt werden, aber auch Folgeprodukte, die sich im Verlauf der fraktionierenden Kondensation des Produktgasstroms der Partialoxidation aus den Bestandteilen derselben erst bilden. Zu diesen Folgeprodukten zählen insbesondere radikalische Polymerisate der Acrylsäure, die sich trotz der Polymerisations-inhibierung in unerwünschter Weise ausbilden. Auch zählen höhermolekulare Verbindungen dazu, die sich durch Kondensationsreaktionen unterschiedlicher Bestandteile des Produktgasgemischs der Partialoxidation ausbilden. Zu diesen gehören nicht zuletzt die Michael-Addukte, die sich durch reversible Michael-Addition von Acrylsäure an sich selbst sowie an das sich dabei bildende Acrylsäure-Dimere ("Diacrylsäure") bzw. -Oligomere ausbilden (der Begriff Acrylsäure - Oligomere meint in dieser Schrift daher stets die entsprechenden Michael-Addukte und nicht durch radikalische Polymerisation entstehende Acrylsäureoligomere). Durch die Einwirkung erhöhter Temperatur unter gleichzeitiger Abtrennung der gebildeten Acrylsäure (z. B. durch "Strippen" mit einem unter den entsprechenden Bedingungen vorzugsweise (chemisch) inerten und nicht kondensierbaren Gas) kann die Michael-Addition umgekehrt werden. Aber auch monomere Acrylsäure selbst ist in nicht unerheblichem Umfang noch Bestandteil der Sumpfflüssigkeit in der Kondensatiönskolonne, was ein Freistrippen derselben lohnend macht. Da die Surüpfflüssigkeit der Kondensationskolonne aber auch Nebenprodukte enthält, die bei Normaldruck nur geringfügig höher wie Acrylsäure sieden (z. B. Benzaldehyd, Furfurale und Maleinsäureanhydrid) ist es vorteilhaft, das beladene Strippgas dieser zweiten Strippung (vor z. B. seiner Rückführung in die Kondensationskolonne) zusätzlich noch einer (Gegenstrom-)Rektifikation (d. h., einem Gegenstrom von Rücklaufflüssigkeit) zu unterwerfen (zweckmäßiger Weise in derselben Kolonne), was eine verbesserte Reinheit des mit Acrylsäure beladenen zweiten Strippgas bedingt.

Die Rückführung des ersten beladenen Strippgases und/oder des zweiten beladenen Strippgases in die Kondensätionskolonne muss nicht notwendiger Weise auf direktem Weg erfolgen.

Vielmehr kann das erste beladene Strippgas und/oder das zweite beladene Strippgas auch wenigstens teilweise oder vollständig mit Produktgasgemisch gemischt und das dabei resultierende Gasgemisch in die Kondensationskolonne geführt werden.

Die Zusammenführung kann dabei vor, während und/oder nach einer gegebenenfalls durchzuführenden direkten und/oder indirekten Kühlung des Produktgasgemischs der Gasphasen-Partialoxidation erfolgen.

Im Folgenden soll aus Gründen der Differenzierbarkeit das mit Acrylsäure beladene Strippgas aus der Extraktstrippung als "erstes beladenes Gas" und das mit Acrylsäure beladene (vorzugsweise rektifizierte) Strippgas aus der (Kondensationskolonne) Sumpfstrippung als "zweites beladenes Gas" bezeichnet werden (in entsprechender Weise soll die Extraktstrippung als "erste Strippung" und die Sumpfstrippung als "zweite Strippung" bezeichnet werden etc.). In der Regel verlässt das erste beladene Gas die Extraktstrippung mit einem Druck von 1,5 bis 3,5 bar, häufig 2 bis 3 bar, z. B. 2,5 bar. Seine Temperatur liegt üblicherweise ca. 10 °C unterhalb derjenigen Temperatur, mit der das Extrakt auf die erste Strippkolonne aufgegeben wird. Typische Temperaturen des ersten beladenen Gases betragen 65 bis 95 °C, häufig 75 bis 95 °C, oder 85 °C.

Zur Durchführung der zweiten Strippung (einschließlich der Rektifikation des Strippgases) kommen grundsätzlich alle Kolonnen mit trennwirksamen Einbauten in Betracht, wobei mögliche Einbauten z. B. Packungen, Füllkörper und/oder Böden sind. Prinzipiell können auch Kolonnen mit rotierenden Einsätzen, sogenannte Rotationskolonnen verwendet werden, die die Rücklaufflüssigkeit in Tropfen versprühen. Erfindungsgemäß bevorzugt wird als zweite Strippkolonne eine solche eingesetzt, die nur Böden und/oder Packungen enthält. Als Böden werden dabei mit Vorteil Dual-Flow-Böden verwendet und mit besonderem Vorteil enthält die Strippkolonne ausschließlich Dual-Flow-Böden als trennwirksame Einbauten.

Unter Dual-Flow-Böden sollen in dieser Schrift Platten mit einfachen Durchtrittstellen (Löcher, Schlitze etc.) verstanden werden. Das in der zweiten Strippkolonne aufsteigende Gas und die in der zweiten Strippkolonne absteigende Rücklaufflüssigkeit treten entgegengesetzt strömend durch die gleichen Durchtrittstellen. Der Querschnitt der Durchtrittstellen wird in an sich bekannter Weise der Belastung der zweiten Strippkolonne angepasst. Ist er zu klein, strömt das aufsteigende Gas mit so hoher Geschwindigkeit durch die Durchtrittstellen, dass die in der Strippkolonne absteigende Rücklaufflüssigkeit im wesentlichen ohne Trennwirkung mitgerissen wird. Ist der Querschnitt der Durchtrittstellen zu groß, bewegen sich aufsteigendes Gas und absteigender Rücklauf im wesentlichen ohne Austausch aneinander vorbei und der Boden läuft Gefahr trocken zu laufen. Üblicherweise weisen Dual-Flow-Böden kein Ablaufrohr auf, das sie mit dem nächsten Boden verbindet. Selbstredend kann jeder Dual-Flow-Boden mit den Wänden der zweiten Strippkolonne bündig abschließen. Er kann aber auch über Stege mit diesen verbunden sein. Mit abnehmender Belastung der zweiten Strippkolonne laufen Dual-Flow-Böden im Unterschied zu hydraulisch abgedichteten Querstromböden trocken.

Erfindungsgemäß zweckmäßig kann die für die zweite Strippung (einschließlich der angesprochenen Rektifikation) verwendbare Dual-Flow-Boden-Strippkolonne bis zu 60 Dual-Flow-Böden enthalten. Mit Vorteil weisen diese ein Öffnungsverhältnis (das Verhältnis D:U, gebildet aus dem Flächenanteil des Bodens, der für das Spaltgas durchlässig ist (D) und der Gesamtfläche des Bodens (U)) von 10 bis 20 %, bevorzugt von 10 bis 15 % auf.

Die Durchtrittstellen der Dual-Flow-Böden sind bevorzugt kreisrunde Löcher mit einem innerhalb des Bodens einheitlichen Kreisdurchmesser. Letzterer beträgt zweckmäßig 10 bis 30 mm. Im oberen Teil der Kolonne beträgt er mit Vorteil 10 bis 20 mm, bzw. 10 bis 15 mm, und im unteren Teil der Kolonne beträgt er mit Vorteil 20 bis 30 mm. Ferner sind die kreisrunden Löcher über den individuellen Dual-Flow-Böden bevorzugt in strenger Dreiecksteilung gleichmäßig angeordnet (vgl. DE-A 102 30 219). Ferner zeigt der Stanzgrat der in den Dual-Flow-Böden herausgestanzten Durchtrittsöffnungen in der zweiten Strippkolonne bevorzugt nach unten. Üblicherweise sind die Dual-Flow-Böden in der zweiten Strippkolonne äquidistant angeordnet. In typischer Weise liegt der Bodenabstand bei 300 mm bis 500 mm. Erfindungsgemäß günstig ist auch ein Bodenabstand von 400 mm. In zweckmäßiger Weise erfolgt die Zufuhr der zu strippenden Sumpfflüssigkeit (sie kann zuvor noch zur Direktkühlung des Produktgasgemischs der Partialoxidation verwendet und/oder durch im unteren Bereich der Kondensationskolonne entnommene Schwersiederfraktion ergänzt worden sein) auf den vierten bis zehnten Dual-Flow-Boden der zweiten Strippkolonne (von unten gerechnet). Die Sumpftemperatur in der zweiten Strippkolonne wird vorteilhaft bei 150 bis 190 °C, bevorzugt bei 160 bis 180 °C gehalten. Der Arbeitsdruck wird in der zweiten Strippkolonne normalerweise > 1 bis 3 bar, häufig 1,5 bis 2,5 bar betragen.
Der in der zweiten Strippkolonne benötigte Energieeintrag wird vorteilhaft mit Hilfe eines ausgelagerten Zwangsumlaufrohrbündelentspannungsverdampfers zugeführt, dem aus der zweiten Strippkolonne entnommene Sumpfflüssigkeit zum Zweck ihrer Überhitzung zugeführt und nachfolgend so überhitzt in die zweite Strippkolonne rückgeführt wird (vgl. DE-A 103 32 758). Grundsätzlich könnte an dieser Stelle aber auch ein reiner Zwangsumlaufverdampfer oder ein Naturumlaufverdampfer, z. B. ein Robertverdampfer, eingesetzt werden, der auch in die zweite Strippkolonne integriert sein kann.

Die zweite Strippkolonne selbst ist (wie auch die Kondensationskolonne und die erste Strippkolonne) anwendungstechnisch zweckmäßig gegen die Umgebung wärmeisoliert.

Die Erzeugung der Rücklaufflüssigkeit kann durch direkte und/oder indirekte Kühlung erfolgen. Erfindungsgemäß vorteilhaft wird die Methode der direkten Kühlung angewendet. Dazu wird in einfachster Weise das den letzten (theoretischen) Boden durchströmende Gas einer Quenchvorrichtung zugeführt, die z. B. über einen Kaminboden von den trennwirksamen Einbauten der zweiten Strippkolonne abgetrennt in die zweite Strippkolonne integriert (auf den trennwirksamen Teil aufgesetzt) sein kann. Grundsätzlich kann die Quenchvorrichtung aber auch aus der zweiten Strippkolonne räumlich ausgelagert sein. Als eine solche Quenchvorrichtung können alle im Stand der Technik für diesen Zweck bekannten Vorrichtungen (z. B. Sprühwäscher, Venturiwäscher, Blasensäulen oder sonstige Apparate mit berieselten Oberflächen) eingesetzt werden, wobei vorzugsweise Venturi-Wäscher oder Sprühkühler verwendet werden. Mit Vorteil wird eine Gleichstromvorrichtung (z. B. eine mit Prallplattendüse) verwendet. Zur indirekten Kühlung der Quenchflüssigkeit wird diese üblicherweise über einen (indirekten) Wärmeübertrager bzw. Wärmeaustauscher geführt. Diesbezüglich eignen sich alle gängigen Wärmeübertrager oder Wärmeaustauscher. Als bevorzugt seien Rohrbündelwärmeaustauscher, Plattenwärmeaustauscher und Luftkühler genannt. Geeignete Kühlmedien sind Luft beim entsprechenden Luftkühler und Kühlflüssigkeiten, insbesondere Wasser (z. B. Oberflächenwasser), bei den anderen Kühlvorrichtungen. Anwendungstechnisch zweckmäßig wird als Quenchflüssigkeit (im Folgenden auch als "Quenchflüssigkeit 0" bezeichnet) eine Teilmenge des beim Quenchen gebildeten Kondensats verwendet. Die andere Teilmenge des beim Quenchen gebildeten Kondensats wird im wesentlichen als Rücklaufflüssigkeit auf den obersten (theoretischen) Boden der zweiten Strippkolonne rückgeführt (eine kleine Teilmenge des Kondensats kann an dieser Stelle auch abgezweigt und der aus der Kondensationskolonne entnommenen Sumpfflüssigkeit zugeführt und mit dieser vereinigt werden; ein Teil des dabei resultierenden Gemischs kann als im weiteren Verlauf dieser Schrift noch einzuführende Quenchflüssigkeit 1 eingesetzt werden; ein anderer Teil dieses Gemischs bildet den Zulauf zur zweiten Strippkolonne und die verbliebene (vergleichsweise geringe) Restmenge dieses Gemischs wird mit der verbliebenen Teilmenge des Kondensats vereinigt und bildet in dieser Vereinigung dann zum einen Teil die Rücklaufflüssigkeit auf den obersten (theoretischen) Boden der zweiten Strippkolonne und zum anderen Teil die Quenchflüssigkeit 0). In typischer Weise beträgt die Temperatur der Quenchflüssigkeit 0 unmittelbar vorab ihrer Verwendung zum Quenchen etwa 40 °C, wohingegen die Rücklaufflüssigkeit in typischer Weise mit etwa 80 °C rückgeführt wird. Das Massenverhältnis von rückgeführter Rücklaufflüssigkeit zu der zweiten Strippkolonne zugeführter Sumpf (sowie gegebenenfalls Schwersieder)flüssigkeit beträgt typisch ≥ 2. Häufig beträgt es 2 bis 10 und vorzugsweise 4 bis 8.

Selbstverständlich muss die zweite Strippkolonne (wie alle anderen Apparate in denen flüssige, nennenswerte Acrylsäuregehalte aufweisende Phasen geführt werden) polymerisationsinhibiert betrieben werden. Als solche Polymerisationsinhibitoren können für diesen Zweck prinzipiell alle im Stand der Technik bekannten Polymerisationsinhibitoren eingesetzt werden.

Beispielhaft genannt seien als solche Phenothiazin (PTZ) und p-Methoxyphenol (MEHQ). Häufig werden diese beiden kombiniert angewendet. Zweckmäßigerweise werden sie dazu in einer reinen Acrylsäure gelöst zugesetzt. MEHQ wird vorzugsweise als Schmelze zudosiert.

In besonders eleganter Weise lässt sich die Polymerisationsinhibierung des Quenchkreises 0 und der zweiten Strippkolonne jedoch dadurch bewerkstelligen, dass man dem Quenchkreis 0 eine Teilmenge der aus der Kondensationskolonne herausgeführten, polymerisationsinhibierten, d. h., Polymerisationsinhibitoren enthaltenden, Sumpfflüssigkeit (und gegebenenfalls Schwersiederfraktion) zusetzt. In typischer Weise enthält die Quenchflüssigkeit 0 so z. B. 0,01 bis 0,1 Gew.-% MEHQ und 0,01 bis 0,5 Gew.-% PTZ. Der im ersten beladenen Gas (das als Strippgas für die zweite Strippkolonne fungiert) üblicherweise noch enthaltene molekulare Sauerstoff fördert die Polymerisationsinhibierung zusätzlich.

Eine Teilmenge der im Sumpf der zweiten Strippkolonne anfallenden Sumpfflüssigkeit wird kontinuierlich ausgeschleust und entsorgt. Durch Zusatz eines organischen Lösungsmittels, z. B. von Methanol, wird der schwerstflüchtige Rückstand bei Bedarf fluid gehalten. Anstelle von Methanol können auch andere hydrophile organische Flüssigkeiten wie z. B. Ethanol oder die in der WO 2004/035514 Empfohlenen (oder Gemische solcher Flüssigkeiten) verwendet werden. Bezogen auf den Zufuhrstrom an aus der Kondensationskolonne zugeführter Sumpfflüssigkeit liegt der vorgenannte Ausschleusestrom bei ca. 10 oder 20 bis 30 Gew.-%. Anwendungstechnisch zweckmäßig wird der Ausschleusungsstrom aus dem den Zwangsumlaufentspannungsverdampfer, der die zweite Strippkoionne mit Energie versorgt, überhitzt verlassenden Stoffstrom abgezweigt. Er wird entgast und z. B. mit Methanol verdünnt z. B. der Rückstandsverbrennung zugeführt.

Das den Quenchkreis 0 gekühlt (typische Temperaturen betragen 70 °C bis 90 °C) verlassende zweite beladene Gas kann nun als solches in die Kondensationskolonne rückgeführt werden (an dieser Stelle sei festgehalten, dass in der zweiten Strippkolonne bezogen auf 1 kg aus der Kondensationskolonne zugeführte Sumpfflüssigkeit in typischer Weise 1 bis 10 Nm³ an erstem beladenem Gas als Strippgas verwendet werden; üblicherweise befindet sich die Flüssigkeit im Sumpf der zweiten Strippkolonne im Siedezustand). Sein Acrylsäuregehalt beträgt in typischer Weise 15 bis 20 Gew.-%. Grundsätzlich könnte die Rückführung der darin enthaltenen Acrylsäure in die Kondensationskolonne auch in auskondensierter Form erfolgen. Ein solches Acrylsäurekondensat kann aber auch mit der aus der Kondensationskolonne entnommenen rohen Acrylsäure vereinigt der Weiterreinigung derselben zugeführt werden. Erfindungsgemäß bevorzugt ist eine Acrylsäure-Rückführung in die Kondensationskolonne als Bestandteil des zweiten beladenen Gases als solchem, d. h., gasförmig (prinzipiell kann die Rückführung auch teilkondensiert erfolgen).

Die Rückführung des zweiten beladenen Gases in die Kondensationskolonne (alle Aussagen gelten in gleicher Weise für eine Rückführung des ersten beladenen Gases als solchem in die Kondensationskolonne) wird vorzugsweise unterhalb des ersten Seitenabzugs vorgenommen (grundsätzlich kann sie auch in den Direktkühlungskreis des Produktgasgemischs der Gasphasen-Partialoxidation hinein erfolgen). Anwendungstechnisch zweckmäßig erfolgt sie in den Sumpfraum der Kondensationskolonne. Man kann diese Rückführung sowohl in die Sumpfflüssigkeit getaucht, als auch oberhalb des Flüssigkeitsspiegels der Sumpfflüssigkeit und unterhalb des ersten (theoretischen) Bodens der Kondensationskolonne vornehmen. Vorteilhaft enthält der Sumpfraum der Kondensationskolonne einen Tropfenabscheider (z. B. einen Zentrifugaltropfenabscheider), um ein Mitreißen von Sumpfflüssigkeitstropfen durch aufsteigendes Gas zu unterdrücken. Außerdem kann der Sumpfraum durch einen ersten Kaminboden von der untersten trennwirksamen Einbaute getrennt werden.

Die Verweilzeit der der zweiten Strippkolonne aus der Kondensationskolonne zugeführten Sumpfflüssigkeit (gegebenenfalls einschließlich entnommener Schwersiederfraktion) in der zweiten Strippvorrichtung sollte üblicherweise 0,5 bis 4 h betragen (für eine abschließend ausgeschleuste Schwersiedernebenkomponente). Selbstredend können dem Sumpf der zweiten Strippvorrichtung Rückspaltkatalysatoren für die Rückspaltung der Acrylsäureoligomeren zudosiert werden, wie es z. B. die WO 2004/035514 empfiehlt.

Selbstverständlich können dem Sumpf der zweiten Strippkolonne auch Hilfsmittel wie Komad^{®}313 der Firma Mol (Ungarn) und/oder Dispergiermittel (z. B. jene der EP-A 1 062 197 und/oder der US-A 3,271,296) wie z. B. Tertiäre Amine (z. B. Trimethylamin, Triethylamin, N,N,N',N'-Tetramethyl-1,6-hexandiamin und Pentamethyldiethylentriamin) zugesetzt werden, die das Fouling im die erforderliche Energiezufuhr gewährleistenden indirekten Wärmeaustauscher mindern und die Acrylsäureausbeute fördern. Die Zusatzmengen können dabei 0,1 bis 10 Gew.-%, bezogen auf den Sumpf der zweiten Strippkolonne, betragen. Da es sich dabei vorteilhaft bereits um schwersiedende Substanzen handelt oder diese Additive mit Acrylsäure solche ausbilden, werden sie nicht mit ausgestrippt.

Die Vorteilhaftigkeit der beschriebenen erfindungsgemäßen Verfahrensweisen ist u. a. darin begründet, dass sie eine Erhöhung der Ausbeute an Acrylsäure ermöglichen ohne die Reinheit der über den ersten Seitenabzug entnommenen rohen Acrylsäure wesentlich zu mindern. Dies betrifft nicht zuletzt auf die beschriebene Strippvariante zu und zwar auch dann, wenn zum Zweck der Strippung wie beschrieben Restgas aus der Kondensationskolonne verwendet wird.

Dabei ist bereits die Reinheit sowohl des ersten als auch des zweiten beladenen Gases mit Bezug auf bei einer Folgeverwendung der Acrylsäure (wie z. B. zur Herstellung von Wasser superabsorbierenden Polymerisaten) störenden Nebenkomponenten so gut, dass (von der jeweiligen Gesamt- oder auch nur von einer Teilmenge des ersten und/oder zweiten beladenen Gases) die darin enthaltene Acrylsäure auch unmittelbar aus der Gasphase des ersten oder zweiten beladenen Gases heraus in die wässrige Lösung eines Metallhydroxids (z.B. eines Alkalimetallhydroxids und/oder Erdalkalimetallhydroxids, z.B. NaOH, KOH, Ca(OH)₂ und/oder Mg(OH)₂) aufgenommen (z. B. in völliger Entsprechung zur in der WO 2003/095410 beschriebenen Verfahrensweise) und die dabei im Fall einer Aufnahme in z. B. wässrige Natronlauge z.B. resultierende wässrige Lösung an Natriumacrylat z. B. unmittelbar zur Herstellung von Wasser superabsorbierenden Polymerisaten durch entsprechende radikalische Polymerisation (vgl. dazu auch WO 2003/014172) verwendet werden kann.

Dies liegt nicht zuletzt daran, dass Nebenprodukte wie Formaldehyd und Ameisensäure normalerweise bevorzugt in der wässrigen Phase des Sauerwassers verbleiben. Das durch z. B. eine Gegenstromwäsche mit wässriger Natronlauge (oder einer anderen der vorgenannten wässrigen Metallhydroxidlösungen) in einer trennwirksame Einbauten enthaltenden Kolonne (Böden, Füllkörper und/oder Packungen) von Acrylsäure befreite erste oder zweite beladene Gas kann nachfolgend in die Extraktstrippung rückgeführt und/oder der Verbrennung zugeführt werden.

In typischer Weise kann das Acrylsäure enthaltende Produktgasgemisch einer heterogen katalysierten Gasphasen-Partialoxidation von C₃-Vorläufern der Acrylsäure mit molekularem Sauerstoff an im festen Aggregatzustand befindlichen Katalysatoren z. B. folgende Gehalte (insbesondere dann, wenn als C₃-Vorläufer Propylen verwendet wird) aufweisen:

| | |
|---|---|
| 1 bis 30 Gew.-% | Acrylsäure, |
| 0,05 bis 10 Gew.-% | molekularer Sauerstoff, |
| 1 bis 30 Gew.-% | Wasser, |
| > 0 bis 5 Gew.-% | Essigsäure, |
| > 0 bis 3 Gew.-% | Propionsäure, |
| > 0 bis 1 Gew.-% | Maleinsäure und/oder Maleinsäure-Anhydrid, |
| 0 bis 2 Gew.-% | Acrolein, |
| 0 bis 1 Gew.-% | Formaldehyd, |
| > 0 bis 1 Gew.-% | Furfurale, |
| > 0 bis 0,5 Gew.-% | Benzaldehyd, |
| 0 bis 1 Gew.-% | Propylen, und als Restmenge im wesentlichen inerte Gase wie z. B. Stickstoff, Kohlenmonoxid, Kohlendioxid, Methan und/oder Propan. |

Üblicherweise enthält das Produktgasgemisch, bezogen auf enthaltene Acrylsäure, ≥ 0,005 mol-%, häufig ≥ 0,03 mol-%, an Furfuralen. In der Regel beträgt der so bezogene Furfuralgehalt jedoch ≤ 3 mol.-%.

Die Gasphasen-Partialoxidation selbst kann wie im Stand der Technik beschrieben durchgeführt werden. Ausgehend von Propylen kann die Gasphasen-Partialoxidation z. B. in zwei aufeinanderfolgenden Oxidationsstufen durchgeführt werden, wie sie in der EP-A 700 714 und in der EP-A 700 893 beschrieben sind. Selbstverständlich können aber auch die in der DE-A 197 40 253 sowie in der DE-A 197 40 252 zitierten Gasphasen-Partialoxidationen zur Anwendung kommen.

Im Sinne einer geringen Menge gebildeter Nebenkomponenten wird die Propylen-Gasphasen-Partialoxidation bevorzugt wie in der DE-A 101 48 566 beschrieben durchgeführt. Als Propylenquelle kann dazu Polymer grade Propylen oder Chemical grade Propylen gemäß der DE-A 102 32 748 verwendet werden. Handelt es sich bei dem verwendeten C₃-Vorläufer um Propan, kann die Partialoxidation wie in der DE-A 102 45 585 beschrieben durchgeführt werden.

Grundsätzlich kann die Gasphasen-Partialoxidation aber auch wie in den Schriften US 2006/0161019, WO 2006/092410, WO 2006/002703, WO 2006/002713, WO 2005/113127, DE-A 10 2004 021 763, EP-A 1 611 076, WO 2005/108342, EP-A 1 656 335, EP-A 1 656 335, EP-A 1 682 478, EP-A 1 682 477, den deutschen Anmeldungen Nr. 10 2006 054 214.2 und Nr. 10 2006 024 901.1, EP-A 1 611 080, EP-A 1 734 030, den deutschen Anmeldungen-Nr. 10 2006 000 996.7, Nr. 10 2005 062 026.4, Nr. 10 2005 062 010.8, der internationalen Anmeldung Nr. PCT/EP 2006/065416, PCT/EP 2006/067784 sowie PCT/EP 2006/067080 beschrieben durchgeführt werden.

Häufig beträgt die Temperatur des die Gasphasen-Partialoxidation verlassenden Produktgasgemisches 150 bis 350 °C, vielfach 200 bis 300 °C, manchmal bis zu 500 °C.

Anwendungstechnisch zweckmäßig wird das heiße Produktgasgemisch anschließend in einer Quenchvorrichtung 1 durch direkte Kühlung in der Regel auf eine Temperatur von 100 bis 180 °C abgekühlt, bevor es, anwendungstechnisch vorteilhaft gemeinsam mit der verwendeten Quenchflüssigkeit 1, zum Zweck der fraktionierenden Kondensation bevorzugt in den unteren Abschnitt (bevorzugt den untersten, z. B. den Sumpfraum) einer trennwirksamen Einbauten enthaltenden Kondensationskolonne geleitet wird.

Als Kondensationskolonneneinbauten kommen prinzipiell alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. In typischer Weise beträgt bei einer Bodenkolonne die Gesamtzahl an Trennböden 20 bis 100, häufig 20 bis 80 und bevorzugt 50 bis 80.

Erfindungsgemäß bevorzugt ist die Kondensationskolonne eine solche, die von unten nach oben, zunächst Dual-Flow-Böden und im Anschluss daran hydraulisch abgedichtete Querstromböden (z. B. Thormann^{®}-Böden) als trennwirksame Einbauten enthält, wie es die DE-A 102 43 625, die DE-A 199 24 532 und die DE-A 102 43 625 empfehlen. Die Anzahl der Dual-Flow-Böden kann dabei 5 bis 60, häufig 25 bis 45, und die Anzahl der hydraulisch abgedichteten Querstromböden ebenfalls 5 bis 60, häufig 30 bis 50 betragen. Für den erfindungsgemäß bevorzugten Bereich der Sauerwasserbildung (Acrylsäuregehalt der Rücklaufflüssigkeit von unten nach oben betrachtet in der Regel ≤ 15 Gew.-%, bzw. teilweise ≤ 10 Gew.-%) kommen als trennwirksame Einbauten bevorzugt Ventilböden in Betracht, wie es die DE-A 199 24 532 und die DE-A 102 43 625 beschreiben. Prinzipiell könnten aber auch andere gängige trennwirksame Einbauten verwendet werden (die einzelnen Bereiche innerhalb der Kondensationskolonne können natürlich ganz generell in völlig äquivalenter Weise (anstelle in einer Kolonne übereinander) auch als eine Hintereinanderschaltung entsprechend kleinerer Kolonnen gestaltet werden, weshalb der in dieser Schrift verwendete Begriff (Kondensationskolonne" im Speziellen, als auch der in dieser Schrift verwendete Begriff der Kolonne im Allgemeinen auch solche Hintereinanderschaltungen von entsprechenden kleineren Kolonnen umfasst).

Als Quenchvorrichtung 1 können alle im Stand der Technik für diesen Zweck bekannten Vorrichtungen (z. B. Sprühwäscher, Venturiwäscher, Blasensäulen oder sonstige Apparate mit berieselten Oberflächen) eingesetzt werden, wobei vorzugsweise VenturiWäscher oder Sprühkühler verwendet werden.

Zur indirekten Kühlung oder Erwärmung der Quenchflüssigkeit 1 wird diese, insbesondere beim Anfahren, bevorzugt, aber nicht in notwendiger Weise, über einen Wärmeüberträger bzw. Wärmeaustauscher geführt. Diesbezüglich eignen sich alle gängigen Wärmeüberträger oder Wärmeaustauscher. Als bevorzugt seien Rohrbündelwärmeaustauscher, Plattenwärmeaustauscher und Luftkühler genannt. Geeignete Kühlmedien sind Luft beim entsprechenden Luftkühler und Kühlflüssigkeiten, insbesondere Wasser, bei den anderen Kühlvorrichtungen.

Als Quenchflüssigkeit 1 kann z. B. aus dem Sumpf der Kondensationskolonne entnommene Sumpfflüssigkeit (gegebenenfalls vereinigt mit aus dem Quenchkreis 0 herausgeführtem Kondensat), oder über einen in Sumpfnähe gelegenen Seitenabzug Schwersiederfraktion oder ein Gemisch aus solcher Sumpfflüssigkeit und Schwersiederfraktion (insbesondere dann, wenn der Sumpfraum und der unterste (theoretische Boden (die unterste trennwirksame Einbaute) durch einen Kaminboden getrennt sind) verwendet werden. Gegebenenfalls wird nur der aus dem Sumpf der Kondensationskolonne entnommene Anteil der Quenchflüssigkeit 1 über den oben genannten Wärmeaustauscher geführt. Die Temperatur der Quenchflüssigkeit 1 beträgt beim Eintritt in die Quenchvorrichtung 1 in der Regel zweckmäßig 90 °C bis 120 °C.

Die Einleitstelle für das gequenchte (oder auf andere Art abgekühlte oder auch nicht abgekühlte) Produktgasgemisch der katalytischen Gasphasen-Partialoxidation (erfindungsgemäß wie beschrieben bevorzugt im Gemisch mit zur direkten Kühlung verwendeter Quenchflüssigkeit 1) in die Kondensationskolonne befindet sich vorteilhaft im Sumpfraum dieser Kolonne, der mit Vorteil einen Zentrifugaltropfenabscheider integriert enthält und in der Regel durch einen ersten Kaminboden von der untersten trennwirksamen Einbaute getrennt ist (anwendungstechnisch zweckmäßig leitet man in diesem Fall über eine Verbindungsleitung oder über einen Überlauf stetig Schwersiederfraktion in den Sumpf der Kondensationskolonne). In einer beispielhaften und bevorzugten Ausführungsvariante (die im folgenden ohne Beschränkung der allgemeinen Ausführbarkeit ausschließlich beschrieben wird) handelt es sich bei dieser um den ersten Dual-Flow-Boden einer ersten Serie von zweckmäßig äquidistant angeordneten, Dual-Flow-Böden. Der Kaminboden fungiert gleichzeitig als Sammelboden, von dem kontinuierlich Kondensat (Schwersiederfraktion) entnommen und als Teil der Quenchflüssigkeit 1 in die Quenchvorrichtung 1 oder in den Sumpfraum geführt wird. Die erste Serie von Dual-Flow-Böden wird von einem zweiten Kaminboden (Fangboden) abgeschlossen. Von diesem zweiten Fangboden wird im ersten Seitenabzug als Mittelsiederfraktion rohe Acrylsäure kontinuierlich entnommen, die bevorzugt eine Reinheit von ≥ 90 bzw. ≥ 95 Gew.-% aufweist.

Zweckmäßigerweise wird man diese rohe Acrylsäure weiteren destillativen (rektifikativen) und/oder kristallisativen Weiterreinigungsstufen zuführen und wenigstens einen Teil der im Rahmen dieser Destillation (Rektifikation) und/oder Kristallisation anfallenden Sumpfflüssigkeiten und/oder Mutterlaugen unterhalb des ersten Seitenabzugs, aber oberhalb des ersten Fangbodens in die Kondensationskolonne rückführen. Vorzugsweise erfolgt diese Rückführung wärmeintegriert. D.h., kalte rückzuführende Mutterlauge wird durch einen oder mehrere hintereinander geschaltete indirekte Wärmeaustauscher (z. B. Spiralwärmeaustauscher) geführt, um in diesen die der Kondensationskolonne entnommene und im Wärmeaustauscher auf der gegenüberliegenden Seite geführte, kristallisativ weiterzureinigende, rohe Acrylsäure abzukühlen. Gleichzeitig wird dadurch eine Erwärmung der Mutterlauge bewirkt. Vorzugsweise werden für diesen Zweck zwei hintereinandergeschaltete Plattenwärmeaustauscher verwendet.

In zweckmäßiger Weise wird man die (als Mittelsiederfraktion) entnommene rohe Acrylsäure zum Zweck der Weiterreinigung einer Kristallisation zuführen. Das zu verwendende Kristallisationsverfahren unterliegt prinzipiell keiner Beschränkung. Die Kristallisation kann kontinuierlich oder diskontinuierlich, einstufig oder mehrstufig bis zu beliebigen Reinheitsgraden durchgeführt werden.

Bei Bedarf kann der kristallisativ zu reinigenden rohen Acrylsäure vorab der Kristallisation vorteilhaft Wasser zugesetzt werden (in der Regel enthält diese dann, bezogen auf die enthaltene Menge an Acrylsäure, bis zu 20 Gew.-% oder bis zu 10 Gew.-%, meist bis zu 5 Gew.-% an Wasser). Bei erhöhten Aldehyd- oder sonstigen Nebenkomponentengehalten kann eine Wasserzugabe unterbleiben, da die Aldehyde in diesem Fall die Funktion des Wasser zu übernehmen vermögen. Erfindungsgemäß ganz besonders vorteilhaft wird das Wasser in Form von Sauerwasser zugesetzt. Dies führt zu einer Erhöhung der Ausbeute an Reinacrylsäure.

Es überrascht, dass selbst bei vorheriger Sauerwasserzugabe zur rohen Acrylsäure (diese Maßnahme bedingt gleichfalls eine Steigerung der Acrylsäureausbeute) eine höchsten Ansprüchen genügende veresterungsgerechte (z. B. für die Herstellung von n-Butylacrylat, 2-Ethylhexylacrylat, Methylacrylat und Ethylacrylat) Acrylsäure (Reinheit ≥ 98 Gew.-%) bereits durch eine einzige Kristallisationsstufe erzielt werden kann. Zweckmäßigerweise wird diese Kristallisationsstufe als Suspensionskristallisation ausgeführt, wie es in Spalte 10 der DE-A 199 24 532 bzw. in Beispiel 1 der DE-A 102 23 058 beschrieben ist (z. B. in einem Kühlscheibenkristallisator wie in der WO 2006/111565 beschrieben). Die bei der Suspensionskristallisation entstehenden Acrylsäure-Kristalle haben eine würfel- bis quaderförmige Erscheinungsform. Das Länge (L) zu Dicke (D) Verhältnis liegt dabei üblicherweise im Bereich von L:D = 1:1 bis L:D = 6:1, bevorzugt im Bereich 1:1 bis 4:1, und besonders bevorzugt im Bereich 1,5:1 bis 3,5:1. Die Dicke D der Kristalle liegt üblicherweise im Bereich von 20 bis 600 µm, oft bei 50 bis 300 µm. Die Länge L der Kristalle liegt üblicherweise im Bereich von 50 bis 1500 µm, oft bei 200 bis 800 µm. Die Abtrennung des Suspensionskristallisats von der verbliebenen Mutterlauge kann im Fall von veresterungsgerechter Acrylsäure auf einer Zentrifuge (z. B. einer 2- oder 3-stufigen Schubzentrifuge) erfolgen, wobei die abgetrennten Kristalle in vorteilhafter Weise auf der Zentrifuge mittels aufgeschmolzenem Reinkristallisat gewaschen werden. Trennt man das Suspensionskristallisat von der verbliebenen Mutterlauge mittels einer Waschkolonne, z. B. eine Schmelz-Waschkolonne (z. B. einer solchen gemäß der WO 01/77056, oder der DE-A 101 56 016, oder der DE-A 102 23 058, oder wie in der WO 2006/111565, der WO 04/35514, der WO 03/41833, der WO 02/09839, der WO 03/41832, der DE-A 100 36 881, der WO 02/55469 sowie der WO 03/78378 beschrieben), kann mittels einer einzigen Kristallisationsstufe sogar superabsorbergerechte Acrylsäure (Reinheit ≥ 99,7 bzw. ≥ 99,9 Gew.-%), d. h., Acrylsäure, die sich zur Herstellung von Wasser superabsorbierenden oder sonstigen Polyacrylaten eignet, erzielt werden. In diesem Fall führt man in zweckmäßiger Weise die Gesamtmenge der abgetrennten Mutterlauge in die Kondensationskolonne zurück.

Die Kristallisation kann aber auch als fraktionierte Fallfilmkristallisation durchgeführt werden, wie sie die EP-A 616 998 empfiehlt. Diese kann z.B. zwei, drei oder mehr (z. B. 2 bis 4) Reinigungsstufen umfassen (diesbezüglich geeignete Fallfilmkristallisatoren können z.B. 1000 bis 1400 Kristallisationsrohre einer Länge von 10 bis 15 m und eines Außendurchmessers von 50 bis 100 mm enthalten). Die in einer höheren Reinigungsstufe abgetrennte Mutterlauge kann in eine der vorhergehenden Reinigungsstufen rückgeführt werden. Die in der ersten Reinigungsstufe abgetrennte Mutterlauge wird vorteilhaft vollständig in die Kondensationskolonne rückgeführt. Alternativ zur Rückführung in eine der vorhergehenden Reinigungsstufen können die Mutterlaugen der einzelnen Reinigungsstufen auch in ihrer Gesamtmenge in die Kondensationskolonne rückgeführt werden. Das Reinprodukt der vorletzten Reinigungsstufe kann vollständig oder nur teilweise der letzten Reinigungsstufe zugeführt werden. Erfolgt nur eine Teilzuführung wird man die verbleibende Restmenge in der Regel mit dem Reinprodukt der letzten Reinigungsstufe zum dann verbrauchsgerechten Endprodukt abmischen.

Erfindungsgemäß zweckmäßig wird man eine Teilmenge der über den ersten Seitenabzug entnommenen rohen Acrylsäure dem unterhalb des zu diesem gehörigen Fangbodens befindlichen Dual-Flow-Boden zuführen. Diesem Boden wird man in der Regel auch gegebenenfalls in die Kondensationskolonne rückzuführende Mutterlauge zuführen. Vorab der Zufuhr wird man die Mutterlauge in der Regel, wie bereits beschrieben, wärmeintegriert auf eine Temperatur erwärmen, die in etwa der Entnahmetemperatur der rohen Acrylsäure entspricht.

Eine andere Teilmenge der über den ersten Seitenabzug entnommenen rohen Acrylsäure wird man vorteilhaft durch indirekten Wärmeaustausch um 10 bis 15°C erwärmen und oberhalb der Entnahmestelle, bevorzugt unmittelbar unterhalb des ersten nachfolgenden Dual-Flow-Bodens, in die Kondensationskolonne rückführen. Diese Maßnahme wirkt sich günstig auf den Essigsäuregehalt der entnommenen rohen Acrylsäure aus.

Oberhalb des zweiten Fangbodens schließt sich zunächst eine zweite Serie von, zweckmäßigerweise äquidistanten, Dual-Flow-Böden an, die dann von hydraulisch abgedichteten Querstrom-Stoffaustauschböden (z. B. Thormann-Böden oder modifizierte Thormann-Böden gemäß DE-A 102 43 625), die zweckmäßigerweise gleichfalls äquidistant angeordnet sind, abgelöst werden. Der oberste Dual-Flow-Boden ist gegebenenfalls als Verteilerboden ausgerüstet. D.h., er weist z. B. Überlaufrinnen mit gezacktem Überlauf auf.

Der erste der Thormann-Böden von unten ist anwendungstechnisch zweckmäßig ein solcher, bei dem die vom Boden ablaufende Flüssigkeit über sechs als Rohre ausgebildete Ablaufschächte abläuft. Diese Rohre sind gegen den Gasraum des darunter liegenden Dual-Flow-Bodens hydraulisch abgedichtet. Die Wehrhöhe der sechs Ablaufrohre nimmt in Strömungsrichtung des Querstrombodens anwendungstechnisch zweckmäßig ab. Mit Vorteil weist die hydraulische Abdichtung Leerlauföffnungen mit Prallplatte auf. Die Ablaufrohre sind bevorzugt in der zweiten Hälfte, besonders bevorzugt im letzten Drittel des Bodenquerschnitts (dem Zulauf auf den Boden gegenüberliegend) gleichmäßig verteilt.

Die hydraulische Abdichtung erfolgt in eine Tasse mit schrägem Überlaufwehr (45°C).

Die Querstrom-Stoffaustauschböden werden mit einem dritten Kaminboden (Fangboden) abgeschlossen.

Oberhalb des dritten Fangbodens befinden sich, vorzugsweise zweiflutige, Ventilböden. Das Prinzip von Ventilböden sowie erfindungsgemäß verwendbare Ventilböden finden sich z.B. in Technische Fortschrittsberichte, Band 61, Grundlagen der Dimensionierung von Kolonnenböden, S. 96 bis 138. Sie sind im wesentlichen dadurch charakterisiert, dass sie dem durchströmenden Dampf über einen weiten Belastungsbereich eine der jeweiligen Belastung entsprechende Durchstromöffnung zur Verfügung stellen. Erfindungsgemäß bevorzugf werden Ballastböden verwendet. D. h., in den Öffnungen des Bodens befinden sich Käfige mit durch Gewichte verschlossenen Öffnungen. Erfindungsgemäß besonders bevorzugt sind VV12-Ventile der Fa. Stahl, DE, Viernheim. Im Ventilbodenraum kondensiert im wesentlichen Wasser sowie schwerer als Wasser flüchtige Bestandteile. Das dabei gewonnene Kondensat ist Sauerwasser. Vom dritten Fangboden wird in einem zweiten Seitenabzug kontinuierlich das Sauerwasser entnommen. Ein Teil des entnommenen Sauerwassers wird auf den obersten der Querstrom-Stoffaustauschböden in die Kondensationskolonne rückgeführt. Ein anderer Teil des entnommenen Sauerwassers wird durch indirekten Wärmetausch abgekühlt und, in zweckmäßiger Weise gesplittet, ebenfalls in die Kondensationskolonne rückgeführt. Eine gekühlte Teilmenge wird dabei auf den obersten Ventilboden (mit einer Temperatur von 15 bis 25, bevorzugt 20 bis 25°C) und die andere gekühlte Teilmenge auf einen zwischen dem dritten Fangboden und dem obersten Ventilboden etwa mittig gelegenen Ventilboden in die Kondensationskolonne rückgeführt (mit einer Temperatur von 20 bis 35, bevorzugt 25 bis 30°C). Aus der restlichen entnommenen Sauerwassermenge kann die enthaltene Acrylsäuremenge erfindungsgemäß abgetrennt werden.

Ein Teil der Abkühlung (die über einen oder mehrere hintereinandergeschaltete indirekte Wärmeaustauscher vorgenommen werden kann) wird dadurch bewirkt, dass die entsprechende Sauerwasserteilmenge über den Verdampfer des C₃-Vorläufers (z.B. den Propylenverdampfer) geführt wird, um flüssig gelagerten C₃-Vorläufer, z.B. Propylen, für die heterogen katalysierte Gasphasenoxidation in die Gasphase zu überführen.

Die leichter als Wasser flüchtigen Bestandteile werden am Kopf der Kondensationskolonne als Restgas (bzw. Restgasgemisch) gasförmig abgezogen und im Normalfall wenigstens teilweise als Verdünnungsgas (Kreisgas) in die Gasphasen-Partialoxidation rückgeführt. Um im Kreisgasverdichter Kondensation zu vermeiden, wird das Restgasgemisch zuvor durch indirekten Wärmeaustausch überhitzt. Der nicht im Kreis geführte Teil des Restgasgemisches wird normalerweise der Verbrennung zugeführt. Ein Teil des (vorzugsweise verdichteten) Restgasgemisches wird, wie bereits beschrieben, in vorteilhafter Weise als Strippgas zur Abtrennung von Acrylsäure aus dem Extrakt und aus der Sumpfflüssigkeit der Kondensationskolonne verwendet. Vorteilhaft wird die Gasphasen-Partialoxidation mit einem Überschuss an molekularem Sauerstoff durchgeführt, so dass das Restgasgemisch und damit das erste und das zweite Strippgas molekularen Sauerstoff enthalten, wenn Restgasgemisch als solches Strippgas verwendet wird.

Zum Zweck der Polymerisationsinhibierung wird dem obersten der hydraulisch abgedichteten Querstrom-Stoffaustauschböden eine Lösung von p-Methoxyphenol (= MEHQ) in Acrylsäure oder (erfindungsgemäß bevorzugt) eine MEHQ-Schmelze und (in beiden Fällen) gegebenenfalls zusätzlich eine Lösung von Phenothiazin in Acrylsäure zugeführt. Als Acrylsäure wird dabei vorzugsweise eine reine Acrylsäure verwendet, wie sie bei der Weiterreinigung der entnommenen rohen Acrylsäure erzeugt wird. Beispielsweise kann die im Rahmen der kristallisativen Weiterreinigung erzeugte Reinacrylsäure (Reinprodukt) verwendet werden. Diese Lösung wird zweckmäßig auch zur Reinproduktstabilisierung verwendet.

Zusätzlich wird etwa in der Mitte des Kolonnenabschnitts mit den hydraulisch abgedichteten Querstrom-Stoffaustauschböden eine Lösung von Phenothiazin (= PTZ) in Reinprodukt zugeführt.

Prinzipiell kann die Sauerwasserbildung z. B. auch hinter einer ersten Kondensationskolonne praktiziert werden (vgl. DE-A 102 35 847). In diesem Fall wird man aus dem dann am Kopf der ersten Kondensationskolonne entweichenden Leichtsiedergasstrom in zweckmäßiger Weise durch direkte Kühlung in einem an Einbauten freien (dieser kann zum Zweck der Sauerwasserbildung auch in die Kondensationskolonne integriert sein; normalerweise ist dann ein solcher an Einbauten freier Quenchraum z. B. über einen Kaminboden von der obersten trennwirksamen Einbaute innerhalb der Kondensationskolonne getrennt) oder Einbauten enthaltenden nachgeschalteten Raum (zweite Kolonne) mittels einer Quenchflüssigkeit 2 im wesentlichen Wasser auskondensieren. Das dabei gewonnene Kondensat ist wiederum das Sauerwasser. Einen Teil des Sauerwassers wird man dann in sinnvoller Weise zur Erhöhung der Trennleistung am Kopf der ersten Kondensationskolonne in selbige rückführen. Ein weiterer Teil des Sauerwassers wird in einem externen Wärmetauscher indirekt abgekühlt und als die Quenchflüssigkeit 2 verwendet und aus der restlichen Sauerwassermenge kann die Acrylsäure wiederum erfindungsgemäß extrahiert werden. Leichter als Wasser flüchtige Bestandteile des Leichtsiederstroms bilden wiederum Restgas, das normalerweise wenigstens teilweise als Kreisgas in die Gasphasen-Partialoxidation rückgeführt bzw. als Strippgas verwendet wird.

Zweckmäßig erstrecken sich die Dual-Flow-Böden bei der bevorzugten Variante des erfindungsgemäßen Verfahrens in der Kondensationskolonne in etwa bis zu dem Querschnitt in der Kondensationskolonne, von dem ab die Acrylsäuregehalte der Rücklaufflüssigkeit zum Kolonnenkopf hin betrachtet ≤ 90 Gew.-%, bezogen auf das Gewicht der Rücklaufflüssigkeit, betragen.

Die Anzahl an Dual-Flow-Böden beträgt, wie bereits gesagt, für die beschriebene Vorzugsvariante der fraktionierenden Kondensation in der Regel 25 bis 45. Ihr Öffnungsverhältnis liegt zweckmäßig bei 12 bis 25 %. Als Durchtrittsstellen weisen die Dual-Flow-Böden dabei bevorzugt kreisrunde Löcher mit einem einheitlichen Kreisdurchmesser auf. Letzterer beträgt zweckmäßig 10 bis 20 mm. Bei Bedarf kann man in der Kondensationskolonne die Lochdurchmesser von oben nach unten verjüngen oder vergrößern und/oder die Anzahl der Löcher vermindern oder vergrößern (z. B. kann der Lochdurchmesser einheitlich 14 mm betragen und das Öffnungsverhältnis von oben nach unten von 17,4 % auf 18,3 % zunehmen). Jedoch kann die Lochanzahl auch über alle Dual-Flow-Böden konstant sein. Ferner sind die kreisrunden Löcher über den individuellen Dual-Flow-Böden bevorzugt in strenger Dreiecksteilung gleichmäßig angeordnet (vgl. DE-A 102 30 219).

Außerdem zeigt der Stanzgrat der in den Dual-Flow-Böden herausgestanzten Durchtrittsöffnungen in der Kondensationskolonne bevorzugt nach unten (unerwünschte Polymerisatbildung wird dadurch gemindert).

Erfindungsgemäß sinnvoll ist es, wenn die Anzahl der in der Kondensationskolonne eingesetzten Dual-Flow-Böden etwa 10 bis 15 theoretischen Trennstufen entspricht.

Die Anzahl der sich an die Dual-Flow-Böden in der erfindungsgemäß bevorzugten Kondensationskolonne anschließenden hydraulisch abgedichteten Querstrom-Stoffaustauschböden wird, wie bereits erwähnt, in der Regel 30 bis 50 betragen. Ihr Öffnungsverhältnis wird zweckmäßig 5 bis 25 %, bevorzugt 10 bis 20 % betragen (das Öffnungsverhältnis gibt ganz generell den prozentualen Anteil der Durchtrittsquerschnitte am Gesamtquerschnitt wieder; es liegt bei den vorzugsweise mitzuverwendenden Querstrom-Stoffaustauschböden ganz generell zweckmäßig im vorgenannten Bereich).

Einflutige Querstrom-Stoffaustauschböden werden erfindungsgemäß bevorzugt eingesetzt.

In der Regel wird die Anzahl der hydraulisch abgedichteten Querstromböden für die Vorzugsvariante der fraktionierenden Produktgasgemischkondensation so bemessen, dass sie etwa 10 bis 30, häufig 25 theoretischen Trennstufen entspricht.

Sowohl die hydraulisch abgedichteten Querstromböden als auch gegebenenfalls mitverwendete Ventilböden weisen wenigstens einen Ablaufschacht auf. Sie können beide sowohl einflutig als auch mehrflutig, z. B. zweiflutig gestaltet sein. Dabei können sie auch bei einflutiger Gestaltung mehr als einen Ablaufschacht aufweisen. In der Regel sind auch die Zulaufschächte der Ventilböden hydraulisch abgedichtet.

Die Polymerisationsinhibierung des Quenchsystems 1 für das Produktgasgemisch der partiellen Gasphasenoxidation kann sowohl über in zum Quenchen verwendeter Sumpfflüssigkeit (aus der Kondensationskolonne) enthaltene Polymerisationsinhibitoren als auch über in zum Quenchen verwendeter Schwersiederfraktion (aus der Kondensationskolonne) enthaltene Polymerisationsinhibitoren bewerkstelligt werden.

Der Vorteil des erfindungsgemäßen Verfahrens liegt nochmals darin begründet, dass es bei im wesentlichen gleicher Reinheit eine erhöhte Ausbeute an roher Acrylsäure ermöglicht. Alle in dieser Schrift gemachten Aussagen gelten insbesondere für ein Produktgasgemisch, das durch (vorzugsweise zweistufige) heterogene Partialoxidation von Propylen zu Acrylsäure erhalten wurde. Die vorstehend beschriebene bevorzugte Ausführungsvariante des erfindungsgemäßen Verfahrens beschränkt in keinster Weise die allgemeine Ausführbarkeit.

Abschließend sei noch festgehalten, dass sowohl das erste Strippgas als auch das zweite Strippgas mit Vorteil molekularen Sauerstoff enthält.

Ferner sei noch ergänzt, dass beim erfindungsgemäßen Verfahren der Kondensationskolonne über eine zwischen ihrem ersten Seitenabzug und ihrem zweiten Seitenabzug gelegene Zufuhrstelle gegebenenfalls noch ein flüssiges (von der Rücklaufflüssigkeit verschiedenes) Absorptionsmittel zugeführt wird, dessen Siedetemperatur T_{S} bei einem Druck von 1 atm größer oder gleich der Siedetemperatur T_{W} von Wasser bei einem Druck von 1 atm ist. Grundsätzlich kann dabei z. B. so wie in der EP-A 1 818 324 beschrieben vorgegangen werden. Als solche Absorptionsmittel kommen z. B. höher siedende organische Flüssigkeiten in Betracht. Beispielsweise eignen sich diejenigen organischen Flüssigkeiten, die in der DE-A 103 36 386 und dem in dieser Schrift zitierten Stand der Technik als Absorptionsmittel empfohlen werden. Selbstredend können aber auch die in der EP-A 1 818 324 empfohlenen Absorptionsmittel an dieser Stelle eingesetzt werden.

Beispielsweise sind als solche Absorptionsmittel hochsiedende (inerte) flüssige hydrophobe organische Flüssigkeiten geeignet, wie sie in der EP-A 722 926, der DE-A 44 36 243 und der DE-A 103 36 386 aufgeführt sind (z. B. Dimethyphthalat, Diethylphthalat und/oder Diphyl). Das sind z. B. Flüssigkeiten, deren Siedetemperatur bei Normaldruck (1 atm) oberhalb der Siedetemperatur der Acrylsäure liegt und die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Als solche Absorptionsmittel beispielhaft genannt seien als Diphyl^{®} bezeichnete Gemische aus Diphenylether (70 bis 75 Gew.-%) und Diphenyl (25 bis 30 Gew.%) sowie das Gemisch aus einer Mischung aus 70 bis 75 Gew.-% Diphenylether und 25 bis 30 Gew.-% Diphenyl sowie, bezogen auf diese Mischung, 0,1 bis 25 Gew.-% Dimethylphthalat.

Selbstverständlich können als solche Absorptionsmittel auch wässrige Flüssigkeiten verwendet werden. Beispielsweise kommen als solche wässrigen Flüssigkeiten diejenigen in Betracht, die in der EP-A 1 818 324 empfohlen werden. Unter anderem gehört Wasser zu diesen Absorptionsmitteln. Selbstverständlich kann als ein solches wässriges Absorptionsmittel auch die bei der erfindungsgemäß durchzuführenden Sauerwasserextraktion extrahiert verbleibende wässrige Phase eingesetzte werden.

Üblicherweise wird die vorgenannte Absorptionsmittelzugabe den Zweck verfolgen, den Anteil an bestimmten, in der über den ersten Seitenabzug aus der Kondensationskolonne herausgeführten rohen Acrylsäure enthaltenen Nebenkomponenten gezielt zu senken. Es ist daher von Vorteil, wenn das eingesetzte Absorptionsmittel selbst diese Nebenkomponenten allenfalls in einem sehr geringen Gewichtsanteil (vorzugsweise überhaupt nicht) enthält.

Im Fall der Verwendung eines wässrigen Absorptionsmittels ist es z. B. günstig, wenn dessen individuelle Nebenkomponentengewichtsanteile kleiner als die jeweils entsprechenden des Sauerwasser sind.

Die Zufuhr wässriger Absorptionsmittel (z. B. von Wasser) eignet sich u. a. in besonders günstiger Weise, um den Gehalt an einen mit Acrylsäure vergleichbaren Siedepunkt aufweisenden Aldehyden (insbesondere den Gehalt der Furfurale) in der über den ersten Seitenabzug aus der Kondensationskolonne herausgeführten rohen Acrylsäure zu verringern. Grundsätzlich kann als ein solches Absorptionsmittel auch ein Gemisch aus einer wässrigen und aus einer organischen Flüssigkeit eingesetzt werden. Prinzipiell kann ein solches Gemisch auch mehrphasig sein.

Das Absorptionsmittel kann vorteilhaft auf derselben Höhe in die Kondensationskolonne geführt werden wie die Rücklaufflüssigkeit. Beide können auch im Gemisch in die Kondensationskolonne geführt werden.

Die Temperatur des Absorptionsmittels kann bei seiner Zufuhr in die Kondensationskolonne über einen weiten Bereich variieren. Sie kann sowohl oberhalb als auch unterhalb der Temperatur der Rücklaufflüssigkeit liegen. Häufig wird die Temperatur des Absorptionsmittels im Intervall ± 20 °C um die Temperatur der Rücklaufflüssigkeit herum liegen. Vorzugsweise sind die Temperatur der Rücklaufflüssigkeit und des Absorptionsmittels gleich.

Enthält die über den ersten Seitenabzug entnommene rohe Acrylsäure Teile von dem mitverwendeten Absorptionsmittel, so werden diese in der Regel über die in dieser Schrift beschriebene kristallisative Weiterreinigung der rohen Acrylsäure von selbiger abgetrennt. Ansonsten fällt ein-mitverwendetes Absorptionsmittel normalerweise in der aus der Kondensationskolonne herausgeführten Sumpfflüssigkeit an und kann nach einer Abtrennung von selbiger in der vorstehend beschriebenen Weise als Absorptionsmittel wiederverwendet werden.

Erfindungsgemäß zweckmäßig wird der Massenstrom eines in die Kondensationskolonne geführten Absorptionsmittels, bezogen auf den als Bestandteil des der Kondensationskolonne zugeführten Produktgasgemischs in die Kondensationskolonne geführten Massenstroms an Acrylsäure, 0 bis 30 % betragen.

Bezogen auf den zwischen dem ersten Seitenabzug und dem zweiten Seitenabzug aus der Kondensationskolonne gelegenen Längsabschnitt der Kondensationskolonne, wird man die Zufuhr eines Absorptionsmittels in die Kondensationskolonne anwendungstechnisch zweckmäßig in das obere Drittel dieses Längsabschnitts hinein vornehmen.

Anstatt auf eine erfindungsgemäß erhältlich rohe Acrylsäure kann die in dieser Schrift beschriebene kristallisative Weiterreinigung von roher Acrylsäure auch auf eine gemäß der in der DE-A 103 36 386 beschriebenen Verfahrensweise erhältliche rohe Acrylsäure angewendet werden (insbesondere eine solche kristallisative Weiterreinigung gemäß der Ansprüche 16 bis 20 der vorliegenden Schrift). Selbstverständlich kann eine solche kristallisative Weiterreinigung auch auf jedes beliebige Gemisch aus den beiden vorerwähnten Arten an roher Acrylsäure angewendet werden. Von einer solchen Verfahrensweise wird man z. B. dann Gebrauch machen, wenn das erfindungsgemäße Abtrennverfahren und ein Abtrennverfahren gemäß der DE-A 103 36 386 parallel betrieben werden, um Acrylsäure aus Produktgasgemischen von (gegebenenfalls parallel betriebenen) heterogen katalysierten partiellen Gasphasenoxidationen von C₃-Vorläufern der Acrylsäure abzutrennen. Für den Fall, dass der Betrieb der erfindungsgemäßen fraktionierenden Kondensation für eine solche Acrylsäureabtrennung dabei temporär außer Betrieb sein sollte, gemäß der DE-A 103 36 386 erzeugte rohe Acrylsäure aber weiterhin dieser kristallisativen Weiterreinigung zugeführt würde, würde man einen Teilmengenstrom (bezogen auf den der kristallisativen Weiterreinigung zugeführten Massenstrom an roher Acrylsäure einen Massenstrom von 5 bis 30 %, typisch 10 bis 20 % (das abgetrennte Kristallisat muss spezifikationsgerecht bleiben); den verbleibenden Restmengenstrom an Mutterlauge wird man normalerweise (gemeinsam (im Gemisch) mit dem Massenstrom an roher Acrylsäure) in die Kristallisation rückführen) der bei der Abtrennung des Suspensionskristallisats aus der Acrylsäurekristallisatsuspension anfallenden Mutterlauge in einem Lagertank zwischenspeichern (die Zuführung in den Lagertank kann kontinuierlich oder getaktet erfolgen) und nach erfolgter Wiederinbetriebnahme der fraktionierenden Kondensation sukzessive im Gemisch mit dann erfindungsgemäß anfallender Mutterlauge in die fraktionierende Kondensation rückführen (bezogen auf den dann an erfindungsgemäß anfallender Mutterlauge in die fraktionierende Kondensation rückgeführten Massenstrom in einem Massenstrom von > 0 bis 20 %, vorzugsweise > 0 bis 10 %). Die Entnahme aus dem Lagertank kann zu diesem Zweck kontinuierlich oder getaktet erfolgen. Normalerweise erfolgt sie so, dass das anfallende Kristallisat spezifikationsgerecht ist (die Design-Kapazität der Kristallisation liegt in der Regel oberhalb der Design-Kapazität der Herstellung der rohen Acrylsäure; kurzzeitig kann aber auch oberhalb der Design-Kapazität der Kristallisation kristallisativ weitergereinigt werden).

Damit umfasst vorliegende Erfindung insbesondere folgende Ausführungsformen:
1. Ein Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, die Temperatur des Acrylsäure, Wasserdampf und Nebenkomponenten enthaltenden Produktgasgemischs gegebenenfalls durch direkte und/oder indirekte Kühlung verringert, und das Acrylsäure, Wasserdampf und Nebenkomponenten enthaltende Produktgasgemisch anschließend in eine mit trennwirksamen Einbauten ausgerüstete Kondensationskolonne leitet, innerhalb der Kondensationskolonne in sich selbst aufsteigen lässt und dabei fraktionierend kondensiert sowie über einen ersten, oberhalb der Zuführstelle des Produktgasgemischs in die Kondensationskolonne befindlichen, Seitenabzug eine Wasser und Nebenkomponenten entreichert enthaltende rohe Acrylsäure als Zielprodukt und über einen zweiten, oberhalb des ersten Seitenabzugs befindlichen, Flüssigphasenabzug (vorzugsweise einen Seitenabzug) noch Acrylsäure und Nebenkomponenten enthaltendes saures Wasser und am Kopf der Kondensationskolonne ein tiefer als Wasser siedende Nebenkomponenten enthaltendes Restgasgemisch sowie aus dem Sumpfraum der Kondensationskolonne eine noch Acrylsäure und schwerer als Acrylsäure siedende Folgeprodukte und Nebenkomponenten enthaltende Sumpfflüssigkeit aus der Kondensationskolonne herausführt, eine Teilmenge des entnommenen sauren Wassers als solches und/oder nach Abkühlung desselben als Rücklaufflüssigkeit in die Kondensationskolonne rückführt und die rohe Acrylsäure gegebenenfalls wenigstens einem weiteren thermischen Trennverfahren zum Zweck ihrer Weiterreinigung unterwirft, dadurch gekennzeichnet, dass man wenigstens bei einer Teilmenge von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes aus dem sauren Wasser heraus in das organische Lösungsmittel aufnimmt, nachfolgend die Acrylsäure aus dem organischen Extrakt durch Strippen mit einem ersten Strippgas abtrennt und das mit Acrylsäure beladene erste Strippgas in die Kondensationskolonne rückführt und/oder die im ersten beladenen Strippgas enthaltene Acrylsäure in die wässrige Lösung eines Metallhydroxids aufnimmt oder das dabei anfallende mit Acrylsäure beladene erste Strippgas als ein zweites Strippgas dazu verwendet, um in aus der Kondensationskolonne herausgeführter Sumpfflüssigkeit noch enthaltene Acrylsäure freizustrippen und das dabei anfallende mit Acrylsäure beladene zweite Strippgas in die Kondensationskolonne rückführt und/oder die im zweiten Strippgas enthaltene Acrylsäure in die wässrige Lösung eines Metallhydroxids aufnimmt.
2. Ein Verfahren gemäß Ausführungsform 1, dadurch gekennzeichnet, dass der C₃-Vorläufer Propylen, oder Acrolein, oder ein Gemisch aus Propylen und Acrolein ist.
3. Ein Verfahren gemäß Ausführungsform 1 oder 2, dadurch gekennzeichnet, dass man bei wenigstens 25 Gew.-% von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes in das organische Lösungsmittel aufnimmt.
4. Ein Verfahren gemäß einer der Ausführungsformen 1 bis 3, dadurch gekennzeichnet, dass die Extraktion der Acrylsäure aus dem Sauerwasser in einer Extraktionskolonne durchgeführt wird, die als trennwirksame Einbauten Packungen und/oder Siebböden enthält.
5. Ein Verfahren gemäß Ausführungsform 4, dadurch gekennzeichnet, dass das organische Lösungsmittel am Kopf der Extraktionskolonne und das Sauerwasser im Sumpfbereich der Extraktionskolonne aufgegeben wird und das organische Lösungsmittel als dispers verteilte Phase in der kontinuierlichen Sauerwasserphase absteigt oder dadurch gekennzeichnet, dass das Sauerwasser am Kopf der Extraktionskolonne und das organische Lösungsmittel im Sumpfbereich der Extraktionskolonne aufgegeben wird, und das organische Lösungsmittel als dispers verteilte Phase in der kontinuierlichen Sauerwasserphase aufsteigt.
6. Ein Verfahren gemäß einer der Ausführungsformen 1 bis 5, dadurch gekennzeichnet, dass das organische Lösungsmittel wenigstens einen Ester aus einer 5 bis 20 C-Atome enthaltenden aliphatischen oder aromatischen Monocarbonsäure und einem 1 bis 8 C-Atome aufweisenden Alkohol enthält.
7. Ein Verfahren nach einer der Ausführungsformen 1 bis 5, dadurch gekennzeichnet, das das organische Lösungsmittel wenigstens einen Diester aus einer 5 bis 20 C-Atome enthaltenden aliphatischen oder aromatischen Dicarbonsäure und einem 1 bis 8 C-Atome aufweisenden Alkohol enthält.
8. Ein Verfahren gemäß einer der Ausführungsformen 1 bis 5 oder gemäß Ausführungsform 7, dadurch gekennzeichnet, dass das organische Lösungsmittel Dimethylphthalat, Diethylphthalat, Dimethylisophthalat, Diethylisophthalat, Dirnethyltherephthalat und/oder Diethyltherephthalat ist.
9. Ein Verfahren gemäß einer der Ausführungsformen 1 bis 8, dadurch gekennzeichnet, dass der Siedepunkt des organischen Lösungsmittels bei Atmosphärendruck ≥ 200 °C beträgt.
10. Ein Verfahren gemäß einer der Ausführungsformen 1 bis 9, dadurch gekennzeichnet, dass die Strippung der aus der Kondensationskolonne herausgeführten Sumpfflüssigkeit in einer mit trennwirksamen Einbauten versehenen Strippkolonne durchgeführt wird, und die Temperatur im Sumpf der Strippkolonne 150 bis 190 °C beträgt.
11. Ein Verfahren gemäß einer der Ausführungsformen 1 bis 10, dadurch gekennzeichnet, dass man das mit Acrylsäure beladene zweite Strippgas einer Gegenstrom-Rektifikation unterwirft, bevor es mit Acrylsäure beladen in die Kondensationskolonne rückgeführt und/oder die in ihm enthaltene Acrylsäure in die wässrige Lösung eines Metallhydroxids aufgenommen wird.
12. Ein Verfahren gemäß einer der Ausführungsformen 1 bis 11, dadurch gekennzeichnet, dass als erstes Strippgas Luft, N₂, CO₂ und/oder Wasserdampf verwendet wird.
13. Ein Verfahren gemäß einer der Ausführungsformen 1 bis 11, dadurch gekennzeichnet, dass als erstes Strippgas Restgasgemisch verwendet wird.
14. Ein Verfahren gemäß einer der Ausführungsformen 1 bis 13, dadurch gekennzeichnet, dass die rohe Acrylsäure kristallisativ weitergereinigt wird.
15. Ein Verfahren gemäß Ausführungsform 14, dadurch gekennzeichnet, dass der rohen Acrylsäure vorab der kristallisativen Weiterreinigung, eine Teilmenge des nicht in die Kondensationskolonne rückgeführten sauren Wassers zugeführt wird.
16. Ein Verfahren gemäß Ausführungsform 14 oder 15, dadurch gekennzeichnet, dass die kristallisative Weiterreinigung der rohen Acrylsäure oder deren Gemisch mit saurem Wasser durch Suspensionskristallisation erfolgt.
17. Ein Verfahren gemäß Ausführungsform 16, dadurch gekennzeichnet, dass zur Trennung von bei der Suspensionskristallisation verbliebener Mutterlauge und gebildetem Suspensionskristallisat eine Waschkolonne mitverwendet wird.
18. Ein Verfahren gemäß einer der Ausführungsformen 15 bis 17, dadurch gekennzeichnet, dass sich ein Verfahren der radikalischen Polymerisation anschließt, in welchem aufgeschmolzenes Acrylsäurekristallisat und/oder dessen Metallsalz miteinpolymerisiert wird.
19. Ein Verfahren gemäß einer der Ausführungsformen 1 bis 9 oder nach Ausführungsform 11, dadurch gekennzeichnet, dass sich ein Verfahren der radikalischen Polymerisation anschließt, in welchem aus beladenem erstem und/oder zweitem Strippgas in die wässrige Lösung eines Metallhydroxids aufgenommene Acrylsäure miteinpolymerisiert wird.
20. Ein Verfahren gemäß einer der Ausführungsformen 1 bis 19, dadurch gekennzeichnet, dass eine Teilmenge des Restgasgemischs als Kreisgas in die Gasphasen-Partialoxidation rückgeführt wird.
21. Ein Verfahren gemäß einer der Ausführungsformen 1 bis 20, dadurch gekennzeichnet, dass das organische Lösungsmittel eine Massendichte aufweist, die sich unter den Bedingungen der Extraktion von derjenigen von Wasser um ≥ 25 kg/m³ unterscheidet.
22. Ein Verfahren gemäß einer der Ausführungsformen 1 bis 21, dadurch gekennzeichnet, dass die wässrige Lösung eines Metallhydroxids NaOH, KOH, Ca(OH)₂ und/oder Mg(OH)₂ gelöst enthält.
23. Ein Verfahren gemäß einer der Ausführungsformen 1 bis 22, dadurch gekennzeichnet, dass der Kondensationskolonne über eine zwischen ihrem ersten Seitenabzug und ihrem zweiten Seitenabzug gelegene Zufuhrstelle ein flüssiges Absorptionsmittel zugeführt wird, dessen Siedetemperatur T_{S} bei einem Druck von 101.3 kPa (1 atm) größer oder gleich der Siedetemperatur T_{W} von Wasser bei einem Druck von 101.3 kPa (1 atm) ist.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

### Beispiele und Vergleichsbeispiel

### Beispiel 1 (beschrieben wird ein stationärer Zustand)

Aus einer zweistufig ausgeführten heterogen katalysierten Gasphasen-Partialoxidation von Propylen der Reinheit "Chemical grade" in drei parallel betriebenen Tandemreaktor-Reaktorstraßen wird ein eine Temperatur von 270°C und einen Druck von 1,5 bar aufweisendes gemeinsames Produktgasgemisch der nachfolgenden Zusammensetzung erhalten:

| | |
|---|---|
| 10,3141 Gew.-% | Acrylsäure, |
| 0,2609 Gew.-% | Essigsäure, |
| 4,6513 Gew.-% | Wasser, |
| 0,0251 Gew.-% | Ameisensäure, |
| 0,0851 Gew.-% | Formaldehyd, |
| 0,1052 Gew.-% | Acrolein, |
| 0,0024 Gew.-% | Propionsäure, |
| 0,0028 Gew.-% | Furfurale, |
| 0,0012 Gew.-% | Allylacrylat, |
| 0,0013 Gew.-% | Allylformiat, |
| 0,0032 Gew.-% | Benzaldehyd, |
| 0,1151 Gew.-% | Maleinsäureanhydrid, |
| 0,0096 Gew.-% | Benzoesäure, |
| 0,0126 Gew.-% | Phthalsäureanhydrid, |
| 2,0334 Gew.-% | CO₂, |
| 0,6604 Gew.-% | CO, |
| 0,6259 Gew.-% | Propan, |
| 0,1459 Gew.-% | Propylen, |
| 2,3772 Gew.-% | O₂, und |
| 78,5670 Gew.-% | N₂, |

Das den drei Tandemreaktor-Reaktorstraßen jeweils zuzuführende Reaktionsgasgemisch ist jeweils ein Gemisch aus Kreisgas und Chemical grade Propylen, dem nachfolgend noch Primärluft zudosiert wird. Die Mischung bewerkstelligt jeweils ein statischer Mischer.
Zwischen dem Erst- und dem Zweitstufenreaktor wird jeweils noch Sekundärluft zugeführt.

Die Mengenverhältnisse sind dem Betriebszustand der jeweiligen Reaktorstraße angepasst und betragen vor dem Erststufenreaktor:
A) Kreisgas = 25339 kg/h, Chemical grade Propylen = 3481 kg/h, Luft = 17791 kg/h.
B) Kreisgas = 38341 kg/h, Chemical grade Propylen = 4035 kg/h, Luft = 19561 kg/h.
C) Kreisgas = 30874kg/h, Chemical grade Propylen = 4689 kg/h, Luft = 23936 kg/h.

Die wesentlichen Gehalte der drei resultierenden Reaktionsgasgemischströme sind:

| | | |
|---|---|---|
| A) | 10,2909 Gew.-% | O₂, |
| | 1,2926 Gew.-% | CO₂, |
| | 0,4136 Gew.-% | CO, |
| | 0,6533 Gew.-% | Propan, |
| | 7,2976 Gew.-% | Propylen, |
| | 1,5383 Gew.-% | H₂O, und |
| | 78,2693 Gew.-% | N₂; |
| | | |
| B) | 8,9782 Gew.-% | O₂, |
| | 1,4660 Gew.-% | CO₂, |
| | 0,4710 Gew.-% | CO, |
| | 0,6744 Gew.-% | Propan, |
| | 6,3907 Gew.-% | Propylen, |
| | 1,6626 Gew.-% | H₂O, und |
| | 80,0789 Gew.-% | N₂; |
| | | |
| | und | |
| | | |
| C) | 10,6981 Gew.-% | O₂, |
| | 1,2357 Gew.-% | CO₂, |
| | 0,3948 Gew.-% | CO, |
| | 0,6500 Gew.-% | Propan, |
| | 7,6922 Gew.-% | Propylen, |
| | 1,4968 Gew.-% | H₂O, und |
| | 77,5992 Gew.-% | N₂. |

Das Produktgasgemisch (177184 kg/h) wird in einem im Gleichstrom betriebenen Sprühkühler (Quench 1) durch direkte Kühlung auf eine Temperatur von 107,3°C abgekühlt.

Die zur Direktkühlung des Produktgasgemischs zu verwendende Flüssigkeit (Quenchflüssigkeit 1) ist eine Teilmenge eines Gemisches 1 aus Sumpfflüssigkeit, die dem Sumpf der im folgenden beschriebenen Kondensationskolonne entnommen wird, und einer geringen Menge (251 kg/h) an aus dem Quenchkreis 0 entnommenem Kondensat.

Gehalte dieses Gemisches 1 (Temperatur = 104,9°C) sind:

| | | | |
|---|---|---|---|
| 64,6170 Gew.-% | Acrylsäure, | | |
| 0,3883 Gew.-% | Essigsäure, | | |
| 1,0300 Gew.-% | Wasser, | | |
| 0,0132 Gew.-% | Ameisensäure, | | |
| 0,0011 Gew.-% | Formaldehyd, | | |
| 0,0083 Gew.-% | Acrolein, | | |
| 0,0384 Gew.-% | Propionsäure, | | |
| 0,3331 Gew.-% | Furfurale, | | |
| 0,0016 Gew.-% | Allylacrylat, | | |
| 0,0008 Gew.-% | Allylformiat | | |
| 0,1609 Gew.-% | Benzaldehyd, | | |
| 4,4001 Gew.-% | Maleinsäureanhydrid, | | |
| 0,3976 Gew.-% | Benzoesäure, | | |
| 0,5228 Gew.-% | Phthalsäureanhydrid, | | |
| 12,8970 Gew.-% | Diacrylsäure, | 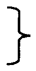 | Michael-Addukte, |
| 13,9178 Gew.-% | Polyacrylsäure | | |
| 0,1258 Gew.-% | Phenothiazin, | | |
| 0,4408 Gew.-% | MEHQ, | | |
| 0,7052 Gew.-% | sonstige hochsiedende Bestandteile, und | | |
| 0,0002 Gew.-% | Sauerstoff. | | |

Dem Sprühkühler des Quenchkreises 1 zur Direktkühlung des Produktgasgemischs wird mit der vorgenannten Temperatur nur eine Menge von 458 m³/h zugeführt 3137 kg/h werden als Zulauf der zweiten Strippkolonne zugeführt und 1195 kg/h werden dem Quenchkreis 0 zugeführt, um so die Quenchflüssigkeit 0 und die auf den obersten Boden der zweiten Strippkolonne zu führende Rücklaufflüssigkeit gegen unerwünschte Polymerisation zu inhibieren.

Das bei der Direktkühlung resultierende Gemisch aus auf 107,3 °C abgekühltem Produktgasgemisch und nicht verdampfter Quenchflüssigkeit 1 wird als solches in den Sumpf der Kondensationskolonne geführt. Der Druck im Sumpfraum und im Quench 1 beträgt 1,50 bar.

Der Innendurchmesser der Kondensationskolonne beträgt im Bereich der Thormann-Böden 6,5 m und ansonsten 6,0 m.

3137 kg/h des Gemischs 1 werden der zweiten Strippkolonne als Zulauf zugeführt, die als trennwirksame Einbauten 50 Dual-Flow-Böden enthält. Ebenso wie die Kondensationskolonne ist die zweite Strippkolonne gegen die Umgebung thermisch isoliert. Der Innendurchmesser der zweiten Strippkolonne beträgt über alle Dual-Flow-Böden einheitlich 2,4 m. Die Dual-Flow-Böden sind in der zweiten Strippkolonne äquidistant (400 mm) angeordnet. Ihre Öffnungsverhältnis beträgt einheitlich 12 %. Von unten nach oben betrachtet beträgt der Lochdurchmesser der ersten acht Dual-Flow-Böden einheitlich 14 mm (Lochanordnung entsprechend strenger Dreiecksteilung; Abstand von Lochmitte zu Lochmitte = 26 mm) und der Lochdurchmesser aller nachfolgenden Dual-Flow-Böden liegt einheitlich bei 14 mm (Lochanordnung ebenfalls entsprechend strenger Dreiecksteilung; Abstand von Lochmitte zu Lochmitte = 25 mm).

Die Zufuhr der 3137 kg/h des Gemischs 1 erfolgt mit einer Temperatur von 105,2 °C auf den achten Dual-Flow-Boden (von unten).

Die Energiezufuhr in die zweite Strippkolonne erfolgt mittels eines ausgelagerten Zwangsumlaufdreistromrohrbündelentspannungsverdampfers (vgl. Grundoperationen chemischer Verfahrenstechnik, 4. Auflage, Steinkopff Verlag Dresden, 1974, S. 434). Diesem werden 271650 kg/h an aus dem Sumpf der zweiten Strippkolonne mit einer Temperatur von 151,7 °C und einem Druck von 1,655 bar entnommener Sumpfflüssigkeit zugeführt, die folgende Gehalte aufweist:

| | | | |
|---|---|---|---|
| 2,0713 Gew.-% | Acrylsäure, | | |
| 0,2201 Gew.-% | Essigsäure, | | |
| 0,2608 Gew.-% | Wasser, | | |
| 0,0094 Gew.-% | Ameisensäure, | | |
| 0,0001 Gew.-% | Formaldehyd, | | |
| 0,0036 Gew.-% | Acrolein, | | |
| 0,0017 Gew.-% | Propionsäure, | | |
| 0,0001 Gew.-% | Furfurale, | | |
| 0,0008 Gew.-% | Allylformiat, | | |
| 0,5731 Gew.-% | Phthalsäurediethylester, | | |
| 0,7638 Gew.-% | Benzaldehyd, | | |
| 23,2670 Gew.-% | Maleinsäureanhydrid, | | |
| 2,2693 Gew.-% | Benzoesäure, | | |
| 2,9848 Gew.-% | Phthalsäureanhydrid, | | |
| 38,3947 Gew.-% | Diacrylsäure | 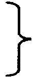 | Michael-Addukte, |
| 21,2842 Gew.-% | Polyacrylsäure | | |
| 0,7185 Gew.-% | Phenothiazin, | | |
| 2,5151 Gew.-% | MEHQ, | | |
| 4,6615 Gew.-% | sonstige hochsiedende Bestandteile, und | | |
| 0,0001 Gew.-% | Sauerstoff. | | |

Als Wärmeträger wird Wasserdampf (Druck = 16 bar) durch den die Wärmeaustauscherrohre umgebenden Raum geführt (mäanderformig, von entsprechenden Umlenkblechen geführt). Beim Durchströmen der Wärmeaustauscherrohre wird die Sumpfflüssigkeit erwärmt und 270900 kg/h der durch den Wärmeaustauscher geführten Gesamtmenge werden mit einer Temperatur von 158,6 °C in den Sumpf der zweiten Strippkolonne rückgeführt. 750 kg/h der durch den Wärmeaustauscher insgesamt geführten Menge an Sumpfflüssigkeit werden abgezweigt, entgast und mit Methanol verdünnt der Rückstandsverbrennung zugeführt.

Zusätzlich werden in den Sumpf der zweiten Strippkolonne mit einer Temperatur von 82,2 °C und einem Druck von ca. 2,50 bar 17424 kg/h aus der ersten Strippkolonne an deren Kopf herausgeführtes erstes beladenes Gas zugeführt, das folgende Gehalte aufweist:

| | |
|---|---|
| 3,3977 Gew.-% | Acrylsäure, |
| 1,1198 Gew.-% | Essigsäure, |
| 4,1030 Gew.-% | Wasser, |
| 0,0622 Gew.-% | Ameisensäure, |
| 0,0321 Gew.-% | Formaldehyd, |
| 0,1181 Gew.-% | Acrolein, |
| 0,0030 Gew.-% | Propionsäure, |
| 0,0006 Gew.-% | Furfurale, |
| 0,0139 Gew.-% | Allylformiat, |
| 0,0247 Gew.-% | Phthalsäurediethylester, |
| 2,5667 Gew.-% | O₂, |
| 2,1951 Gew.-% | CO₂, |
| 0,7129 Gew.-% | CO, |
| 0,6756 Gew.-% | Propan, |
| 0,1575 Gew.-% | Propylen, und |
| 84,8133 Gew.-% | N₂. |

Aus dem Kopf der zweiten Strippkolonne wird in einer Menge von 25831 kg/h zweites beladenes Gas herausgeführt (Temperatur = 96,7 °C, Druck = 1,58 bar) und in einem im Gegenstrom betriebenen Sprühkühler (Quench 0) durch direkte Kühlung auf eine Temperatur von 80,9 °C abgekühlt und partiell kondensiert.

Das bei der Direktkühlung verbleibende Gasgemisch wird mit einem Druck von 1,58 bar in einer Menge von 20755 kg/h und mit den nachfolgenden Gehalten in den Sumpfraum der Kondensationskolonne (nicht getaucht) rückgeführt:

| | |
|---|---|
| 18,5793 Gew.-% | Acrylsäure, |
| 0,9849 Gew.-% | Essigsäure, |
| 3,6185 Gew.-% | Wasser, |
| 0,0571 Gew.-% | Ameisensäure, |
| 0,0271 Gew.-% | Formaldehyd, |
| 0,1006 Gew.-% | Acrolein, |
| 0,0099 Gew.-% | Propionsäure, |
| 0,0436 Gew.-% | Furfurale, |
| 0,0003 Gew.-% | Allylacrylat, |
| 0,0117 Gew.-% | Allylformiat, |
| 0,0053 Gew.-% | Benzaldeyd, |
| 0,0642 Gew.-% | Maleinsäureanhydrid, |
| 0,0001 Gew.-% | Benzoesäure, |
| 0,0001 Gew.-% | Diacrylsäure, |
| 0,0001 Gew.-% | MEHQ, |
| 2,1548 Gew.-% | O₂, |
| 1,8428 Gew.-% | CO₂, |
| 0,5985 Gew.-% | CO, |
| 0,5672 Gew.-% | Propan, |
| 0,1322 Gew.-% | Propylen, und |
| 71,2018 Gew.-% | N₂. |

Als Quenchflüssigkeit 0 werden 32956 kg/h einer Mischung aus 1195 kg/h aus Gemisch 1 und 37761 kg/h an im Quench 0 bei der Direktkühlung gebildetem Kondensat verwendet (dazu wird diese Teilmenge der Mischung in einem indirekten Wärmeaustauscher (Typ: Spiralwärmetauscher; gegen Wasser) von 80,9 °C auf 40,1 °C abgekühlt). 6020 kg/h dieser Mischung, die eine Temperatur von 80,9 °C aufweist, wird als Rücklaufflüssigkeit auf den obersten Boden der zweiten Strippkolonne geführt.

Die Mischung weist folgende Gehalte auf:

| | | | |
|---|---|---|---|
| 87,9166 Gew.-% | Acrylsäure, | | |
| 2,3313 Gew.-% | Essigsäure, | | |
| 2,6130 Gew.-% | H₂O, | | |
| 0,0688 Gew.-% | Ameisensäure, | | |
| 0,0010 Gew.-% | Formaldehyd, | | |
| 0,0151 Gew.-% | Acrolein, | | |
| 0,0480 Gew.-% | Propionsäure, | | |
| 0,2980 Gew.-% | Furfurale, | | |
| 0,0008 Gew.-% | Allylacrylat, | | |
| 0,0096 Gew.-% | Allylformiat, | | |
| 0,0568 Gew.-% | Benzaldehyd, | | |
| 1,1099 Gew.-% | Maleinsäureanhydrid, | | |
| 0,0761 Gew.-% | Benzoesäure, | | |
| 0,1000 Gew.-% | Phthalsäureanhydrid, | | |
| 2,4592 Gew.-% | Diacrylsäure | 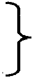 | Michael-Addukte, |
| 2,6522 Gew.-% | Polyacrylsäure | | |
| 0,0240 Gew.-% | Phenotiazin, | | |
| 0,0847 Gew.-% | MEHQ, | | |
| 0,1345 Gew.-% | sonstige hochsiedende Bestandteile, und | | |
| 0,0004 Gew.-% | Sauerstoff. | | |

In den Sumpfraum der Kondensationskolonne ist ein Zentrifugaltropfenabscheider integriert, der verhindert, dass Tropfen der Sumpfflüssigkeit aus dem Sumpfraum heraus nach oben mitgerissen werden.

Der Sumpfraum der Kondensationskolonne ist, wie bereits erwähnt, auf einer Kolonnenhöhe (wie alle Höhen vom Sumpfboden aus gerechnet) von 7,80 m durch einen ersten Fangboden (Sammelboden; Kaminböden mit 16 etwa gleichverteilten bedachten Kaminen; Kamindurchmesser: 600 mm; Kaminhöhe: 1 m) abgeschlossen.

Der Sammelboden ist doppelwandig mit 2° Gefälle nach Innen und mit zentraler Abzugstasse und Abzugsstutzen (DN ~ 200) gestaltet. Der freie Gasquerschnitt beträgt ca. 30 %.

Von diesem ersten Fangboden werden 88579 kg/h Schwersiederfraktion in den unter dem ersten Fangboden befindlichen Sumpfraum geführt.

Die Schwersiederfraktion weist bei einer Temperatur von 99,8°C und einem Druck von ca. 1,50 bar folgende Gehalte auf:

| | |
|---|---|
| 94,6665 Gew.-% | Acrylsäure, |
| 0,5402 Gew.-% | Essigsäure, |
| 1,3577 Gew.-% | Wasser, |
| 0,0160 Gew.-% | Ameisensäure, |
| 0,0014 Gew.-% | Formaldehyd, |
| 0,0071 Gew.-% | Acrolein, |
| 0,0577 Gew.-% | Propionsäure, |
| 0,3814 Gew.-% | Furfurale, |
| 0,0023 Gew.-% | Allylacrylat, |
| 0,0010 Gew.-% | Allylformiat, |
| 0,1279 Gew.-% | Benzaldehyd, |
| 2,1925 Gew.-% | Maleinsäureanhydrid, |
| 0,0051 Gew.-% | Benzoesäure, |
| 0,0046 Gew.-% | Phthalsäureanhydrid, |
| 0,6008 Gew.-% | Diacrylsäure, |
| 0,0061 Gew.-% | Phenotiazin, |
| 0,0314 Gew.-% | MEHQ, und |
| 0,0002 Gew.-% | O₂. |

Die Sumpftemperatur beträgt 104,9°C und der Sumpfdruck (am Flüssigkeitsspiegel) liegt bei 1,51 bar.

2,0 m oberhalb des ersten Fangbodens befindet sich der erste von zunächst 15 Dual-Flow-Böden. Diese Dual-Flow-Böden (Lochanzahl einheitlich 33678) sind äquidistant angebracht mit einem Bodenabstand von 380 mm. Die Durchtrittsöffnungen bestehen aus kreisrunden Öffnungen des einheitlichen Durchmessers von 14 mm, wobei der Stanzgrat in der Trennkolonne nach unten zeigt. Die Anordnung der Mittelpunkte der Durchtrittskreise folgt einer strengen Dreiecksteilung. Der nächstliegende Abstand zweier Kreismittelpunkte liegt bei 24,5 mm.

Der fünfzehnte Dual-Flow-Boden fungiert als Verteilerboden. Zu diesem Zweck enthält die Kolonnenwand zwischen dem zweiten Fangboden und dem fünfzehnten Dual-Flow-Boden zwei Einsteckrohre (DN ~ 150) mit 45 Auslaufbohrungen (Durchmesser: 15 mm) je Einsteckrohr.

Über die Einsteckrohre wird rohe Acrylsäure und Mutterlauge in die Kondensationskolonne rückgeführt.

Die erste Serie von Dual-Flow-Böden wird mit einem zweiten Fangboden (Sammelboden; Kaminboden mit 16 ca. gleichmäßig verteilten bedachten Kaminen; Kaminhöhe ca. 1,70 m, Zentrale Abzugstasse mit Abzugsstutzen (DN ~ 250), freier Gasquerschnitt von ~ 30 %) abgeschlossen, der 1,50 m oberhalb des letzten Dual-Flow-Bodens untergebracht ist.

Von diesem zweiten Fangboden wird als erster Seitenabzug bei 1,48 bar kontinuierlich rohe Acrylsäure mit einer Temperatur von 97,1 °C entnommen, die folgende Gehalte aufweist:

| | |
|---|---|
| 96,7716 Gew.-% | Acrylsäure, |
| 0,8253 Gew.-% | Essigsäure, |
| 1,6640 Gew.-% | Wasser, |
| 0,0213 Gew.-% | Ameisensäure, |
| 0,0018 Gew.-% | Formaldehyd, |
| 0,0070 Gew.-% | Acrolein, |
| 0,0681 Gew.-% | Propionsäure, |
| 0,1642 Gew.-% | Furfurale, |
| 0,0027 Gew.-% | Allylacrylat, |
| 0,0012 Gew.-% | Allylformiat, |
| 0,0164 Gew.-% | Benzaldehyd, |
| 0,1052 Gew.-% | Maleinsäureanhydrid, |
| 0,3278 Gew.-% | Diacrylsäure, |
| 0,0050 Gew.-% | Phenothiazin, |
| 0,0180 Gew.-% | MEHQ, und |
| 0,0002 Gew.-% | Sauerstoff. |

18474 kg/h der dem zweiten Fangboden entnommenen rohen Acrylsäure werden gemeinsam mit im Rahmen der kristallisativen Weiterreinigung von entnommener roher Acrylsäure anfallender und im indirekten Wärmeaustausch mit entnommener roher Acrylsäure und Wasserdampf als Wärmeträger auf 90 °C erwärmter Mutterlauge (72716 kg/h) über vorgenannte Einsteckrohre unmittelbar unterhalb des zweiten Fangbodens auf den diesem nach unten folgenden Dual-Flow-Boden in die Kondensationskolonne rückgeführt.

89303 kg/h der dem zweiten Fangboden entnommenen rohen Acrylsäure werden mehrstufig durch indirekten Wärmeaustausch (u. a. wärmeintegriert gegen in die Kondensationskolonne rückzuführende vorstehend erwähnte Mutterlauge) auf eine Temperatur von 29 °C abgekühlt und gegebenenfalls in einem Tanklager zwischengepuffert. Dann werden der abgekühlten rohen Acrylsäure 1204 kg/h aus dem zweiten Seitenabzug der Kondensationskolonne entnommenes Sauerwasser zugefügt.

Das Sauerwasser weist folgende Gehalte auf:

| | |
|---|---|
| 10,7677 Gew.-% | Acrylsäure, |
| 6,4390 Gew.-% | Essigsäure, |
| 79,5610 Gew.-% | Wasser, |
| 0,7038 Gew.-% | Ameisensäure, |
| 2,47712 Gew.-% | Formaldehyd, |
| 0,0132 Gew.-% | Acrolein, |
| 0,0082 Gew.-% | Propionsäure, |
| 0,0013 Gew.-% | Furfurale, |
| 0,0331 Gew.-% | Allylformiat, |
| 0,0001 Gew.-% | MEHQ, und |
| 0,0013 Gew.-% | Sauerstoff. |

Das resultierende Gemisch wird durch nochmaligen indirekten Wärmeaustausch (gegen Kühlsole (Wasser/Glykol-Gemisch; 25-35 Gew.-% Glykol und 65-75 Gew.-% Wasser) auf 16 °C abgekühlt und dann in zwei bis drei parallel betriebene Kühlscheibenkristallisatoren (vgl. WO 2006/111565) verteilt geführt. Bei diesen handelt es sich jeweils um einen Trog, in welchem 24 gewischte kreisförmige Kühlplatten (die innerlich jeweils von einem Kühlmedium (Gemisch aus Wasser und Glykol; Glykolanteil = 25 bis 35 Gew.-%) durchflossen werden) im äquidistanten Abstand von 30 ± 1 cm hintereinander hängend angeordnet sind (Plattendurchmesser = 3,3 m). Das jeweilige Kühlmedium wird dabei im Gegenstrom zum kristallisierenden Gemisch durch den jeweiligen Kristallisator von Kühlscheibe zu übernächster Kühlscheibe weitergereicht. D.h., das jeweilige Kühlmedium wird in Gestalt zweier paralleler Ströme über die Kühlplatten des jeweiligen Kristallisators aufgeteilt geführt. Ein Strom führt durch die numerisch geraden Kühlplatten, der andere Strom führt durch die numerisch ungeraden Kühlplatten (Bezifferung der Kühlscheiben in Strömungsrichtung mit 1 beginnend). Die jeweilige Kühlmediummenge beträgt je Kristallisator insgesamt 180-220 t/h (metric tons), d. h., je Strom 90-110 t/h. Der Druckverlust beträgt je Kühlscheibe 60 bis 100 mbar. Die Eintrittstemperatur des Kühlmediums (der Sole) beträgt +2,5 bis +3 °C. Die Austrittstemperatur liegt 2,5 °C höher. Die Wanddicke der aus Edelstahl gefertigten Kühlflächen liegt bei 4 mm. Der Wärmeübergangskoeffizient liegt soleseitig bei etwa 1500 bis 2500 W/(m²•K). Die Wärmedurchgangskoeffizienten liegen meist bei 380 bis 420 W/(m²•K). Die spezifische Kühlleistung beträgt 1,5 ± 0,2 kW/m² Kühlfläche. Durch das Wischen der Kühlplatten wird die Ausbildung einer Kristallschicht unterdrückt. Die angewässerte rohe Acrylsäure wird von hinten nach vorne kontinuierlich durch den jeweiligen Kristaller geführt (gepumpt oder überlaufgeregelt). Die einphasige angewässerte rohe Acrylsäure verdickt dabei (Verweilzeit 2,5 h) zu einer zweiphasigen, Acrylsäurekristalle als feste Phase enthaltenden Suspension einer Temperatur von 7 bis 8,5°C und einem Feststoffgehalt am Austritt von etwa 25 Gew.-%. Die Massendichte der Suspension liegt üblicherweise bei 1110 bis 1115 kg/m³. Die Drehzahl der Wischer beträgt 5 bis 6 Umdrehungen je Minute. Die die Wischer treibende, zentriert durch die Kühlscheiben geführte Welle, ist mit wassergespülten Stopfbuchspackungen (Packungsschnüre aus Teflon oder Graphit, Spülmenge = wenige Liter pro Stunde bis einige 10 l/h je Dichtung) abgedichtet.

Auf dem Umfang der Kühlscheiben, wo nicht gewischt werden kann, ist ein Hohlprofil (z. B. in einfachster Ausführungsform ein Rohr) aufgebracht (z. B. aufgeschweißt), das mittels eines zweiten Wärmeträgers (z. B. ebenfalls Wasser/Glykol Gemisch) beheizt wird (auf eine Temperatur oberhalb der Kristallisationstemperatur; meist aus den Temperaturbereich 8 bis 20 °C, bevorzugt 10 bis 14 °C). Diese Umfangbeheizungen werden mit dem zweiten Wärmeträger parallel angeströmt.

Darüber hinaus sind die Wischer in radialer Richtung bevorzugt segmentiert (4 Segmente). Die spezifische Anpresskraft der Wischer liegt im eingebauten Zustand senkrecht zur Kühlfläche bei 3 bis 5 N pro cm aktiver Wischkantenlänge. Als Wischermaterial wird High Molecular Weight Polyethylene oder Ultra High Molecular Weight Polyethylene, z.B. Multilene^{®} PE 1000, verwendet. Zusätzlich zu den Wischern treibt die Welle Paddel an (zwischen zwei Kühlscheiben und vor der ersten und letzten Kühlscheibe zweckmäßig jeweils zwei in symmetrischer Anordnung), die eine verbesserte Durchmischung bewirken.

Im in Förderrichtung der Suspension hinteren Teil des jeweiligen Kristallisators (bevorzugt hinter der letzten Kühlscheibe) wird die Suspension über ein angeschlossenes Rohr (zweckmäßigerweise getaucht angebracht; alternativ kann die Suspension über ein Überlaufwehr in einen gerührten Sammelbehälter fließen, von dem aus die Waschkolonnen beschickt werden) zu hydraulischen Schmelze-Waschkolonnen geführt, wie sie in der EP-A 1 272 453, EP-A 1 448 283, WO 03/041833, EP-A 1 305 097, DE-A 101 56 016, DE-A 10 2005 018 702 und in der DE-A 102 23 058 beschrieben sind, um die Mutterlauge vom Suspensionskristallisat abzutrennen. Der Waschkolonnendurchmesser beträgt 1,4 m. Die Beschickung der Waschkolonnen mit Kristallsuspension erfolgt mittels einer Kreiselpumpe (Typ Kanalrad), wobei die Mengensteuerung bevorzugt über eine Drehzahlregelung der Pumpe erfolgt. Die Steuerstrompumpe ist ebenfalls als Kreiselpumpe mit Regelventil ausgeführt. Üblicherweise liegt die zur Regelung einer Waschkolonne angewandte Steuerstrommenge bei 5 bis 60 t/h, meist 8 bis 30 t/h. Teilweise ist es möglich, die jeweilige Waschkolonne ohne Steuerstrom zu betreiben, wenn die mit der Suspension zugeführte Flüssigkeitsmenge bereits für den Transport des Kristallbetts ausreicht. Übliche Verhältnisse von wirksamer Transportdruckdifferenz zu wirksamer Waschdruckdifferenz liegen bei 1,1 bis 3, meist bei 1,2 bis 1,8. Die Messerdrehzahl liegt meist bei Werten von 5 bis 10 pro Minute. Die Temperatur im Schmelzkreis beträgt normalerweise 13 bis 16°C. Die Erfassung der Filtrationsfront wird gemäß der DE-A 10 2005 018 702 über zwei zueinander ins Verhältnis gesetzte Druckverlustmessungen über unterschiedliche Bettlängen vorgenommen. Die Waschfront wird mittels Temperaturmessung im Kristallbett geregelt.

Die Gesamthöhe des Kristallbetts liegt regelungsbedingt bei 250 bis 1500 mm, meist bei 600 bis 1100 mm. Die Waschfront befindet sich typisch 100 bis 200 mm oberhalb des Messers. Als Schmelzkreispumpe eignet sich eine Kreiselpumpe mit produktseitiger Spülung der Wellenabdichtung (Gleitringdichtung; doppelt ausgeführt, mit auf 15 - 30 °C gekühltem Sperrmedium (Wasser/Glykol-Gemisch)) oder eine magnetgekuppelte Pumpe mit erhöhter Gleitlagerspülung. Die Umlaufmenge im jeweiligen Schmelzkreis beträgt 10 bis 15 m³/h pro Tonne mit dem Messer abgetragenem gereinigtem Kristallisat. Die Stabilisierung des Schmelzkreises erfolgt in Abhängigkeit von der Nachfolgeverwendung kolonnenspezifisch mit 200 bis 300 Gew.-ppm MEHQ, oder mit 40 bis 70 Gew.-ppm MEHQ, oder mit 100 bis 300 Gew.-ppm PTZ. Zusätzlich wird in den Schmelzkreis Luft eingetragen oder Magerluft (Stickstoff-Luft-Mischung mit ≤ 6 Vol.-% Sauerstoff), deren Überschuss (= nicht in der Waschschmelze gelöster Anteil) vor Eintritt der Waschschmelze in die Waschkolonne via Gasabscheider abgetrennt wird. Dadurch stellt sich ein Gehalt an gelöstem Sauerstoff von 5 bis 40 Gew.-ppm im geschmolzenen Reinprodukt ein.
[a) Zur Herstellung einer veresterungsgerechten Acrylsäure ist es ausreichend die Abtrennung des Suspensionskristallisats anstelle in einer Schmelze-Waschkolonne mittels einer Zentrifuge (z. B. einer 2- oder 3-stufigen Schubzentrifuge) durchzuführen. Geeignete Siebspaltweiten liegen bei 150 bis 300 µm; anwendbare Zentrifugalbeschleunigungen liegen bei 500 bis 900 g, meist 600 bis 800 g; geeignet sind Hubzahlen von 40 bis 80 Schüben/min..
   Vorzugsweise werden die auf der 2. oder 3. Stufe der Zentrifuge abgetrennten Kristalle mit 0,15 bis 0,3 kg Waschflüssigkeit pro kg Kristallisat gewaschen. Die Temperatur der Waschflüssigkeit liegt bei 15 bis 30 °C, bevorzugt bei 20 bis 30 °C. Zur Vermeidung von Ablagerungen wird der Feststoffabwurfschacht der Zentrifuge mit auf 15 bis 30 °C temperierter Spülflüssigkeit gespült. Spül- und Waschflüssigkeit sind bevorzugt aufgeschmolzenes, über die Zentrifuge abgetrenntes und gewaschenes Kristallisat. Zur Vermeidung von Ablagerungen und Verkrustungen ist es zweckmäßig, das Zentrifugengehäuse, das Suspensionszuführrohr und das Waschflüssigkeitszuführrohr auf eine Temperatur ≥ 15 °C und ≤ 40 °C zu temperieren. Der Produktraum der Zentrifuge wird zweckmäßig mit Stickstoff oder mit einem Gemisch aus Luft und Stickstoff inertisiert. Die Wellenabdichtung wird mit Gas (z. B. Stickstoff oder ein Gemisch aus Luft und Stickstoff) oder mit Wasser gespült.
b) Alternativ zur Suspensionskristallisation kann auch eine Schichtkristallisation (z. B. Fallfilmkristallisation gemäß EP-A 616 998 oder voll durchströmtes Rohr) mit 2 bzw. 3 oder mehr (z. B. 2 bis 4) Reinigungsstufen angewendet werden. Anstelle die Mutterlauge einer nachgehenden Reinigungsstufe in eine vorgehende Reinigungsstufe rückzuführen, kann man sie auch gemeinsam in die Kondensationsskolonne rückführen.]

Den Schmelzkreisen, die durch den Zusatz von insgesamt 104 kg/h einer Lösung (Temperatur = 25 °C, Druck = 1,1 bar) von 3 kg/h MEHQ in 101 kg/h aus den Schmelzkreisen entnommener Reinacrylsäure (25 °C) stabilisiert werden, werden 17894 kg/h an Reinacrylsäure (Temperatur = 14 °C, Druck = 1,5 bar) der nachfolgenden Gehalte entnommen:

| | |
|---|---|
| 99,7334 Gew.-% | Acrylsäure, |
| 0,2091 Gew.-% | Essigsäure, |
| 0,0180 Gew.-% | Wasser, |
| 0,0230 Gew.-% | Propionsäure, |
| 0,0001 Gew.-% | Furfurale, |
| < 0,0001 Gew.-% | Benzaldehyd, |
| 0,0001 Gew.-% | Maleinsäureanhydrid, |
| 0,0002 Gew.-% | Diacrylsäure, |
| 0,0150 Gew.-% | MEHQ, und |
| 0,001 Gew.-% | O₂. |

Sie eignet sich in hervorragender Weise zur Herstellung von Superabsorbern auf der Basis von Poly-Na-Acrylat.

In 352 kg/h der vorgenannten erwärmten Reinacrylsäure werden 5 kg/h PTZ zur Herstellung einer 25 °C aufweisenden Inhibitorlösung 1 gelöst. In 30 kg/h Inhibitorlösung 1 werden 19 kg/h MEHQ unter Bildung der ebenfalls 25 °C aufweisenden Inhibitorlösung 2 gelöst.

17439 kg/h an mit MEHQ stabilisierter Reinacrylsäure (25 °C, 1,5 bar) werden kontinuierlich dem Lagertank zugeführt.

Die in den Waschkolonnen abgetrennte Mutterlauge wird zunächst in einen beheizbaren Sammelbehälter und von dort in einen Tank gefahren. Von diesem wird sie (wie bereits erwähnt) wärmeintegriert auf 90 °C erwärmt und in einer Menge von 72716 kg/h gemeinsam mit 18474 kg/h an dem zweiten Fangboden entnommener roher Acrylsäure auf den fünfzehnten Dual-Flow-Boden der Kondensationskolonne (von unten gerechnet) rückgeführt. Die Zusammensetzung dieser rückgeführten Mutterlauge ist wie folgt:

| | |
|---|---|
| 94,6188 Gew.-% | Acrylsäure, |
| 1,0690 Gew.-% | Essigsäure, |
| 3,3562 Gew.-% | Wasser, |
| 0,0378 Gew.-% | Ameisensäure, |
| 0,0431 Gew.-% | Formaldehyd, |
| 0,0088 Gew.-% | Acrolein, |
| 0,0782 Gew.-% | Propionsäure, |
| 0,2016 Gew.-% | Furfurale, |
| 0,0034 Gew.-% | Allylacrylat, |
| 0,0021 Gew.-% | Allylformiat, |
| 0,0202 Gew.-% | Benzaldehyd, |
| 0,1292 Gew.-% | Maleinsäureanhydrid, |
| 0,4025 Gew.-% | Diacrylsäure, |
| 0,0061 Gew.-% | Phenothiazin, |
| 0,0227 Gew.-% | MEHQ, und |
| 0,0003 Gew.-% | Sauerstoff. |

2,9 m oberhalb des zweiten Fangbodens befindet sich in der Kondensationskolonne der erste von 21 weiteren Dual-Flow-Böden der bereits beschriebenen Art (Lochdurchmesser wieder einheitlich 14 mm, Lochanzahl jedoch einheitlich 32020, nächstliegender Abstand zweier Durchtrittskreismittelpunkte = 24,5mm), die wieder äquidistant mit einem Bodenabstand von 380 mm angeordnet sind.

800 mm oberhalb des letzten Dual-Flow-Bodens beginnt sich die Kondensationskolonne konisch zu erweitern. 500 mm oberhalb des letzten Dual-Flow-Bodens endet diese Erweiterung bei einem Kolonneninnendurchmesser von 6,50 m.

Auf dieser Höhe, d.h., 1,50 m oberhalb des letzten Dual-Flow-Bodens, beginnt eine äquidistante (Bodenabstand = 500 mm) Anordnung von 28 konventionellen, einflutigen Thormann-Böden. Der erste der Thormann-Böden von unten ist ein solcher, bei dem die vom Boden ablaufende Flüssigkeit über sechs als Rohre ausgebildete Ablaufschächte abläuft. Diese Rohre sind gegen den Gasraum des darunter liegenden Dual-Flow-Bodens hydraulisch abgedichtet. Die Wehrhöhe der sechs Ablaufrohre nimmt in Strömungsrichtung des Querstrombodens ab. Die hydraulische Abdichtung weist Leerlauföffnungen mit Prallplatte auf. Die Ablaufrohre sind im letzten Drittel des Bodenquerschnitts (dem Zulauf auf den Boden gegenüberliegend) gleichmäßig verteilt. Die hydraulische Abdichtung erfolgt in eine Tasse mit schrägem Überlaufwehr (45°).

Im übrigen sind die Thormann-Böden derart ausgestaltet, dass über die Anordnung der Treibschlitze in den Hauben der Thormannn-Böden in in Querstrom-Richtung aufeinanderfolgenden Rinnen jeweils eine zueinander entgegengesetzte Strömungsrichtung der Flüssigkeit erzeugt wird.

Das Öffnungsverhältnis der Thormann-Böden beträgt 14 %. Das Verhältnis von Kaminfläche zu Schlitzaustrittsfläche beträgt 0,8. Die Kaminhöhe und die Höhe des Ablaufwehrs beträgt 40 mm. Die Bodenfreiheit der Glocke (Abstand zwischen Unterkante Schlitz und Boden) beträgt 10 mm. Die Schlitzhöhe beträgt 15 mm. Der Winkel zwischen ausgestelltem Schlitz und Längskante der Haube beträgt 30 Grad. Die Länge der Längskante der Haube beträgt maximal 800 mm. Im Randbereich der Kolonne reduziert sich die Haubenlänge auf bis zu 200 mm aus Gründen der Anpassung an die Rundheit der Kolonne. Der Abstand zwischen zwei in Querstromrichtung auf einer Linie befindlichen Hauben beträgt 66 mm. Die Ablauffläche des Ablaufschachts beträgt 1,5 % bezogen auf die Querschnittsfläche des Bodens. Die Breite zwischen den beiden unteren Längsrändern einer Haube beträgt 64 mm.

Auf der Höhe des obersten Thormann-Bodens beginnt sich die Trennkolonne wieder konisch zu verengen. 700 mm oberhalb des obersten Thormann-Bodens ist diese Verengung abgeschlossen und der Kolonneninnendurchmesser wieder auf 6,00 m zurückgeschrumpft.

1,70 m oberhalb des obersten Thormann-Bodens befindet sich der dritte Fangboden (Sammelboden, Kaminboden mit 16 ca. gleichmäßig verteilten bedachten Kaminen, Kaminhöhe = 1,50 m).

Vom dritten Fangboden werden als zweiter Seitenabzug 535506 kg/h Sauerwasser mit einer Temperatur von 65,1 °C. und bei einem Druck von ~ 1,24 bar entnommen.

Das Sauerwasser weist, wie bereits erwähnt, folgende Gehalte auf:

| | |
|---|---|
| 10,7677 Gew.-% | Acrylsäure, |
| 6,4390 Gew.-% | Essigsäure, |
| 79,5610 Gew.-% | Wasser, |
| 0,7038 Gew.-% | Ameisensäure, |
| 2,4712 Gew.-% | Formaldehyd, |
| 0,0132 Gew.-% | Acrolein, |
| 0,0082 Gew.-% | Propionsäure, |
| 0,0013 Gew.-% | Furfurale, |
| 0,0331 Gew.-% | Allylformiat, und |
| 0,0001 Gew.-% | MEHQ, und |
| 0,0013 Gew.-% | Sauerstoff. |

25537 kg/h des entnommenen Sauerwassers (65,1 °C) werden zusammen mit der Inhibitorlösung 2 auf den obersten Thormann-Boden rückgeführt.

329 kg/h der Inhibitorlösung 1 werden (von unten betrachtet) auf den 19ten Thormann-Boden rückgeführt (mit einer Temperatur von 25 °C).

316 kg/h des entnommenen Sauerwassers werden der Verbrennung zugeführt.

310 m³/h des entnommenen Sauerwassers werden mit einer Temperatur von 29,1 °C auf den sechsten der nachfolgend zu beschreibenden Ventilböden (von unten gerechnet) rückgeführt (die Kühlung erfolgt durch mehrstufigen indirekten Wärmeaustausch).

194011 kg/h des entnommenen Sauerwassers werden mit einer Temperatur von 23 °C auf den obersten der nachfolgend zu beschreibenden Ventilböden rückgeführt (die Abkühlung erfolgt gemeinsam mit der vorstehenden Sauerwassermenge durch mehrstufigen indirekten Wärmeaustausch; die letzte Kühlstufe von 29,1 °C auf 23 °C erfolgt getrennt und wärmeintegriert (flüssiges Chemical grade Propylen wird als Kühlmittel verwendet und verdampft dabei; das resultierende gasförmige Propylen wird anschließend zur Gestaltung des Reaktionsgasgemischs für die Gasphasen-Partialoxidation verwendet).

1204 kg/h des entnommenen Sauerwassers werden, wie bereits beschrieben, der kristallisativ weiterzureinigenden rohen Acrylsäure hinzugefügt.

6010 kg/h des entnommenen Sauerwassers werden zum Zweck der im Folgenden noch auszuführenden erfindungsgemäßen Extraktion der Extraktionskolonne zugeführt.

2300 mm oberhalb des dritten Fangbodens sind in der Kondensationskolonne in äquidistanter Anordnung (Bodenabstand = 500 mm) 11 zweiflutige Ventilböden angebracht. Die Höhe des Ablaufwehrs beträgt 18 bis 35 mm (die der oberen Böden sind höher wie diejenigen der unteren Böden). Das Öffnungsverhältnis (spezifische Bohrungsfläche) liegt bei 14,8 % und die Summe der Ablaufflächen der Ablaufschächte von zwei aufeinanderfolgenden Ventilböden beträgt ~10 % der Kolonnenquerschnittsfläche. Als Ventile wurden VV12-Ventile der Fa. Stahl, DE, Viernheim verwendet.

Der Druck am Kopf der Kolonne beträgt 1,17 bar.
Am Kolonnenkopf verlassen 170121 kg/h Restgas mit einer Temperatur von 31 °C und den nachfolgenden Gehalten die Trennkolonne:

| | |
|---|---|
| 0,1946 Gew.-% | Acrylsäure, |
| 0,1246 Gew.-% | Essigsäure, |
| 2,3031 Gew.-% | Wasser, |
| 0,0062 Gew.-% | Ameisensäure, |
| 0,1212 Gew.-% | Acrolein, |
| 0,0002 Gew.-% | Propionsäure, |
| 0,0001 Gew.-% | Furfurale, |
| 0,0027 Gew.-% | Allylformiat, |
| 2,3427 Gew.-% | CO₂, |
| 0,7609 Gew.-% | CO, |
| 0,7211 Gew.-% | Propan, |
| 0,1681 Gew.-% | Propylen |
| 2,7387 Gew.-% | O₂, und |
| 90,5158 Gew.-% | N₂. |

In einem indirekten Wärmeaustauscher wird das Restgas auf 38 °C erwärmt und anschließend werden 110880 kg/h dieses Restgases über einen Kreisgasverdichter auf einen Druck von 2,9 bar verdichtet, wobei die Temperatur auf ca. 160 °C steigt. 94553 kg/h des verdichteten Restgases werden als Kreisgas in die Gasphasen-Partialoxidation rückgeführt. 16327 kg/h des verdichteten Restgases werden der ersten Strippkolonne zum Zweck der Strippung des Extraktes aus der Sauerwasserextraktion zugeführt und 59241 kg/h des Restgases werden der Verbrennung zugeführt.

Die Extraktionskolonne für die Sauerwasserextraktion enthält als trennwirksame Einbauten randbündig eingepasst gelochte strukturierte Packungen (Höhe eines Packungselements: 200 mm) aus Edelstahlblechen (Werkstoff 1.4571) vom Typ Montz-Pak B1-350 mit einer aktiven Gesamthöhe von 10 m, die übereinander angeordnet sind.

Der Innendurchmesser der Extraktionskolonne beträgt über alle Packungen einheitlich 800 mm. Ihre Höhe liegt bei 14 m. Als Extraktionsmittel wird Palatinol^{®}A verwendet. Sumpf- und Kopfgefäß der Kolonne sind im Durchmesser auf 1100 mm erweitert, um die Phasentrennung im Sumpf zu verbessern und den Mitriss von Extraktionsmittel im Kopf der Kolonne zu reduzieren. Zusätzlich ist im Kopf der Kolonne eine Schüttung aus Kunststofffüllkörpern (z. B. Polyethylen oder Teflon) als Koaleszenzhilfe eingebracht.

Die Zufuhr von 6010 kg/h zu extrahierendem Sauerwasser (Temperatur = 65,1 °C) erfolgt unterhalb der untersten Packung in die Extraktionskolonne über entsprechende Durchtrittsöffnungen (Bohrungen des Durchmessers 8 mm) aufweisende Rohrverteiler. Oberhalb der obersten Packung der Extraktionskolonne wird ein Gemisch aus ca. 25 kg/h frischem Palatinol^{®}A) und 5987 kg/h aus der ersten Strippkolonne rückgeführtem und zuvor in dieser freigestripptem Extraktionsmittel (Temperatur = 50 °C) aufgegeben.

Das rückgeführte Extraktionsmittel weist folgende Gehalte auf:

| | |
|---|---|
| ≤ 0,5 Gew.-% | Acrylsäure, |
| ≤ 0,03 Gew.-% | Essigsäure, |
| ≤ 0,02 Gew.-% | Wasser, |
| ≤ 0,001 Gew.-% | Ameisensäure, |
| ≤ 0,0035 Gew.-% | Acrolein, |
| ≤ 0,0005 Gew.-% | Propionsäure, |
| ≤ 00,001 Gew.-% | Furfurale, |
| ≤ 0,001 Gew.-% | Allylformiat, |
| 0,03 Gew.-% | MEHQ, |
| 0,0001 Gew.-% | Sauerstoff, und |
| ≥ 99,5 Gew.-% | Palatinol^{®}A. |

Die spezifische Masse des Sauerwassers beträgt 967,5 kg/m³. Das Extraktionsmittel wird ebenfalls über entsprechende Durchtrittsöffnungen (Bohrungen des Durchmessers 4 mm) aufweisende Rohrverteiler aufgegeben.

Das Sauerwasser bildet die kontinuierliche Phase und das Extraktionsmittel bildet die tropfenförmig dispers verteilte Phase (Tropfendurchmesser im Bereich von 2 bis 5 mm liegend), die in der wässrigen Phase absteigt.

Am Kopf der Extraktionskolonne werden 4930 kg/h an Raffinat (Temperatur ~ 57,6°C) entnommen, das folgende Gehalte aufweist:

| | |
|---|---|
| 1,7618 Gew.-% | Acrylsäure, |
| 4,3046 Gew.-% | Essigsäure, |
| 90,1197 Gew.-% | Wasser, |
| 0,6446 Gew.-% | Ameisensäure, |
| 2,8993 Gew.-% | Formaldehyd, und |
| 0,27 Gew.-% | Palatinol^{®}A. |

Es wird gemeinsam mit zu verbrennendem Restgas der Verbrennung zugeführt.
Dem Sumpf der Extraktionskolonne werden 7090 kg/h Extrakt entnommen, das folgende Gehalte aufweist (Temperatur ~ 64,5°C)

| | |
|---|---|
| 8,1556 Gew.-% | Acrylsäure, |
| 2,4838 Gew.-% | Essigsäure, |
| 4,7901 Gew.-% | Wasser, |
| 0,1490 Gew.-% | Ameisensäure, |
| 0,0788 Gew.-% | Formaldehyd, |
| 0,0140 Gew.-% | Acrolein, |
| 0,0073 Gew.-% | Propionsäure, |
| 0,0014 Gew.-% | Furfurale, |
| 0,0282 Gew.-% | Allylformiat, und |
| 0,0192 Gew.-% | MEHQ, |
| 84,2726 Gew.-% | Palatinol^{®} A. |

Die Gesamtmenge des Extraktes wird auf den Kopf der ersten Strippkolonne geführt. Zuvor wird das Extrakt durch indirekten Wärmeaustausch in einem Plattenwärmeaustauscher auf 95 °C erwärmt. Als Wärmeträger werden 5987 kg/h an der ersten Strippkolonne entnommener Sumpfflüssigkeit verwendet. Die erste Strippkolonne enthält als trennwirksame Einbauten 5 Dual-Flow-Böden und 15 Thormann-Böden. Ebenso wie die Extraktionskolonne ist die erste Strippkolonne gegen die Umgebung thermisch isoliert. Der Innendurchmesser der ersten Strippkolonne beträgt über alle Böden einheitlich 1,5 m.

Ihre Höhe liegt bei 14,5 m. Die untersten 5 Böden sind als Dual-Flow-Böden ausgeführt und sind in der ersten Strippkolonne äquidistant (500 mm) angeordnet. Ihr Öffnungsverhältnis beträgt einheitlich 18 %. Der Lochdurchmesser der Dual-Flow-Böden beträgt einheitlich 14 mm (Lochanordnung entsprechend strenger Dreicksteilung). Oberhalb des obersten Dual-Flow-Bodens befinden sich 15 einflutige Thörmann-Böden die äquidistant angeordnet sind (500 mm Abstand). Die Thormann-Böden sind derart ausgestaltet, dass über die Anordnung der Treibschlitze in den Hauben der Thormannn-Böden in in Querstrom-Richtung aufeinanderfolgenden Rinnen jeweils eine zueinander entgegengesetzte Strömungsrichtung der Flüssigkeit erzeugt wird. Das Öffnungsverhältnis (auf den Querschnitt bezogene Gasdurchtrittsfläche) beträgt 14 %.

Oberhalb des letzten Bodens befindet sich noch eine Schüttung (Höhe 400 mm, Pall-Ringe aus Metall, 25 x 25) als Tropfenfänger.

Unterhalb des untersten Dual-Flow-Bodens werden 13000 Nm³/h an verdichtetem Restgas (Druck ~ 2,9 bar, Temperatur - 160 °C) in die erste Strippkolonne geführt, wo es im Gegenstrom zum in der Strippkolonne ablaufenden Extrakt aufsteigt.

Am Kopf der ersten Strippkolonne werden 17424 kg/h an erstem beladenem Gas herausgeführt (Temperatur = 82,2 °C) und der zweiten Strippkolonne zugeführt. Die Temperatur im Sumpf der ersten Strippkolonne beträgt ca. 155 °C. 49311 kg/h Sumpfflüssigkeit werden dem Sumpf der ersten Strippkolonne kontinuierlich entnommen. 5987 kg/h der aus der ersten Strippkolonne entnommenen Sumpfflüssigkeit werden durch zweistufigen indirekten Wärmeaustausch (die erste Stufe in einem Plattenwärmeaustauscher wärmeintegriert gegen Extrakt) auf 50 °C abgekühlt und auf den Kopf der Extraktionskolonne rückgeführt. 43324 kg/h an aus der ersten Strippkolonne entnommener Sumpfflüssigkeit werden in einem außenliegenden Zwangsumlaufrohrbündelentspannungsverdampfer auf 160 °C erhitzt und in den Sumpf der ersten Strippkolonne rückgeführt.

### Vergleichsbeispiel

Es wird wie das Beispiel ausgeführt. Das im Beispiel extrahierte Sauerwasser wird jedoch nicht extrahiert, sondern wie im Stand der Technik verbrannt.

Die Extraktionskolonne und die erste Strippkolonne entfallen. Zum Strippen der aus der Kondensationskolonne entnommenen Sumpfflüssigkeit wird die entsprechende Menge an verdichtetem Restgas verwendet. Der über den ersten Seitenabzug entnommene Strom an roher Acrylsäure beträgt 87307 kg/h und enthält 96,863 Gew.-% an Acrylsäure.
Die dem Lagertank zuzuführende Menge an Reinacrylsäure beträgt 16994 kg/h. Ihre Reinheit liegt bei 99,736 Gew.-% Acrylsäure.

### Beispiel 2

In ein mit einem zweistufigen Dreiflügelrührer gerührten Doppelmantelrührgefäß (thermostatisiert mit Wasser) mit einem Innenvolumen von 1,3 I wurden bei 50 °C 498 g nicht mit Inhibitor versetztes Sauerwasser mit einer Temperatur von 50 °C gegeben, das folgende Gehalte aufwies:

| | |
|---|---|
| 2,19 Gew.-% | Formaldeyhd, |
| 82,00 Gew.-% | Wasser, |
| 4,01 Gew.-% | Essigsäure, |
| 11,09 Gew.-% | Acrylsäure, |
| 0,69 Gew.-% | Ameisensäure, und |
| 0,01 Gew.-% | Diacrylsäure. |

Unter Rühren wurden anschließend 499 g Dimethylphthalat zugegeben, das ebenfalls 50 °C aufwies. Bei konstanter Temperatur von 50 °C wurde die resultierende Mischung während 10 min bei einer Drehzahl von 250 Umdrehungen/min gerührt. Nach dem Abschalten des Rührers wurde das Gemisch bei 50 °C sich selbst überlassen, wobei innerhalb kurzer Zeit Phasentrennung eintrat. Die Masse der organischen Phase betrug 565 g, die Masse der wässrigen Phase betrug 432 g. Gaschromatographische Analyse der wässrigen Phase ergab, dass diese noch 20,26 g Acrylsäure und 13,0 g Essigsäure enthielt.

### Beispiel 3

Zur Extraktion von Acrylsäure aus Sauerwasser wurde eine Extraktionskolonne aus Glas eingesetzt. Das nicht mit Inhibitor versetzte Sauerwasser wies folgende Gehalte auf:

| | |
|---|---|
| 2,36 Gew.-% | Formaldeyhd, |
| 83,12 Gew.-% | Wasser, |
| 3,98 Gew.-% | Essigsäure, |
| 9,70 Gew.-% | Acrylsäure, |
| 0,68 Gew.-% | Ameisensäure, und |
| 0,01 Gew.-% | Diacrylsäure. |

Die Extraktionskolonne war über einen Doppelmantel thermostatisierbar (mit 60°C warmem Wasser). Als trennwirksame Einbauten enthielt die Extraktionskolonne strukturierte Edelstahlblechpackungen (Edelstahl vom Typ 1.4404, gelochte Packungen der Firma Montz vom Typ B1-350). Der Innendurchmesser der Extraktionskolonne betrug im bepackten Teil 40 mm. In den Sumpf der Kolonne wurden 10 kg/h der wässrigen Lösung (Temperatur = 60 °C) geführt. Am Kopf der Extraktionskolonne wurden im Gegenstrom als disperse Phase (Tropfengröße: 4 bis 5 mm) 10 kg/h Diethylphthalat zugefahren (Temperatur = 60 °C). Das am Kopf der Kolonne herausgeführte wässrige Raffinat enthielt noch 0,8 Gew.-% Acrylsäure und 2,9 Gew.-% Essigsäure. Der Gehalt an Acrylsäure im Sauerwasser konnte somit (bezogen auf den ursprünglichen Gewichtsgehalt) um 93,3 Gew.-% abgereichert werden und der von Essigsäure um 41 Gew.-%. Im Raffinat waren 0,2 Gew.-% Diethylphthalt gelöst.

### Beispiel 4

Es wurde wie in Beispiel 3 verfahren. Als Extraktionsmittel und disperse Phase wurden am Kopf der Extraktionskolonne 27,5 kg/h an Dimethylphthalat (T = 60°C) aufgegeben (Tropfengröße: 4 bis 5 mm). Das Sauerwasser, von dem ebenfalls als Mengenstrom 27,5 kg/h mit einer Temperatur von 60°C in den Sumpf der Kolonne als kontinuierliche Phase zugefahren wurde, hatte folgende Gehalte:

| | |
|---|---|
| 2,77 Gew.-% | Formaldeyhd, |
| 82,82 Gew.-% | Wasser, |
| 4,02 Gew.-% | Essigsäure, |
| 9,53 Gew.-% | Acrylsäure, |
| 0,63 Gew.-% | Ameisensäure, und |
| 0,05 Gew.-% | Diacrylsäure. |

Das am Kopf der Extraktionskolonne ablaufende wässrige Raffinat enthielt noch 0,2 Gew.-% Acrylsäure und 2,4 Gew.-% Essigsäure. Der Gehalt an Acrylsäure im Sauerwasser konnte somit (bezogen auf den ursprünglichen Gewichtsgehalt) um 98,3 Gew.-% abgereichert werden und der von Essigsäure um 51,5 Gew.-%. Im Raffinat waren 0,9 Gew.-% Dimethylphthalat gelöst.

### Beispiel 5

Es wird wie im Beispiel 1 verfahren. Zusätzlich zur Rücklaufflüssigkeit (Sauerwasser) werden gemeinsam mit derselben und mit der gleichen Temperatur 1000 kg/h an Wasser (zusätzliches Absorptionsmittel) in die Kondensationskolonne geführt. Der Furfuralgehalt der vom zweiten Fangboden als erster Seitenabzug aus der Kondensationskolonne herausgeführten rohen Acrylsäure fällt dadurch von 0,1642 Gew.-% (Wert im Beispiel 1) auf 0,1225 Gew.-%.

### Beispiel 6

Es wird wie im Beispiel 1 verfahren. Zusätzlich zur Rücklaufflüssigkeit (Sauerwasser) werden gemeinsam mit derselben und mit der gleichen Temperatur 5000 kg/h an Wasser (zusätzliches Absorptionsmittel) in die Kondensationskolonne geführt. Der Furfuralgehalt der von zweiten Fangboden als erster Seitenabzug aus der Kondensationskolonne herausgeführten rohen Acrylsäure fällt dadurch von 0,1642 Gew.-% (Wert im Beispiel 1) auf 0,1125 Gew.-%.

## Patentansprüche

1. Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, die Temperatur des Acrylsäure, Wasserdampf und Nebenkomponenten enthaltenden Produktgasgemischs gegebenenfalls durch direkte und/oder indirekte Kühlung verringert, und das Acrylsäure, Wasserdampf und Nebenkomponenten enthaltende Produktgasgemisch anschließend in eine mit trennwirksamen Einbauten ausgerüstete Kondensationskolonne leitet, innerhalb der Kondensationskolonne in sich selbst aufsteigen lässt und dabei fraktionierend kondensiert sowie über einen ersten, oberhalb der Zuführstelle des Produktgasgemischs in die Kondensationskolonne befindlichen, Seitenabzug eine Wasser und Nebenkomponenten insgesamt entreichert enthaltende rohe Acrylsäure als Zielprodukt und über einen zweiten, oberhalb des ersten Seitenabzugs befindlichen, Flüssigphasenabzug noch Acrylsäure und Nebenkomponenten enthaltendes saures Wasser und am Kopf der Kondensationsationskolonne ein tiefer als Wasser siedende Nebenkomponenten enthaltendes Restgasgemisch sowie aus dem Sumpfraum der Kondensationskolonne eine noch Acrylsäure und schwerer als Acrylsäure siedende Folgeprodukte und Nebenkomponenten enthaltende Sumpfflüssigkeit aus der Kondensationskolonne herausführt, eine Teilmenge des entnommenen sauren Wassers als solches und/oder nach Abkühlung desselben als Rücklaufflüssigkeit in die Kondensationskolonne rückführt und die rohe Acrylsäure gegebenenfalls wenigstens einem weiteren thermischen Trennverfahren zum Zweck ihrer Weiterreinigung unterwirft, **dadurch gekennzeichnet, dass** man wenigstens bei einer Teilmenge von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes aus dem sauren Wasser heraus in das organische Lösungsmittel aufnimmt, nachfolgend die Acrylsäure aus dem organischen Extrakt durch Strippen mit einem ersten Strippgas abtrennt und
das mit Acrylsäure beladene erste Strippgas in die Kondensationskolonne rückführt und/oder die im ersten beladenen Strippgas enthaltene Acrylsäure in die wässrige Lösung eines Metallhydroxids aufnimmt, oder
das dabei anfallende mit Acrylsäure beladene erste Strippgas als ein zweites Strippgas dazu verwendet, um in aus der Kondensationskolonne herausgeführter Sumpfflüssigkeit noch enthaltene Acrylsäure freizustrippen und das dabei anfallende mit Acrylsäure beladene zweite Strippgas in die Kondensationskolonne rückführt und/oder die im zweiten Strippgas enthaltene Acrylsäure in die wässrige Lösung eines Metallhydroxids aufnimmt.

2. Verfahren nach Anspruch 1, **dadurch gekennzeichnet, dass** der C₃-Vorläufer Propylen, oder Acrolein, oder ein Gemisch aus Propylen und Acrolein ist.

3. Verfahren nach Anspruch 1 oder 2, **dadurch gekennzeichnet, dass** man bei wenigstens 25 Gew.-% von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes in das organische Lösungsmittel aufnimmt.

4. Verfahren nach einem der Ansprüche 1 bis 3, **dadurch gekennzeichnet, dass** die Extraktion der Acrylsäure aus dem Sauerwasser in einer Extraktionskolonne durchgeführt wird, die als trennwirksame Einbauten Packungen und/oder Siebböden enthält.

5. Verfahren nach Anspruch 4, **dadurch gekennzeichnet, dass** das organische Lösungsmittel am Kopf der Extraktionskolonne und das Sauerwasser im Sumpfbereich der Extraktionskolonne aufgegeben wird und das organische Lösungsmittel als dispers verteilte Phase in der kontinuierlichen Sauerwasserphase absteigt, oder **dadurch gekennzeichnet, dass** das Sauerwasser am Kopf der Extraktionskolonne und das organische Lösungsmittel im Sumpfbereich der Extraktionskolonne aufgegeben wird, und das organische Lösungsmittel als dispers verteilte Phase in der kontinuierlichen Sauerwasserphase aufsteigt.

6. Verfahren nach einem der Ansprüche 1 bis 5, **dadurch gekennzeichnet, dass** das organische Lösungsmittel wenigstens einen Ester aus einer 5 bis 20 C-Atome enthaltenden aliphatischen oder aromatischen Monocarbonsäure und einem 1 bis 8 C-Atome aufweisenden Alkohol enthält.

7. Verfahren nach einem der Ansprüche 1 bis 5, **dadurch gekennzeichnet**, das das organische Lösungsmittel wenigstens einen Diester aus einer 5 bis 20 C-Atome enthaltenden aliphatischen oder aromatischen Dicarbonsäure und einem 1 bis 8 C-Atome aufweisenden Alkohol enthält.

8. Verfahren nach einem der Ansprüche 1 bis 5 oder nach Anspruch 7, **dadurch gekennzeichnet, dass** das organische Lösungsmittel Dimethylphthalat, Diethylphthalat, Dimethylisophthalat, Diethylisophthalat, Dimethyltherephthalat und/oder Diethyltherephthalat ist.

9. Verfahren nach einem der Ansprüche 1 bis 8, **dadurch gekennzeichnet, dass** der Siedepunkt des organischen Lösungsmittels bei Atmosphärendruck ≥ 200 °C beträgt.

10. Verfahren nach einem der Ansprüche 1 bis 9, **dadurch gekennzeichnet, dass** die Strippung der aus der Kondensationskolonne herausgeführten Sumpfflüssigkeit in einer mit trennwirksamen Einbauten versehenen Strippkolonne durchgeführt wird, und die Temperatur im Sumpf der Strippkolonne 150 bis 190 °C beträgt.

11. Verfahren nach einem der Ansprüche 1 bis 10, **dadurch gekennzeichnet, dass** man das mit Acrylsäure beladene zweite Strippgas einer Gegenstrom-Rektifikation unterwirft, bevor es mit Acrylsäure beladen in die Kondensationskolonne rückgeführt und/oder die in ihm enthaltene Acrylsäure in die wässrige Lösung eines Metallhydroxids aufgenommen wird.

12. Verfahren nach einem der Ansprüche 1 bis 11, **dadurch gekennzeichnet, dass** als erstes Strippgas Luft, N₂, CO₂ und/oder Wasserdampf verwendet wird.

13. Verfahren nach einem der Ansprüche 1 bis 11, **dadurch gekennzeichnet, dass** als erstes Strippgas Restgasgemisch verwendet wird.

14. Verfahren nach einem der Ansprüche 1 bis 13, **dadurch gekennzeichnet, dass** die rohe Acrylsäure kristallisativ weitergereinigt wird.

15. Verfahren nach Anspruch 14, **dadurch gekennzeichnet, dass** der rohen Acrylsäure vorab der kristallisativen Weiterreinigung eine Teilmenge des nicht in die Kondensationskolonne rückgeführten sauren Wassers zugeführt wird.

16. Verfahren nach Anspruch 14 oder 15, **dadurch gekennzeichnet, dass** die kristallisative Weiterreinigung der rohen Acrylsäure oder deren Gemisch mit saurem Wasser durch Suspensionskristallisation erfolgt.

17. Verfahren nach Anspruch 16, **dadurch gekennzeichnet, dass** zur Trennung von bei der Suspensionskristallisation verbliebener Mutterlauge und gebildetem Suspensionskristallisat eine Waschkolonne mitverwendet wird.

18. Verfahren nach einem der Ansprüche 15 bis 17, **dadurch gekennzeichnet, dass** sich ein Verfahren der radikalischen Polymerisation anschließt, in welchem aufgeschmolzenes Acrylsäurekristallisat und/oder dessen Metallsalz miteinpolymerisiert wird.

19. Verfahren nach einem der Ansprüche 1 bis 9 oder nach Anspruch 11, **dadurch gekennzeichnet, dass** sich ein Verfahren der radikalischen Polymerisation anschließt, in welchem aus beladenem erstem und/oder zweitem Strippgas in die wässrige Lösung eines Metallhydroxids aufgenommene Acrylsäure miteinpolymerisiert wird.

20. Verfahren nach einem der Ansprüche 1 bis 19, **dadurch gekennzeichnet, dass** eine Teilmenge des Restgasgemischs als Kreisgas in die Gasphasen-Partialoxidation rückgeführt wird.

21. Verfahren nach einem der Ansprüche 1 bis 20, **dadurch gekennzeichnet, dass** das organische Lösungsmittel eine Massendichte aufweist, die sich unter den Bedingungen der Extraktion von derjenigen von Wasser um ≥ 25 kg/m³ unterscheidet.

22. Verfahren nach einem der Ansprüche 1 bis 21, **dadurch gekennzeichnet, dass** die wässrige Lösung eines Metallhydroxids, NaOH, KOH, Ca(OH)₂ und/oder Mg(OH)₂ gelöst enthält.

23. Verfahren nach einem der Ansprüche 1 bis 22, **dadurch gekennzeichnet, dass** der Kondensationskolonne über eine zwischen ihrem ersten Seitenabzug und ihrem zweiten Seitenabzug gelegene Zufuhrstelfe ein flüssiges Absorptionsmittel zugeführt wird, dessen Siedetemperatur Tₛ bei einem Druck von 101.3 kPa (1 atm) größer oder gleich der Siedetemperatur T_{w} von Wasser bei einem Druck von 101.3 kPa (1 atm) ist.

## Claims

1. A process for preparing acrylic acid, in which a product gas mixture comprising acrylic acid, steam and secondary components is obtained by heterogeneously catalyzed gas phase partial oxidation of at least one C₃ precursor of acrylic acid with molecular oxygen over solid-state catalysts at elevated temperature, the temperature of the product gas mixture comprising acrylic acid, steam and secondary components is optionally reduced by direct and/or indirect cooling, and the product gas mixture comprising acrylic acid, steam and secondary components is then passed into a condensation column equipped with separating internals, allowed to ascend into itself within the condensation column and thus fractionally condensed, and crude acrylic acid comprising water and secondary components depleted overall is conducted as the target product out of the condensation column via a first side draw disposed above the feed point of the product gas mixture into the condensation column, as are acid water still comprising acrylic acid and secondary components via a second liquid phase draw disposed above the first side draw and a residual gas mixture comprising secondary components having lower boiling points than water at the top of the condensation column and a bottoms liquid still comprising acrylic acid and conversion products and secondary components having higher boiling points than acrylic acid from the bottom space of the condensation column, a portion of the acid water withdrawn is recycled as such and/or after cooling thereof as reflux liquid into the condensation column and the crude acrylic acid is optionally subjected to at least one further thermal separation process for the purpose of its further purification, wherein acrylic acid present at least in a portion of acid water not recycled into the condensation column is taken up from the acid water into an organic solvent by extraction with the organic solvent to form an organic extract comprising acrylic acid, then the acrylic acid is removed from the organic extract by stripping with a first stripping gas and
the acrylic acid-laden first stripping gas is recycled into the condensation column and/or the acrylic acid present in the first laden stripping gas is taken up into the aqueous solution of a metal hydroxide,
or the resulting acrylic acid-laden first stripping gas is used as the second stripping gas in order to strip the bottoms liquid conducted out of the condensation column free of acrylic acid still present, and the resulting acrylic acid-laden second stripping gas is recycled into the condensation column and/or the acrylic acid present in the second stripping gas is taken up into the aqueous solution of a metal hydroxide.

2. The process according to claim 1, wherein the C₃ precursor is propylene or acrolein or a mixture of propylene and acrolein.

3. The process according to claim 1 or 2, wherein acrylic acid present at least in 25% by weight of acid water not recycled into the condensation column is taken up from the acid water into an organic solvent by extraction with the organic solvent to form an organic extract comprising acrylic acid.

4. The process according to any of claims 1 to 3, wherein the extraction of the acrylic acid from the acid water is performed in an extraction column which comprises structured packings and/or sieve trays as separating internals.

5. The process according to claim 4, wherein the organic solvent is introduced at the top of the extraction column and the acid water in the bottom region of the extraction column, and the organic solvent descends as a dispersed phase in the continuous acid water phase, or wherein the acid water is introduced at the top of the extraction column and the organic solvent in the bottom region of the extraction column, and the organic solvent ascends as a dispersed phase in the continuous acid water phase.

6. The process according to any of claims 1 to 5, wherein the organic solvent comprises at least one ester of an aliphatic or aromatic monocarboxylic acid comprising from 5 to 20 carbon atoms and an alcohol having from 1 to 8 carbon atoms.

7. The process according to any of claims 1 to 5, wherein the organic solvent comprises at least one diester of an aliphatic or aromatic dicarboxylic acid comprising from 5 to 20 carbon atoms and an alcohol having from 1 to 8 carbon atoms.

8. The process according to any of claims 1 to 5 or according to claim 7, wherein the organic solvent is dimethyl phthalate, diethyl phthalate, dimethyl isophthalate, diethyl isophthalate, dimethyl terephthalate and/or diethyl terephthalate.

9. The process according to any of claims 1 to 8, wherein the boiling point of the organic solvent at atmospheric pressure is ≥ 200°C.

10. The process according to any of claims 1 to 9, wherein the stripping of the bottoms liquid conducted out of the condensation column is performed in a stripping column provided with separating internals, and the temperature in the bottom of the stripping column is from 150 to 190°C.

11. The process according to any of claims 1 to 10, wherein the acrylic acid-laden second stripping gas is subjected to a countercurrent rectification before it is recycled into the condensation column laden with acrylic acid and/or the acrylic acid present therein is taken up into the aqueous solution of a metal hydroxide.

12. The process according to any of claims 1 to 11, wherein the first stripping gas used is air, N₂, CO₂ and/or steam.

13. The process according to any of claims 1 to 11, wherein the first stripping gas used is residual gas mixture.

14. The process according to any of claims 1 to 13, wherein the crude acrylic acid is purified further by crystallization.

15. The process according to claim 14, wherein a portion of the acid water not recycled into the condensation column is added to the crude acrylic acid before the crystallizative further purification.

16. The process according to claim 14 or 15, wherein the crystallizative further purification of the crude acrylic acid or the mixture thereof with acid water is effected by suspension crystallization.

17. The process according to claim 16, wherein a wash column is used additionally to separate mother liquor remaining in the suspension crystallization and suspension crystals formed.

18. The process according to any of claims 15 to 17, which is followed by a process for free-radical polymerization in which molten acrylic acid crystals and/or the metal salt thereof is polymerized.

19. The process according to any of claims 1 to 9 or according to claim 11, which is followed by a process for free-radical polymerization in which acrylic acid taken up from laden first and/or second stripping gas into the aqueous solution of a metal hydroxide is polymerized.

20. The process according to any of claims 1 to 19, wherein a portion of the residual gas mixture is recycled as cycle gas into the gas phase partial oxidation.

21. The process according to any of claims 1 to 20, wherein the organic solvent has a mass density which, under the conditions of the extraction, differs from that of water by ≥ 25 kg/m³.

22. The process according to any of claims 1 to 21, wherein the aqueous solution of a metal hydroxide comprises NaOH, KOH, Ca(OH)₂ and/or Mg(OH)₂ in dissolved form.

23. The process according to any of claims 1 to 22, wherein a liquid absorbent whose boiling point Tₛ at a pressure of 101.3 kPa (1 atm) is greater than or equal to the boiling point T_{w} of water at a pressure of 101.3 kPa (1 atm) is fed to the condensation column via a feed point disposed between its first side draw and its second side draw.

## Revendications

1. Procédé pour la production d'acide acrylique, dans lequel on produit par oxydation partielle en phase gazeuse, catalysée en catalyse hétérogène, d'au moins un précurseur en C₃ de l'acide acrylique avec de l'oxygène moléculaire sur des catalyseurs se trouvant à l'état d'agrégats solides, à température élevée, un mélange gazeux de produits contenant de l'acide acrylique, de la vapeur d'eau et des composants secondaires, éventuellement on abaisse par refroidissement direct et/ou refroidissement indirect la température du mélange gazeux de produits contenant de l'acide acrylique, de la vapeur d'eau et des composants secondaires, et ensuite on fait passer le mélange gazeux de produits contenant de l'acide acrylique, de la vapeur d'eau et des composants secondaires dans une colonne de condensation munie d'inserts efficaces pour la séparation, on le laisse monter de lui-même à l'intérieur de la colonne de condensation et en même temps se condenser en se fractionnant, ainsi que, par une première décharge latérale se trouvant au-dessus du point d'arrivée du mélange gazeux de produits dans la colonne de condensation, un acide acrylique brut contenant en en étant globalement appauvri de l'eau et des composants secondaires est évacué en tant que produit recherché et de l'eau acide contenant encore de l'acide acrylique et des composants secondaires est évacuée par une deuxième décharge de phase liquide, se trouvant au-dessus de la première décharge latérale, et un mélange de gaz résiduaires contenant des composants secondaires à plus bas point d'ébullition que l'eau est évacué à la tête de la colonne de condensation, ainsi qu'un liquide de pied, contenant encore de l'acide acrylique et des dérivés et produits secondaires à plus haut point d'ébullition que l'acide acrylique est évacué de la colonne de condensation à partir de l'espace de pied de la colonne de condensation, une quantité partielle de l'eau acide prélevée est renvoyée, telle quelle et/ou après refroidissement de celle-ci, en tant que liquide de recyclage, dans la colonne de condensation et éventuellement on soumet l'acide acrylique brut à au moins un nouveau processus de fractionnement thermique afin de le purifier davantage, **caractérisé en ce qu'**au moins pour une quantité partielle d'eau acide non recyclée dans la colonne de condensation l'acide acrylique contenu dans celle-ci est extrait de l'eau acide par extraction à l'aide d'un solvant organique avec formation d'un extrait organique contenant de l'acide acrylique, et absorbé dans le solvant organique, puis on sépare l'acide acrylique de l'extrait organique par rectification avec un premier gaz de strippage et
le premier gaz de strippage chargé d'acide acrylique est renvoyé dans la colonne de condensation et/ou l'acide acrylique contenu dans le premier gaz de strippage est absorbé dans la solution aqueuse d'un hydroxyde métallique, ou
le premier gaz de strippage ainsi produit, chargé d'acide acrylique, est utilisé en tant qu'un deuxième gaz de strippage pour séparer par strippage l'acide acrylique encore contenu dans le liquide de pied évacué de la colonne de condensation et le deuxième gaz de strippage ainsi produit, chargé d'acide acrylique, est renvoyé dans la colonne de condensation et/ou l'acide acrylique contenu dans le deuxième gaz de strippage est absorbé dans la solution aqueuse d'un hydroxyde métallique.

2. Procédé selon la revendication 1, **caractérisé en ce que** le précurseur en C₃ est le propylène, ou l'acroléine, ou un mélange de propylène et d'acroléine.

3. Procédé selon la revendication 1 ou 2, **caractérisé en ce que** pour au moins 25% en poids d'eau acide non recyclée dans la colonne de condensation on absorbe dans le solvant organique l'acide acrylique contenu dans celle-ci par extraction à l'aide d'un solvant organique avec formation d'un extrait organique contenant de d'acide acrylique.

4. Procédé selon l'une quelconque des revendications 1 à 3, **caractérisé en ce qu'**on effectue l'extraction de l'acide acrylique à partir de l'eau acide dans une colonne d'extraction qui contient en tant qu'inserts efficaces pour la séparation des garnissages et/ou des plateaux perforés.

5. Procédé selon la revendication 4, **caractérisé en ce qu'**on introduit le solvant organique à la tête de la colonne d'extraction et l'eau acide dans la zone du pied de la colonne d'extraction et le solvant organique descend, en tant que phase dispersée, dans la phase continue d'eau acide, ou **caractérisé en ce qu'**on introduit l'eau acide à la tête de la colonne d'extraction et le solvant organique dans la zone du pied de la colonne d'extraction, et le solvant organique monte, en tant que phase divisée en dispersion, dans la phase d'eau acide continue.

6. Procédé selon l'une quelconque des revendications 1 à 5, **caractérisé en ce que** le solvant organique contient au moins un ester d'un acide monocarboxylique aliphatique ou aromatique contenant de 5 à 20 atomes de carbone et d'un alcool comportant de 1 à 8 atomes de carbone.

7. Procédé selon l'une quelconque des revendications 1 à 5, **caractérisé en ce que** le solvant organique contient au moins un diester d'un acide dicarboxylique aliphatique ou aromatique contenant de 5 à 20 atomes de carbone et d'un alcool comportant de 1 à 8 atomes de carbone.

8. Procédé selon l'une quelconque des revendications 1 à 5 ou selon la revendication 7, **caractérisé en ce que** le solvant organique est le phtalate de diméthyle, le phtalate de diéthyle, l'isophtalate de diméthyle, l'isophtalate de diéthyle, le téréphtalate de diméthyle et/ou le téréphtalate de diéthyle.

9. Procédé selon l'une quelconque des revendications 1 à 8, **caractérisé en ce que** le point d'ébullition du solvant organique sous la pression atmosphérique est ≥ 200 °C.

10. Procédé selon l'une quelconque des revendications 1 à 9, **caractérisé en ce que** le strippage du liquide de pied évacué de la colonne de condensation est effectué dans une colonne de strippage munie d'inserts efficaces pour la séparation, et la température dans le pied de la colonne de strippage vaut 150 à 190 °C.

11. Procédé selon l'une quelconque des revendications 1 à 10, **caractérisé en ce qu'**on soumet à une rectification à contre-courant le deuxième gaz de strippage chargé d'acide acrylique, avant de le renvoyer, chargé d'acide acrylique, dans la colonne de condensation et/ou que l'acide acrylique qu'il contient soit absorbé dans la solution aqueuse d'un hydroxyde métallique.

12. Procédé selon l'une quelconque des revendications 1 à 11, **caractérisé en ce qu'**on utilise comme premier gaz de strippage de l'air, N₂, CO₂ et/ou de la vapeur d'eau.

13. Procédé selon l'une quelconque des revendications 1 à 11, **caractérisé en ce qu'**on utilise comme premier gaz de strippage un mélange de gaz résiduaires.

14. Procédé selon l'une quelconque des revendications 1 à 13, **caractérisé en ce que** l'acide acrylique brut est purifié davantage par cristallisation.

15. Procédé selon la revendication 14, **caractérisé en ce qu'**avant de purifier davantage par cristallisation l'acide acrylique brut on y ajoute une quantité partielle de l'eau acide non recyclée dans la colonne de condensation.

16. Procédé selon la revendication 14 ou 15, **caractérisé en ce que** la purification plus poussée par cristallisation de l'acide acrylique brut ou du mélange de celui-ci avec de l'eau acide s'effectue par cristallisation en suspension.

17. Procédé selon la revendication 16, **caractérisé en ce que** pour la séparation de la liqueur mère restant lors de la cristallisation en suspension et du cristallisat en suspension formé on utilise en même temps une colonne de lavage.

18. Procédé selon l'une quelconque des revendications 15 à 17, **caractérisé en ce qu'**à la polymérisation radicalaire fait suite un processus dans lequel on incorpore par copolymérisation le cristallisat d'acide acrylique fondu et/ou un sel métallique de ce dernier.

19. Procédé selon l'une quelconque des revendications 1 à 9 ou selon la revendication 11, **caractérisé en ce qu'**à la polymérisation radicalaire fait suite un processus dans lequel à partir du premier et/ou du deuxième gaz de strippage chargé on incorpore par copolymérisation l'acide acrylique absorbé dans la solution aqueuse d'un hydroxyde métallique.

20. Procédé selon l'une quelconque des revendications 1 à 19, **caractérisé en ce qu'**une quantité partielle du mélange de gaz résiduels est renvoyée en tant que gaz en circuit dans l'oxydation partielle en phase gazeuse.

21. Procédé selon l'une quelconque des revendications 1 à 20, **caractérisé en ce que** le solvant organique présente une densité massique qui dans les conditions de l'extraction diffère de ≥ 25 kg/m³ de celle de l'eau.

22. Procédé selon l'une quelconque des revendications 1 à 21, **caractérisé en ce que** la solution aqueuse d'un hydroxyde métallique contient en solution NaOH, KOH, Ca(OH)₂ et/ou Mg(OH)₂.

23. Procédé selon l'une quelconque des revendications 1 à 22, **caractérisé en ce qu'**on envoie à la colonne de condensation par un point d'alimentation situé entre sa première décharge latérale et sa deuxième décharge latérale un absorbant liquide dont la température d'ébullition Tₛ sous une pression de 101,3 kPa (1 atm) est supérieure ou égale à la température d'ébullition T_{w} de l'eau sous une pression de 101,3 kPa (1 atm).
